# EUROPEAN PATENT APPLICATION

(11) **EP 3 922 641 A1**
(43) Date of publication of application: **15.12.2021**
(21) Application number: 20305624.7
(22) Date of filing: 09.06.2020
(51) Int. Cl.: C07K 14/47, C07K 14/74, C12N 5/0787, C12N 5/0786, G01N 33/50, G01N 33/569

(54) **METHODS FOR DETECTING AND TREATING COVID PATIENTS REQUIRING INTENSIVE CARE**

(71) Applicant: Institut Gustave-Roussy, 94800 Villejuif (FR)
(72) Inventor: The designation of the inventor has not yet been filed
(74) Representative: Regimbeau

(57) **Abstract**

The present invention provides novel, easy-to-monitor and sensitive parameters, biomarkers and methods for the prediction of SARS-CoV-2 patients outcome, allowing in particular an early identification of patients evolving towards severe COVID-19 forms.

The examples presented in the application show that a calprotectin-driven emergency myelopoisis in patients undergoing a switch toward a severe form of COVID 19 generates an innate immune cell signature of severe COVID-19.

More precisely, the present inventors have found that the amount of CD14^{low}/CD16⁺ monocytes, of HLA-DR^{low} classical monocytes and of CD16^{low} neutrophils can independently or concomitantly be used as biomarkers of the severity of betacoronavirus infection, before the switch to the severe form of the infection (and related painful symptoms) actually occurs. The methods of the invention comprise the quantification, in a biological sample from an infected subject, of the amount of these immune cells.

In addition, the Inventors have observed a surprising burst of calprotectin levels in COVID-19 patients that are undergoing a switch toward a severe disease. They therefore propose to use calprotectin plasma level as a biomarker of COVID-19 switch to severe form, and also as a therapeutic target for alleviating the disease symptoms.

SEQUENCE LISTING

<110>  INSTITUT GUSTAVE-ROUSSY

<120>  METHODS FOR DETECTING AND TREATING COVID PATIENTS REQUIRING
       INTENSIVE CARE

<130>  B76091 D40630

<160>  8

<170>  PatentIn version 3.5

<210>  1
<211>  26
<212>  DNA
<213>  artificial

<220>
<223>  GUS forward primer

<400>  1
gaaaatatgt ggttggagag ctcatt                                          26


<210>  2
<211>  22
<212>  DNA
<213>  artificial

<220>
<223>  GUS reverse primer

<400>  2
   ccgagtgaag atcccctttt ta                                             22


<210>  3
<211>  21
<212>  DNA
<213>  artificial

<220>
<223>  HPRT forward primer

<400>  3
ggacaggact gaacgtcttg c                                               21


<210>  4
<211>  22
<212>  DNA
<213>  artificial

<220>
<223>  HPRT reverse primer

<400>  4
cttgagcaca cagagggcta ca                                              22


<210>  5
<211>  25

<212>  DNA
<213>  artificial

<220>
<223>  forward primer S100A8

<400>  5
caacactgat ggtgcagtta acttc                                          25


<210>  6
<211>  20
<212>  DNA
<213>  artificial

<220>
<223>  reverse primer S100A8

<400>  6
ctgccacgcc catctttatc                                                20


<210>  7
<211>  22
<212>  DNA
<213>  artificial

<220>
<223>  forward primer S100A9

<400>  7
ctgagcttcg aggagttcat ca                                             22


<210>  8
<211>  20
<212>  DNA
<213>  artificial

<220>
<223>  reverse primer S100A9

<400>  8
cgtcaccctc gtgcatcttc                                                20

## Description

### SUMMARY OF THE INVENTION

The present invention provides novel, easy-to-monitor and sensitive parameters, biomarkers and methods for the prediction of SARS-CoV-2 patients outcome, allowing in particular an early identification of patients evolving towards severe COVID-19 forms.

The examples presented in the application show that a calprotectin-driven emergency myelopoisis in patients undergoing a switch toward a severe form of COVID 19 generates an innate immune cell signature of severe COVID-19.

More precisely, the present inventors have found that the amount of CD14^{low}/CD16⁺ monocytes, of HLA-DR^{low} classical monocytes and of CD16^{low} neutrophils can independently or concomitantly be used as biomarkers of the severity of betacoronavirus infection, before the switch to the severe form of the infection (and related painful symptoms) actually occurs. The methods of the invention comprise the quantification, in a biological sample from an infected subject, of the amount of these immune cells.

In addition, the Inventors have observed a surprising burst of calprotectin levels in COVID-19 patients that are undergoing a switch toward a severe disease. They therefore propose to use calprotectin plasma level as a biomarker of COVID-19 switch to severe form, and also as a therapeutic target for alleviating the disease symptoms.

### BACKGROUND ART

Coronavirus disease 2019 (COVID-19) is caused by severe acute respiratory syndrome-coronavirus 2 (SARS-CoV-2), which infects the lungs leading to fever, cough and dyspnea (Guan and Zhong, 2020). Most patients presenting with mild disease develop an efficient immune response (Thevarajan et al., 2020), but some go on to suffer acute respiratory distress syndrome leading to intensive care unit (ICU) admission, often culminating in multi-organ dysfunction and death (Wang et al., 2020). SARS-CoV-2 belongs to the species *Coronavirus,* in the genus *Betacoronavirus* and family *Coronaviridae.*

Coronaviruses are enveloped viruses with a helically symmetrical capsid. They have a single-stranded, positive-sense RNA genome and are capable of infecting cells in birds and mammals.

The morphology of the virions is typical, with a halo of protein protuberances ('Spike') which gave them their name of 'crown virus'. Among the four genera of coronaviruses, the *Betacoronavirus* genus (β-CoVs or Beta-CoVs), comprising virus infecting animals and/or humans, is subdivided into four lineages designated as A, B, C and D: Lineage A also designated as subgenus *Embecovirus* includes HCoV-OC43 and HCoV-HKU1, virus able to infect various species; Lineage B also designated as subgenus *Sarbecovirus* includes SARS-CoV-1, SARS-CoV-2, and Bat SL-CoV-WIV1; Lineage C also designated as subgenus *Merbecovirus* includes Tylonycteris bat coronavirus HKU4 (BtCoV-HKU4), Pipistrellus bat coronavirus HKU5 (BtCoV-HKU5), and MERS-CoV, able to infect notably camels and humans; Lineage D also designated as subgenus *Nobecovirus* includes Rousettus bat coronavirus HKU9 (BtCoV-HKU9).

The Betacoronaviruses of the greatest clinical importance concerning humans are:
- OC43 and HKU1 of the A lineage,
- SARS-CoV-1 and SARS-CoV-2 of the B lineage, and
- MERS-CoV of the C lineage.

In humans, coronavirus infections can cause respiratory pathologies associated with symptoms similar to the common cold, bronchiolitis and more serious diseases such as the Severe Acute Respiratory Syndrome caused by SARS-CoV-1, which generated an epidemic in 2003, and the Middle Eastern Respiratory Syndrome caused by MERS-CoV, which generated an epidemic in 2012. SARS-CoV-2 is the betacoronavirus causing the coronavirus epidemic of 2019-2020, generating the form of pneumonia known as coronavirus disease 2019 or COVID-19. According to the World Health Organization (WHO), by April 5, 2020, 1 133 758 cases of COVID-19 have been confirmed and 62 784 patients have died worldwide. This epidemic was declared a public health emergency of international concern by WHO on January 30, 2020.

In addition to cell autonomous effects of the viral infection, a dysregulated immune response participates in the sudden deterioration of COVID-19 patients ultimately overwhelming infected and non-infected tissues. This over inflammatory response commonly includes a cytokine storm (Chen et al., 2020a), with elevated blood concentrations of interleukin-6 (IL-6). Accordingly, therapeutic agents targeting the IL-6/IL-6R-gp130 axis can alleviate the inflammatory response (Michot et al., 2020) and ameliorate immune dysregulation (Giamarellos-Bourboulis et al., 2020), emphasizing the clinical significance of this cytokine. A marked lymphocytopenia is also associated with COVID-19 severity (Chen et al., 2020a). However, the primary source both of the cytokine storm and the mechanisms behind the lymphocytopenia remain elusive (Li et al., 2020).

A growing body of evidence also points to dysregulation of innate immune cells of the granulomonocytic lineage in response to lung viral infections. A variety of human viruses, including the middle-east respiratory syndrome coronavirus (MERS-CoV), infects monocytes and macrophages to spread through the tissues (Al-Qahtani et al., 2017; Desforges et al., 2007; Nottet et al., 1996; Smith et al., 2004; Yilla et al., 2005). SARS-CoV-2 mRNA is detectable in lung monocytes/macrophages of severe COVID-19 patients (Bost, et al. 2020 Cell in press). However, the ability of SARS-CoV-2 to enter these cells in the peripheral blood and activate them directly remains unclear (Hinman et al., 1979; Okabe et al., 1985; Vuk-Pavlovic et al., 1989). Nevertheless, tissue damage induced by SARS-CoV-2 infection may lead to the release of pathogen and damage-associated molecular patterns that, in turn induce the activation and recruitment of inflammatory cytokine and chemokine producing innate immune cells in an amplifying loop (Ong et al. , in press, 2020; Liao et al. , 2020).

In this context, it remains unclear whether the recently described immune patterns associated with COVID19 pathophysiology are exacerbating the disease and/or could be used for rapid and accurate patient stratification for prognosis of mild to severe.

The switch into severe forms of the infection requires that approximately 20% of patients remain in hospital and about 5% require admission to intensive care. WHO estimates the mortality rate to be around 3.4% (data of March 2020). Therefore, there is a pressing urgency to identify at an early stage patients that are at risk of progressing to a severe COVID-19 form and/or of evolving into lymphocytopenia. This will allow an earlier, quicker and more effective management of these patients, ultimately allowing a decrease in the mortality rate.

### DETAILED DESCRIPTION OF THE INVENTION

The present invention fulfils this need. Indeed, the present Inventors have identified one of the mechanisms triggering the switch of the infection into the severe form. This mechanism is surprisingly based on the modulation of immune cells populations.

Indeed, the Inventors demonstrated that the severity and the outcome of the infection correlates with the level of non-classical monocytes CD14^{low}CD16⁺ monocytes, the level of HLA-DR^{low} classical monocytes, the level of CD16⁺CD10⁻CD101⁻CXCR4^{+/-} immature neutrophils, and the level of erythroid cells with an immunosuppressive cell genotype and phenotype.

More precisely, the Inventors have shown that the switch of COVID-19 to a severe disease is associated with 1) the extinction of the distinctive type I IFN response that releases CXCL8 secretion control, and a decrease in the IFNα2a plasma level; 2) a burst of circulating calprotectin that precedes the cytokine release syndrome; 3) the rapid clearance of non-classical monocytes from the peripheral blood and accumulation of HLA-DR^{low} classical monocytes; 4) changes in neutrophil subsets and 5) an emergency myelopoiesis that releases immature and dysplastic myeloid cells with an immune suppressive phenotype.

As shown in the examples below, the Inventors have shown that this rapid decrease in the fraction of CD14^{low} CD16⁺ non-classical monocytes as well as the accumulation of HLA-DR^{low} classical monocytes in COVID-19 severely infected patients is not observed in any other viral infections. These data show that these features are both specific features of COVID-19. Most importantly, the data show that this decrease of non-classical monocytes generates an unusual, yet highly characteristic biological signature of COVID-19 disease switch to an aggressive form. Their data also demonstrate that an increased release of CD16⁺CD10⁻CD101⁻ CXCR4^{+/-} immature neutrophils with an immunosuppressive cell genotype and phenotype is associated with COVID-19 disease switch to an aggressive form.

In addition, the present Inventors demonstrated that the decrease in non-classical monocyte fraction and/or the accumulation of HLA-DR^{low} classical monocytes and/or the accumulation of immature neutrophils can be implemented in a routine lab, for example by FACS, to detect patients at high risk of switching to severe disease.

In addition, the Inventors have observed a surprising burst of calprotectin in COVID-19 patients that are undergoing a switch toward a severe disease. They therefore propose to use calprotectin plasma level as a biomarker of COVID-19 switch to severe form, as well as for therapeutic target.

The detection of these biomarkers can be easily done by flow cytometry (FACS). With such a technology, it is easy to detect a decrease in the fraction of circulating non-classical monocytes as well as the accumulation of immature neutrophils with an immunosuppressive phenotype in the peripheral blood.

Thus, the present invention provides COVID-19 risk factor screening tools integrating blood non-classical monocytes, immature neutrophils and calprotectin level, for the stratification of the patients and their risk of evolving into a severe form. It also proposes to use therapeutics targeting calprotectin to alleviate severe COVID-19.

The invention proposes easy-to-implement assays that detect COVID-19 patients at high risk of severe outcome, thereby providing the first rapid and efficient tests to identify patients that are at high risk of switching to a severe form of the COVID disease.

### Definitions

Unless specifically defined, all technical and scientific terms used herein have the same meaning as commonly understood by a skill artisan in chemistry, biochemistry, cellular biology, molecular biology, and medical sciences.

The *Betacoronavirus* genus (β-CoVs or Beta-CoVs), comprising virus infecting animals and/or humans, is subdivided into four lineages designated as A, B, C and D:
- Lineage A also designated as subgenus *Embecovirus* includes HCoV-OC43 and HCoV-HKU1, virus able to infect various species;
- Lineage B also designated as subgenus *Sarbecovirus* includes SARS-CoV-1, SARS-CoV-2, and Bat SL-CoV-WIV1;
- Lineage C also designated as subgenus *Merbecovirus* includes Tylonycteris bat coronavirus HKU4 (BtCoV-HKU4), Pipistrellus bat coronavirus HKU5 (BtCoV-HKU5), and MERS-CoV, able to infect notably camels and humans;
- Lineage D also designated as subgenus *Nobecovirus* includes Rousettus bat coronavirus HKU9 (BtCoV-HKU9).

As used herein, the term **"betacoronavirus"** designates any virus belonging to the *Betacoronavirus* genus (β-CoVs or Beta-CoVs) within the *Coronaviridae* family, in particular any *betacoronavirus* belonging to one of the four lineages designated as A, B, C and D. It designates a betacoronavirus infecting animals (preferably a mammal) and/or humans. In particular, this designation includes the betacoronaviruses infecting human organisms selected from the group consisting of OC43, HKU1, SARS-CoV-1, SARS-CoV-2 and MERS-CoV.

**"SARS-CoV-2"** herein refers to Severe Acute Respiratory Syndrome Coronavirus 2. SARS-CoV-2 belongs to the species *Coronavirus,* in the genus *Betacoronavirus* and family *Coronaviridae.*

**"Viral infection due to a betacoronavirus"** or **"betacoronavirus infection"** designates the fact that cells of an organism have been infected by at least one *betacoronavirus,* the whole organism being said to suffer from said viral infection.

In particular, **"Viral infection due to SARS-CoV-2"** or **"SARS-CoV-2 infection"** or **"SARS-CoV2** infection" designates the fact that cells of an organism have been infected by the SARS-CoV-2 virus, the whole organism being said to suffer from said viral infection.
As used herein, the terms **"COVID disease"** or **"COVID"** or **"betacoronavirus disease"** or **"coronavirus disease"** mean the disease linked to (associated with) the infection with at least one betacoronavirus, as listed above. In particular, the terms **"COVID-19 disease"** or **"COVID-19"** or **"coronavirus disease 19"** mean the disease linked to (or associated with) the infection with (at least) the SARS-CoV-2 (Severe Acute Respiratory Syndrome Coronavirus 2).

Betacoronavirus disease, such as COVID-19 disease (SARS-CoV-2 infection), can manifest several forms, depending the severity of the symptoms/signs. Betacoronavirus disease, such as COVID-19 disease, can be asymptomatic in some people. It can induce a simple fever accompanied by cough in others. But betacoronavirus disease, such as COVID-19, can also cause acute respiratory distress and death. Thus, the clinical forms of betacoronavirus disease (such as COVID-19) as used herein are :
- An asymptomatic form;
- A mild form;
- A strong form;
- A severe form.

As used herein, an **"asymptomatic form"** or **"asymptomatic betacoronavirus disease"** or **"asymptomatic betacoronavirus infection" or "asymptomatic** COVID" or **"an asymptomatic COVID-19 form"** or **"an asymptomatic COVID-19"** is characterized by a subject who is a carrier for a disease or infection (e.g. COVID-19 and/or with SARS-CoV-2 infection) but experiences no symptoms. An asymptomatic disease is in contrast to a symptomatic disease. Advantageously, an **"asymptomatic COVID subject"** is a subject that is infected with at least on betacoronavirus but shows no symptom. Thus, an **"asymptomatic COVID-19 subject"** is a subject that is infected with SARS-CoV-2 but shows no symptom.

As used herein, a **"symptomatic form"** or **"symptomatic betacoronavirus disease"** or **"symptomatic betacoronavirus infection"** or **"symptomatic COVID"** is characterised by a subject who shows at least one symptom of a betacoronavirus disease. More particularly, a **"symptomatic COVID-19"** is characterised by a subject who shows at least one symptom of the COVID-19 disease. The COVID symptoms (such as COVID-19) comprise: dry cough, muscle pain, headache, fever, fatigue, loss of taste or smell.

A **"mild COVID form"** as used herein present is characterized by a subject who shows the first symptoms (or signs) of a COVID disease, as listed above. A **"mild COVID-19 form"** as used herein is characterized by a subject who shows the first symptoms (or signs) of a COVID-19 disease. Mild COVID symptoms (such as COVID-19) comprise e.g., mild dry cough, mild muscle pain, mild headache, mild fever, mild fatigue, loss of taste or smell.

2 to 7 days later, the subject may switch to a stronger betacoronavirus disease form. As used herein, a **"strong COVID form"** (e.g. **"strong COVID-19 form"**) is defined by strong respiratory symptoms such as difficulty breathing, lack of oxygen; other stronger symptoms such as fever, dry cough, aches and pains, nasal congestion, strong headache, conjunctivitis, sore throat, skin rash, discoloration of fingers or feet, or any combination thereof; as well as a deterioration of the general state of health with frequent diarrhoea, but also liver or urinary disorders, dizziness or neuromuscular problems; some of these symptoms may require hospitalisation. Most patients have an abnormal chest X-ray or CT scan within the first few days of illness, even in the absence of respiratory signs.

Severe cases progress to respiratory distress that requires hospitalisation and, in the worst cases, intensive care (including, but not limited to, oxygen therapy, assisted ventilation, etc.). Severe cases include subjects in critical condition and often culminates into multi-organ dysfunction. Thus, **"severe COVID form"** or **"critical COVID form"** or **"aggressive COVID form"** (including **"severe COVID-19 form"** or **"critical COVID-19 form"** or **"aggressive COVID-19 form"**) herein refers to subject having life-threatening symptoms (or signs) of COVID (e.g. COVID-19), comprising at least one selected from (but not limited to) : respiratory distress, lung disorders, liver disorders, kidney disorders, neuromuscular disorders, brain disorders, etc, that requires hospitalisation and/or intensive care (in intensive care units (ICU)). The severe COVID form, in particular severe COVID-19 form, is characterized by severe symptoms of COVID disease, in particular life-threatening respiratory symptoms. In the case of COVID-19, about 20% of patients switch to a severe form that requires hospitalisation and about 5% require admission to intensive care (according to WHO data of March 2020).

As used herein, a **"subject"** or an **"individual"** is an animal, preferably a mammal, including, but not limited to, human, dog, cat, cattle, goat, pig, swine, sheep and monkey. More preferably, the subject is a human subject. A human subject can be known as a patient. As used herein, **"subject in need"** refers to an animal, preferably a mammal, more preferably a human, that may suffer from, or susceptible to suffer from COVID (e.g. COVID-19), or that is suspected of suffering from COVID (e.g. COVID-19), or that has been diagnosed with COVID (e.g. COVID-19). As used herein, a **"COVID suffering subject"** refers to an animal, preferably a mammal, more preferably a human, that suffers from COVID and/or has been diagnosed with COVID and/or is infected with at least one betacoronavirus and/or suffers from at least one betacoronavirus infection. **"COVID-19 patients"** are patients (preferably human subjects) infected and diagnosed with the SARS-CoV-2. As used herein, a" **COVID-19 suffering subject"** refers to an animal, preferably a mammal, more preferably a human, that suffers from COVID-19 and/or has been diagnosed with COVID-19 and/or is infected with SARS-CoV2 and/or suffers from a SARS-CoV2 infection. **"COVID-19 patients"** are patients (preferably human subjects) infected with the SARS-CoV-2. Diagnosis of this infection can be done by known molecular means enabling to detect the presence of the virus in a biological sample of the subject.

The tested subject is preferably suffering from a mild COVID form, displaying at least one of the symptom defined above. It can also suffer from a strong COVID form, as defined above.

The inventors have observed that the methods of the invention remain reliable even if the tested subject also suffers from a comorbidity, such as obesity, diabete, asthma, cancer or cardiovascular disease.

A **"healthy subject"** or **"a healthy reference/control subject"** refers to an animal, preferably a mammal, more preferably a human, that is not suffering from COVID (e.g. COVID-19) or that is not suspected of suffering from COVID (e.g. COVID-19) or that has not been diagnosed with COVID (e.g. COVID-19). Advantageously, the **"healthy subject"** does not suffer from any disease, or has not been diagnosed with any disease.

A **"control subject"** or a **"reference subject"** is a subject that may either be a healthy subject or a subject suffering from a disease (preferably a COVID, in particular COVID-19) at a specific stage (e.g. an asymptomatic, or a mild, or a strong, or a severe form of COVID, such as an asymptomatic, or a mild, or a strong, or a severe form of COVID-19).

As used herein, the terms **"biological sample"** or **"sample"** refer to an entire organ or tissue of a subject or a portion thereof, or cells or cell components thereof, or a fraction or portion of tissue, organ or cell. "Biological sample" further refers to a homogenate, lysate or extract prepared from an entire organ or tissue of a subject or a portion thereof, or cells or cell components thereof, or a fraction or portion of tissue, organ or cell. Preferably, a "biological sample" is any tissue (preferably portions or fractions thereof) which may contain monocytes and/or neutrophils and/or calprotectins, including, but not limiting to, whole blood (e.g. non-purified, raw blood), blood plasma, or bone marrow. More preferably, the biological sample is a peripheral blood sample. Indeed, such a blood sample may be obtained by a completely harmless and non-invasive blood collection from the subject.

**"Prognosis"** herein means the prediction/determination/assessment of the risk of disease (or an ailment, or a condition) progression (or evolution, or development) in an individual. Prognosis includes the assessment of the future development of the subject's condition and the possible chances of cure. The prognosis can be determined on the basis of observations and/or measurements, carried out using various tools.

As used herein, **"stratification"** refers to the separation/classification of subjects into subgroups by disease severity/severity. The different subgroups include the subgroup of healthy subjects as well as different subgroups of subjects with a disease, classified according to the stage of disease progression/advancement (in the present case, mild, strong or severely infected patients).
**"Monitoring"** or **"Follow-up"** herein refers to the identification/assessment of the progression (or evolution, or development) of a disease (or an ailment, or a condition) in a subject. Monitoring may be carried out on the basis of observations and/or measurements, using different tools, at different time intervals. Intervals may be regular or irregular. Their frequency depends on the disease but also on the stage of disease progression. It can range from a few days (e.g. in case of severe/advanced/severe disease and/or rapidly progressing disease and/or exacerbation phase) to a few years (e.g. in case of early, mild or moderate disease and/or slowly progressing disease).

As used herein, **"determining the outcome"** or **"outcome determination"**, herein means the prediction/determination/assessment of the most probable evolution (or progression or development) of a disease (or an ailment, or a condition) in a subject. **"Determining the outcome"** of the disease thus includes at least assessing the next stages that are most likely to be undergone by the subject, in terms of probability. More specifically, **"determining the outcome of COVID"** includes, but is not limited to, the assessment of the probabilities (or chances), for a subject (suffering from COVID or not), of switching to a severe COVID form; and/or the assessment of the probabilities (or chances), for a subject (suffering from COVID or not), of having or progressing towards an asymptomatic COVID, or a mild form of COVID, or a strong form of COVID, or a severe form of COVID. In particular, **"determining the outcome of COVID-19"** includes, but is not limited to, the assessment of the probabilities (or chances), for a subject (suffering from COVID-19 or not), of switching to a severe COVID-19 form; and/or the assessment of the probabilities (or chances), for a subject (suffering from COVID-19 or not), of having or progressing towards an asymptomatic COVID-19, or a mild form of COVID-19, or a strong form of COVID-19, or a severe form of COVID-19.

As used herein, **"assessing the risk of switching to a severe form"** means the prediction/determination/assessment of the probabilities (or the chances) of exacerbation (or aggravation, or intensification) of a disease (or an ailment, or a condition) from an asymptomatic or mild form or strong form to a severe form. A **"severe form of a disease"** herein refers to a stage of a disease (or an ailment, or a condition) wherein the symptoms of the subject are severe and/or the survival of the subject is at risk, that is wherein the life of the subject is threatened and/or the subject is in a life-threatening state. In most cases, a "severe form of a disease" requires hospitalization of the subject and intensive care.

More specifically, **"assessing the risk of switching to a severe COVID form"** or **"predicting the risk of switching to a severe COVID form"** or **"determining the risk of switching to a severe COVID form"** means the prediction/determination/assessment of the probabilities (or the chances) of exacerbation of COVID from an asymptomatic form or mild form or strong form to a severe form. More specifically, **"assessing the risk of switching to a severe COVID-19 form"** or **"predicting the risk of switching to a severe COVID-19 form"** or **"determining the risk of switching to a severe COVID-19 form"** means the prediction/determination/assessment of the probabilities (or the chances) of exacerbation of COVID-19 from an asymptomatic form or mild form or strong form to a severe form.

As used herein, the terms **"prevent"** or **"preventing"** or **"prevention"** or **"prevention of the onset of a disease"** means the reduction of the risk of appearing, of developing or of amplifying for a disease, for the causes of a disease, for the symptoms of a disease, for the effects (or consequences, preferably adverse, deleterious effects/consequences) of a disease, or any combination thereof ; and/or delaying the onset, development or amplification of a disease, the causes of a disease, the symptoms of a disease, the effects (or consequences, preferably adverse, deleterious effects/consequences) of a disease, or any combination thereof. In the present case, "preventing COVID disease" comprises reducing the likelihood that the patient undergo the switch into a severe form.

As used herein, the terms **"treat"**, **"treating"**, **"treatment"** and the like mean the reduction, inhibition, amelioration, stabilization and/or disappearance of a disease (or an ailment, or a condition), of the causes of a disease, of the symptoms (or signs) of a disease, of the effects (or consequences, preferably adverse, deleterious effects/consequences) of a disease (e.g. COVID such as COVID-19, and/ or symptoms associated therewith), fighting the disease, or any combination thereof. It will be appreciated that, although not precluded, treating a disorder or condition does not require that the disorder, condition or symptoms associated therewith be completely eliminated. For examples, treating results in the reduction of at least one sign or symptom of the disease or condition. Treatment includes (but is not limited to) administration of a therapy, and may be performed either prophylactically, or subsequent or the initiation of a pathologic event. Treatment can require administration of a therapy more than once.

More specifically, **"treating a betacoronavirus infection"** or **"treating COVID"** refers to fighting at least one betacoronavirus infection in a human or animal organism. Advantageously, the rate of viral infection (infectious titre) in the organism will decrease, and the betacoronavirus will completely disappear from the organism within a shorter period of time than expected without treatment. The terms **"treating a betacoronavirus infection"** or **"treating COVID"** also refer to reducing/inhibiting/ameliorating/stabilizing/making disappear, the symptoms/signs associated with a betacoronavirus infection (respiratory syndrome, kidney failure, fever, etc.). More specifically, **"treating SARS-CoV-2"** or **"treating COVID-19"** refers to fighting the SARS-CoV-2 infection in a human or animal organism. Advantageously, the rate of viral infection (infectious titre) in the organism will decrease, and the SARS-CoV-2 will completely disappear from the organism within a shorter period of time than expected without treatment. The terms **"treating SARS-CoV-2"** or **"treating COVID-19"** also refer to reducing/inhibiting/ameliorating/stabilizing/making disappear, the symptoms/signs associated with the SARS-CoV-2 infection (respiratory syndrome, kidney failure, fever, etc.).

As used herein, a **"therapy"** or **"treatment"** includes, but is not limited to, molecule(s) and/or drugs (including any type of molecule or drug, such as chemical or biological compounds, antibody, antigen, gene therapy, cell therapy, immunotherapy, chemotherapy, any combination thereof, etc.), and/or other treatments (such as radio-therapy, immunotherapy, chemotherapy, surgery, endoscopy, interventional radiology, physical oncology, phototherapy, light therapy, ultrasound therapy, thermotherapy, cryotherapy, electrotherapy, electroconvulsive therapy, oxygen therapy, assisted ventilation, hydrotherapy massage, organ/tissue/fluid transplant, implant, and any combination thereof, etc.). A therapy can be administered through various administration modes. The skilled person knowns how to select the most appropriate administration mode(s) depending on the therapy, the disease and the subject to treat. For example, administration modes include, but are not limited to, as oral administration; administration by injection into a vein (intravenously, IV), into a muscle (intramuscularly, IM), into the space around the spinal cord (intrathecally), beneath the skin (subcutaneously, sc); sublingual administration; buccal administration; rectal administration; vaginal administration; ocular route; otic route; nasal administration; by inhalation; by nebulization; cutaneous administration, either topical or systemic; transdermal administration.

As used herein, **"selecting a therapy"** or **"selecting a treatment"** or **"selecting a drug"** refers to the process of selecting (choosing, or deciding for, or opting for) the most appropriate therapy for a subject, in view of the symptoms (or signs) detected (observed and/or measured) in the subject, and/or in view of the subject himself, and the general knowledge in the medical field (preferably the medical field closest to the disease). Selecting a therapy include selecting the most appropriate therapy and may include also selecting the most appropriate administration mode and/or the most appropriate posology.

As used herein, the terms **"assessing the efficacy of a therapy"** or **"assessment of treatment efficacy"** refers to the determination of the clinical status of a subject undergoing treatment (i.e. receiving a therapy). The treatment may be preventive, for example in the case of predisposition to a disease, or it may be curative, for example in the case of a diagnosed disease. For example, the effectiveness of the treatment can be assessed by determining the condition of the subject at different time intervals. In particular, the condition of the subject can be assessed before the first dose of treatment and then at regular (or irregular) intervals after the first dose (e.g. after each new dose of treatment). A comparison of the subject's condition at these different intervals can then be made to identify any changes. The patient's condition can be assessed on the basis of observations and/or measurements made with different tools.

The terms **"quantifying"** or **"quantification of a value (or a level, or a quantity)"** or **"measuring"** or **"measurement of a value (or a level, or a quantity)"** or **"measurement of a quantity"** herein means the act of evaluating a quantity (also called a value or a level) of an object, based on its relationship to a quantity of the same species, taken as a unit and as a reference, using a measuring device. For example, where the quantity of specific cell is to be determined, the reference unit may be one cell. The quantity (or value, or level) can be a relative or an absolute quantity (or value, or level). **"Determination of a value (or a level, or a quantity)"** means the action of evaluating or calculating a quantity (also called value or level) from a value or quantity or signal measured and/or detected using a measuring device and/or detecting device.

The terms **"reference value",** or **"reference quantity"** or **"reference level"** as used herein, refers to the value (or quantity, or level) of a parameter or a biomarker indicating the state of a subject with respect to a specific disease (or ailment, or condition). The suitable reference level of a parameter or a biomarker can be quantified, or determined, or measured by detecting the parameter/biomarker in several suitable reference subjects. Such reference levels can be adjusted to specific subject populations. The reference value or reference level can be an absolute value; a relative value; a value that has an upper or a lower limit; a range of values; an average value; a median value, a mean value, or a value as compared to a particular control or baseline value. A reference value can be based on an individual sample value such as, for example, a value obtained from a sample from the subject being tested, but at an earlier point in time. The reference level can be based on a large number of samples, such as from population of subjects of the chronological age matched group, or based on a pool of samples including or excluding the sample to be tested.

Depending on the context, the reference level corresponds to the value of a parameter (or a biomarker) quantified, or determined, or measured, on a sample from a healthy reference subject; or to the average of the values (mean value) of a parameter (or a biomarker) quantified, or determined, or measured, on different samples of the same healthy reference subject (values quantified/determined/measured on samples taken at separate time intervals from the same healthy reference subject); or to the average of the values (mean value) of a parameter/biomarker determined/measured on the same sample from a healthy reference subject but at separate time intervals; or to the average of the values (or mean value) of a parameter/biomarker quantified/determined/measured on samples from several healthy reference subjects (at least two healthy reference subjects); or to the value of a parameter/biomarker quantified/determined/measured on a sample of a reference subject at a known stage of the disease; or to the average of the values (or mean value) of a parameter/biomarker quantified/determined/measured on different samples from the same reference subject at a known stage of the disease (values quantified/determined/measured on samples taken at separate time intervals from the same reference subject); or to the average of the values (or mean value) of a parameter/biomarker quantified/determined/measured on the same sample from the same reference subject at a known stage of the disease but at separate time intervals; or to the average of the values (or mean value) of a parameter/biomarker quantified/determined/measured on samples from several reference subjects at the same known stage of the disease (at least two reference subjects at the same known stage of the disease).

As used herein, a **"biomarker"** or a **"biological marker"** is a measurable indicator of a biological state, condition, ailment, disease and form/stage thereof. A biomarker can be a substance whose detection indicates a particular disease state, for example, the presence of an antigen or cellular receptor (or a cell associated therewith) may indicate an infection. It can also be used to optimize a treatment/therapy, and to evaluate the likelihood of benefiting or the benefice from a specific therapy, and can serve a role in narrowing down diagnosis. A biomarker can also provide information about the subject overall outcome, regardless of therapy. More specifically, a biomarker indicates a change in expression or state of a protein and/or a cell that correlates with the risk or progression of a disease, or with the susceptibility of the disease to a given treatment. The biomarker can be a molecular biomarker, a cellular biomarker or an imaging biomarker.

The term **"monocytes"** herein refers to a type of leukocytes (representing 2 to 10% of circulating leukocytes, 0.1 to 1 x 10⁹ / L in human peripheral blood) produced by the bone marrow from hematopoietic stem cells. They circulate in the blood, typically between one and 7 days, and most of them migrate into tissues where they differentiate, generating so-called "monocyte-derived cells" with a macrophage phenotype. Monocytes belong to the family of the peripheral mononuclear cell of the blood (PBMCs). PBMCs are a critical component in the immune system to fight infection and adapt to intruders. These cells can be extracted from whole blood using ficoll, a hydrophilic polysaccharide that separates layers of blood, which will separate the blood into a top layer of plasma, followed by a layer of PBMCs and a bottom fraction of polymorphonuclear cells (such as neutrophils and eosinophils) and erythrocytes.

Monocytes are fairly variable in size and appearance, but they show common expression of a number of markers, including cell surface antigens (or receptors). Three subsets of monocytes can be identified in human blood, based on the expression of the CD14 and CD16 markers: a) the "classical" monocyte or MO1 (as used herein **"classical monocyte"**) is characterized by high level expression of the CD14 cell surface receptor and no expression of CD16 cell surface receptor (CD14⁺/CD16⁻ monocyte or CD14^{high}/CD16^{low}), b) the "non-classical" monocyte or MO3 (as used herein **"non-classical monocytes"**) shows low level or no expression of CD14 with additional co-expression of the CD16 receptor (CD14⁻/CD16⁺ monocyte or CD14^{low} / CD16^{high}), and c) the "intermediate" monocyte or MO2 (as used herein **"intermediate monocyte"**) with high level expression of CD14 and the same level of CD16 expression as the MO3 monocytes (CD14⁺/CD16⁺ monocytes or CD14^{high}/CD16^{high}) (Zawada et al., Blood 118(12):e50-61, 2011; Ziegler-Heitbrock et al., Blood, 116(16): e74-80, 2010; Wong et al., Blood, 118(5): e16-31, 2011).

Thus, most of the monocytes, like classical monocytes, express the **"cluster of differentiation CD14"** or **"CD14"** or **"CD14 molecule/antigen".** The amino acid sequence of reference for the human CD14 is the NCBI sequence referenced under NP_000582.1. Numerous antibodies against human CD14 are commercially available. CD14 is expressed at the surface of the monocytic cells and, at 10 times lesser extent, of the neutrophils.

The **"cluster of differentiation CD16"** or **"CD16"** or **"CD16 molecule/antigen"** is the low affinity receptor for the Fc part of IgG (therefore also known as FcγRIII), is a glycoprotein expressed in monocytes, and also in NK cells and neutrophils. Two isoforms (A and B) exist. In human, the isoform A has the NCBI reference sequence NP_000560.5 and the isoform B has the NCBI reference sequence NP_001231682.1. Several monoclonal antibodies have been produced against the isoforms A and B of CD16 / FcγRIII and the corresponding epitopes have been localized on these proteins (see e.g. Fleit et al., Clin Immunol Immunopathol., 59(2): 222-235, 1991; Fleit et al., Clin Immunol Immunopathol., 62(1 Pt 1): 16-24, 1992; Tamm A. et al., J Immunol., 157(4): 1576-1581, 1996). Antibodies against CD16 are available commercially.

Monocytes are easily identified by specific antigens (including cell surface antigens, e.g. CD14 or CD16) combined with morphometric characteristics (e.g. size, shape, granulometry, etc.).

Monocytes, including classical monocytes, can also express the HLA-DR cell surface receptor. These monocytes are referred to as HLA-DR⁺ monocytes. HLA-DR is an MHC (major histocompability complex) class II cell surface receptor encoded by the human leukocyte antigen complex on chromosome 6 region 6p21.31. The complex of HLA-DR (Human Leukocyte Antigen - DR isotype) and peptide, generally between 9 and 30 amino acids in length, constitutes a ligand for the T-cell receptor (TCR). The primary function of HLA-DR is to present peptide antigens, potentially foreign in origin, to the immune system for the purpose of eliciting or suppressing T-(helper)-cell responses that eventually lead to the production of antibodies against the same peptide antigen. Antigen presenting cells (macrophages, B-cells and dendritic cells) are the cells in which DR are typically found. HLA-DR is an αβ heterodimer, cell surface receptor, each subunit of which contains two extracellular domains, a membrane-spanning domain and a cytoplasmic tail. The reference amino acid sequence for human HLA-DR alpha and beta chains can be represented by the NCBI accessions AAA36275.1 or AAA59785.1 or AAA36302.1 (alpha chain) and AAA58651.1 or AAA59816.1 (beta chain). As used herein, a "**HLA-DR**^{low} **classical monocyte"** or "**HLA-DR**^{low} **monocyte"** is a classical monocyte (i.e. a CD14⁺/CD16⁻ monocyte) that present a low expression level of HLA-DR, as compared with the expression measured at the surface of circulating classical monocytes in heathy subjects.

**"Granulocytes"** are a type of leukocytes characterized by the presence of granules in their cytoplasm. The types of these cells are neutrophils, eosinophils, and basophils. Antigens expressed by granulocytes include, but are not limited to CD24, CD15, CD16, etc.

**"Neutrophils",** also known as **"neutrocytes"** or **"heterophils",** are the most abundant type of granulocytes and the most abundant (60% to 70%) type of white blood cells in most mammals. They form an essential part of the innate immune system, with their functions varying in different animals. These specialized innate cells require constant replenishment from proliferative bone marrow precursors as a result of their short half-life. Neutrophils undergo a process called chemotaxis, which allows them to migrate toward sites of infection or inflammation. Neutrophils have a variety of specific receptors, including ones for complement, cytokines like interleukins and IFN-γ, chemokines, lectins, and other proteins. They also express receptors to detect and adhere to endothelium and Fc receptors for opsonin. The antigens expressed by neutrophils include, but are not limited to, CD10, CD11b, CD15, CD16, CD35, CD64, CD66a, CD66b, CD101, CD111, CD177, etc. They can be distinguished from other white blood cells by the expression of CD177 and/or CD15⁺CD66b⁺.

As used herein, "immature neutrophils" are neutrophils characterized by the absence of expression of CD101 and/or CD10 (in other words, immature neutrophils are CD101-CD10-neutrophils). Immature neutrophils can also be characterized by the low expression of CD16 (in other words, immature neutrophils are also CD16low neutrophils). As used herein, "immature neutrophils" thus means CD101-CD10- and/or CD16low neutrophils. CD16 is usually easier to detect than CD101 and/or CD10. Therefore, it is particularly advantageous to use CD16 to detect/select/quantify immature neutrophils (thereby selecting/quantifying CD16low neutrophils (preferably CD101-CD10-CD16low neutrophils). Advantageously, immature neutrophils also exhibit an immunosuppressive genetic profile (indicated for example, by the up-regulation of immunosuppressive genes such as TSPO, SOD2, ITGB2, ICAM1, AIF1, IL1B, RAC1, RAB27A, ZC3H12A, GNAS). Three neutrophil subsets have been identified : a committed proliferative neutrophil precursor (preNeu) which differentiates into non-proliferating immature neutrophils and mature neutrophils. preNeu require the C/EBPε transcription factor for their generation, and their proliferative program is substituted by a gain of migratory and effector function as they mature. preNeus expand and immature neutrophils are recruited to the periphery under microbial stress. These cells can demonstrate an immunosuppressive phenotypic profile (indicated for example by Evrard M et al, Immunity 2018; Tamadaho RSE et al, 2019).

**"CD101"** or **"Cluster of Differentiation 101",** also known as **"Immunoglobulin superfamily member 2"** or **"IGSF2"** is a type I transmembrane glycoprotein. The amino acid sequence of human CD101 is available under NCBI accession numbers NP_001243038.1 or NP_001243035.1 or NP_004249.2.

**"CD10"** or **"Cluster of Differentiation 10"**, also known as **"Neprilysin"**, or **"membrane metallo-endopeptidase (MME)"** or **"neutral endopeptidase (NEP)"** or **"common acute lymphoblastic leukemia antigen (CALLA)"** is a zinc-dependent metalloprotease that cleaves peptides at the amino side of hydrophobic residues and inactivates several peptide hormones and degrades the amyloid beta peptide. It is synthesized as a membrane-bound protein. The amino acid sequence of human CD10 is available under NCBI accession numbers NP_009220.2 or NP_000893.2 or NP_009218.2 or NP_001341571.1 or NP_001341572.1 or NP_009219.2 (isoform a) or NP_001341573.1 (isoform b).

Immature neutrophils can express **"CD184"** (**"cluster of differentiation 184"**) or not. **"CD184"** is also named **"CXCR4"** for C-X-C chemokine receptor type 4, or **"fusin"**, is an alpha-chemokine receptor specific for stromal-derived-factor-1 (SDF-1 also called CXCL12). The amino acid sequence of human CD10 is available under NCBI accession numbers CAA12166.1. Thus, immature neutrophils are herein referred to as CD101⁻CD10⁻CXCR4^{+/-} neutrophils.

**"T cells"** or **"T lymphocytes"** are a type of lymphocyte that plays a central role in cell-mediated immunity. They can be distinguished from other lymphocytes, such as B cells and natural killer cells (NK cells), by the presence of a T-cell receptor (TCR) on the cell surface. Other antigens expressed by T lymphocytes include, but are not limited to CD2, CD3, etc.

**"B cells"** or **"B lymphocytes"** are a type of lymphocyte in the humoral immunity of the adaptive immune system. They can be distinguished from other lymphocytes, such as T cells and natural killer cells (NK cells), by the presence of a protein on the B cells outer cell surface known as a B-cell receptor (BCR). Other antigens expressed by B lymphocytes include, but are not limited to CD24, CD19, etc.

**"Natural killer cells"** or **"NK cells"** are a type of cytotoxic lymphocytes that kill cells by releasing small cytoplasmic granules of proteins called perforin and granzyme. They constitute the third kind of cells differentiated from the common lymphoid progenitor generating B and T lymphocytes. Antigens expressed by NK cells include, but are not limited to CD2, CD56, etc.

As used herein, **"quantifying the level of monocytes"** means evaluating (determining, measuring, counting) the level or the quantity (or number) of monocytes present in a biological sample. More specifically **"quantifying the level of CD14^{low}/CD16⁺ monocytes of a monocyte population"** or **"quantifying the fraction of CD14^{low}/CD16⁺ monocytes"** or **"quantifying the fraction of CD14^{low}/CD16⁺ non-classical monocytes"** or **"quantifying the level of non-classical monocytes"** means evaluating (determining, measuring, counting) the quantity (or number) of non-classical monocytes (CD14^{low}/CD16⁺ monocytes) present in a biological sample, with respect to the total number of monocytes in the sample. Advantageously, the biological sample may have undergone a pre-selection treatment of monocyte selection (monocyte purification), in order to reduce or eliminate contaminant cells and facilitate the quantification of non-classical monocytes (CD14^{low}/CD16⁺). When flow cytometry is used, it is possible to make this pre-selection to select the monocyte population only when data are analysed, so as to easily determine the fraction of non-classical monocytes within said total monocyte population by gating on the appropriate population labelled with the appropriate markers.

More specifically **"quantifying the level of HLA-DR^{low} monocytes of a monocyte population"** or **"quantifying the level of HLA-DR^{low} classical monocytes of a monocyte population"** or **"quantifying the fraction of HLA-DR^{low} monocytes"** or **"quantifying the fraction of HLA-DR^{low} classical monocytes"** means evaluating (determining, measuring, counting) the quantity (or number) of classical monocytes (CD14⁺/CD16⁻ monocytes) expressing low levels of HLA-DR present in a biological sample, with respect to the total number of monocytes present in the sample. Advantageously, the biological sample may have undergone a pre-selection treatment of monocyte selection (monocyte purification), in order to reduce or eliminate contaminant cells and facilitate the quantification of classical monocytes (CD14⁺/CD16⁻ monocytes). When flow cytometry is used, it is possible to make this pre-selection to select the HLA DRl^{ow} monocyte population only when data are analysed, so as to easily determine the fraction of these monocytes within said total monocyte population by gating on the appropriate population labelled with the appropriate markers.

As used herein, **"quantifying the level of neutrophils"** means evaluating (determining, measuring, counting) the level or the quantity (or number) of neutrophils present in a biological sample. More specifically **"quantifying the fraction of immature CD101⁻CD10⁻ neutrophils of a neutrophil population"** or **"quantifying the fraction of immature CD101⁻ CD10⁻ neutrophils"** or **"quantifying the fraction of CD101⁻CD10⁻ neutrophils" or "quantifying the fraction of immature neutrophils"** or **"quantifying the fraction of CD16^{low} neutrophils"** means evaluating (determining, measuring, counting) the quantity (or number) of immature neutrophils (CD101⁻CD10⁻ and/or CD16^{low} neutrophils) present in a biological sample, with respect to the total number of neutrophils in the sample. Advantageously, the biological sample may have undergone a pre-selection treatment of neutrophil selection (neutrophil purification), in order to reduce or eliminate contaminant cells and facilitate the quantification of immature neutrophils (CD101⁻CD10⁻ and/or CD16^{low}). When flow cytometry is used, it is possible to make this pre-selection to select the neutrophil population only when data are analysed, so as to easily determine the fraction of the target neutrophils within the total neutrophil population, by gating on the appropriate population labelled with the appropriate markers.

Monocytes, neutrophils, other granulocytes, T cells, B cells, natural killer cells and other cells may be detected, identified, quantified, sorted, and any combination thereof, using any technique known in the art. Expression of cell surface receptors and/or cell surface antigens (on any cells, including blood and/or plasma and/or bone marrow cells, such as monocytes, neutrophils, lymphocytes and leukocytes) may be notably assessed (measured, or identified, or detected) using well known analytical technologies such as cell membrane staining using biotinylation or other equivalent techniques followed by immunoprecipitation with specific antibodies, flow cytometry, western blot, ELISA, ELISPOT, antibodies microarrays, immunoprecipitation, immunohistology, dot blot, protein microarray, or tissue microarrays coupled to immunohistochemistry. Other suitable techniques include FRET or BRET, single cell microscopic or histochemistery methods using single or multiple excitation wavelength and applying any of the adapted optical methods, such as electrochemical methods (voltametry and amperometry techniques), atomic force microscopy, and radio frequency methods, e.g. multipolar resonance spectroscopy, confocal and non-confocal, detection of fluorescence, luminescence, chemiluminescence, absorbance, reflectance, transmittance, and birefringence or refractive index (e.g., surface plasmon resonance, ellipsometry, a resonant mirror method, a grating coupler waveguide method or interferometry), cell ELISA, radioisotopic, magnetic resonance imaging, analysis by polyacrylamide gel electrophoresis (SDS-PAGE); HPLC-Mass Spectroscopy; Liquid Chromatography/Mass Spectrometry/Mass Spectrometry (LC-MS/MS)). For example, when flow cytometry is used, forward scatter and side scatter information help to identify the monocyte population among other blood cells. Preferably, the cells (including blood and/or plasma and/or bone marrow cells, such as monocytes, neutrophils, lymphocytes and leukocytes, as defined above) are identified, selected, sorted, quantified (and any combination thereof) by flow cytometry.

The calprotectin levels (such as S100A8/S100A9 calprotectin) and inflammatory cytokines and chemokines levels/quantities can also be determined/quantified/measured using any of the above analytical techniques including flow cytometry and ELISA.

As used herein, **"flow cytometry"** or "FCM" is a technique used to detect and measure physical and chemical characteristics of a population of cells or particles. FCM is a useful tool for simultaneously measuring multiple physical properties of individual particles (such as cells, biomarkers, proteins, protein complexes, etc.). Cells pass single-file through a laser beam. As each cell passes through the laser beam, the cytometer records how the cell or particle scatters incident laser light and emits fluorescence. Using a flow cytometric analysis protocol, one can perform a simultaneous analysis of surface molecules at the single-cell level. Advantageously, the use of fluorescent agents or fluorochromes linked or attached to an antibody or antiserum able to specifically recognize a molecule or particle (such as a cell surface molecule (e.g. CD45, CD14, CD16, HLA-DR, CD10, CD101, etc.) attached to a cell or portion thereof, a biomarker, a protein, a protein complex (e.g. calprotectin), etc., enables the flow cytometer to sort the molecules/particles on the basis of size, granularity and fluorescent light. Thus, the amount of information obtained from a single sample can be further expanded by using multiple fluorescent reagents. The information gathered by the flow cytometer can be displayed as any combination of parameters selected by the skilled person. For example, the flow cytometer can be configured to provide information about the relative size (forward scatter or "FSC"), granularity or internal complexity (side scatter or "SSC"), and relative fluorescent intensity of the cell sample. The detection of the biomarkers, the cell surface antigens, the cell surface receptors, the monocytes, the neutrophils, the other granulocytes, T cells, B cells, natural killer cells, other cells, and any combination thereof, is performed by an exclusion gating strategy by flow cytometry. This combination is particularly useful to detect, identify, quantify, and sort cells, such as monocytes, neutrophils, granulocytes, T cells, B cells, natural killer cells and other cells. The detection can be practiced with any antibody or antiserum detecting (or recognizing specifically) the antigens or receptors expressed (and preferably exposed at the cell surface) by the monocytes, the neutrophils, or by the contaminating cells. Advantageously, the cells present in the biological sample to analyse/quantify/sort are contacted with labelled antibodies (preferably labelled with a fluorescent agent or a fluorochrome), each of which recognizing a specific antigen or receptor expressed (and preferably exposed at the cell surface) by the monocytes, by the neutrophils, or by one or more of the contaminant cells (e.g., CD45, CD14, CD15, CD56, CD2, CD24, CD16, CD19, CD3, and any combination thereof). The sample is then analysed by flow cytometry.

As used herein, **"contaminant cells"** or **"contaminant white blood cells"** refer to the cells or the white blood cells which are present in the biological sample (advantageously a blood sample) of the subject and which are not monocytes and/or neutrophils (depending on the targeted biomarkers). Such contaminant cells include eosinophils, basophils, and lymphocytes, e.g., T cells, NK cells, B cells, but also precursors of these cell types.

The existence of markers (including cell surface receptors/antigens) which are specific for each of the contaminant cell types enables the identification of these cells in the blood sample of the subject. Identified contaminant cells can then be removed from the sample (i.e., physically) or from the analysis (i.e., by retaining only the data pertaining to the monocyte population for the analysis), so that the study then only focuses on the monocyte population. In this respect, although any of the above-mentioned analytical techniques can be used to identify the said contaminant white blood cells, flow cytometry is particularly adapted for this task, since it enables the skilled person to eliminate the contaminants and analyse the monocyte population with minimal effort. In particular, flow cytometry (especially exclusion gating by flow cytometry) can be performed with antibodies specific for well-known antigens expressed by granulocytes (CD24, CD15, CD16), T lymphocytes (CD2, CD3), B lymphocytes (CD24, CD19), and/or NK cells (CD2 and/or CD56), so that to discard these contaminant cells and retain only monocytes and/or neutrophils (depending on the targeted biomarkers).

Using anti-HLA-DR, anti-CD10, anti-CD101, anti-CD15, anti-CD56, anti-CD2 or anti-CD24 antibodies therefore enables to detect and thus exclude the cells expressing CD2, CD10, CD101, CD15, CD56 and CD24 proteins, notably the CD2⁺ T lymphocytes, the CD2⁺ NK cells, the CD56⁺ NK cells, the CD24⁺ immature granulocytes as well as the CD15⁺ granulocytes, (if needed) the HLA-DR⁺ monocytes and/or (in needed) the CD10⁺CD101⁺ cells. The detection can be practiced with any antibody or antiserum detecting (or recognizing specifically) the antigens expressed by the monocytes or by the contaminating cells. Using anti-CD16 antibody enables to detect and thus exclude or conversely select (depending on the targeted biomarkers) the cells expressing CD16, notably CD16⁺ neutrophils and/or CD16⁺ non-classical monocytes and/or CD16⁻ classical monocytes, depending on the need. The detection can be practiced with any antibody or antiserum detecting (or recognizing specifically) the antigens expressed by the monocytes or by the contaminating cells.

Advantageously, each of the antibodies (e.g., anti HLA-DR, antiCD10, antiCD101, anti-CD15, anti-CD56, anti-CD2, anti-CD24, and/or anti-CD16 antibodies) is labelled with a specific fluorochrome or fluorescence agent (as used herein : **"labelled antibody"**), enabling the cytometer to identify the contaminant cells carrying the antigen recognized by said antibody, and thus the selection of the cells which do not carry the antigen. The fluorochromes which can be used are well known in the art. They include such fluorochromes as e.g., PE, APC, PE-Cy5, Alexa Fluor 647, PE-Cy-7, PerCP-Cy5.5, Alexa Fluor 488, Pacific Blue, FITC, AmCyan, APC-Cy7, PerCP, and APC-H7.
The identification of the various contaminant cells by flow cytometry can be performed sequentially or simultaneously. Preferably, the identification of the various contaminant cells in the sample is performed simultaneously.

As used therein, **"cluster of differentiation CD45"** or **"CD45 molecule/antigen"** or **"CD45"** is a single chain integral membrane protein, comprising at least 5 isoforms, ranging from 180 to 220 kDa. They are generated by alternative splicing combinations of three exons (A, B, and C) of the genomic sequence. The non-restricted CD45 antigen, Leucocyte Common Antigen (LCA) consists of an extracellular sequence, proximal to the membrane, which is common to all CD45 isoforms. All the monoclonal antibodies that belong to the CD45 cluster react with this part of the antigen and are able to recognize all CD45 isoforms. These isoforms have extra-cytoplasmic sequences ranging from 391 to 552 amino acids long, with numerous N-linked carbohydrate attachment sites. The cytoplasmic portion contains two phospho-tyrosine-phosphatase domains. Cells expressing CD45 at their surface are all human leucocytes (more precisely, lymphocytes, eosinophils, monocytes, basophils and neutrophils, with different level of expression). This cluster of differentiation is however absent from erythrocytes and platelets. Expression of cell surface CD45 on monocytes may be assessed using specific antibodies, in particular using well known technologies such as cell membrane staining using biotinylation (or other equivalent techniques), followed by immunoprecipitation with specific antibodies, flow cytometry, western blot, ELISA or ELISPOT, antibodies microarrays, or tissue microarrays coupled to immunohistochemistry. Preferably, the expression of cell surface CD45 is detected by flow cytometry.

The sequences of the **"cluster of differentiation CD56", "cluster of differentiation CD2"** and **"cluster of differentiation CD24"** are well known in the art, and can be retrieved under the NCBI accession numbers NP_000606, NP_001758, and NP_037362, respectively.

The **"cluster of differentiation CD15"** is the fucosyltransferase 4 (alpha (1,3) fucosyl transferase). In human, it has the sequence NP_002024. Cells expressing CD15 are for example granulocytes.

As used herein, a cell **"expresses CD45"** (or CD56, or CD15 or CD16 or CD2 or CD24 or CD14, etc.) if CD45 (or CD56 or CD15 or CD16 or CD2 or CD24 or CD14, etc.) if said marker is present at a significant level on its surface (such a cell being also defined as a "CD45⁺ cell", or a "CD56⁺ cell", or a "CD15⁺ cell", a "CD16⁺ cell", a "CD2⁺ cell" or a "CD24⁺ cell", etc., respectively). In particular, a cell expresses CD45 (or CD56 or CD15 or CD16 or CD2 or CD24, etc.) if the signal associated to surface CD45 (or CD56 or CD15 or CD16 or CD2 or CD24, etc.) staining (e.g. obtained with an antibody anti-CD45 coupled to a fluorescent marker) which is measured for said cell is superior to the signal corresponding to the staining of one cell being known as not expressing CD45 (or CD56 or CD15 or CD16 or CD2 or CD24, etc.). Preferably, CD45⁺ cells (or CD56⁺ cells, CD15⁺ cells, CD16⁺ cells, CD2⁺ cells, CD24⁺ cells, etc.) are such that the ratio between the surface CD45- (or CD56- or CD15- or CD16- or CD2- or CD24-, etc.) -associated signal measured for said cells and the surface CD45- (or CD56- or CD15- or CD16- or CD2- or CD24-, etc.) -associated signal measured for cells being known as expressing CD45 (or CD56 or CD15 or CD16 or CD2 or CD24, etc.) is superior or egal to 1. Cells expressing CD45 (or CD56 or CD15 or CD16 or CD2 or CD24, etc.) at their surface are well known in the art. Cells expressing CD56 include NK cells, while cells expressing CD2 are, for example, T lymphocytes and cells expressing CD24 are for example B lymphocytes. Cells that do not express CD56 are for example B lymphocyte.

In particular, as used herein, a cell **"expresses CD16"** if CD16 is present at a level on its surface (such a cell being also defined as a **"CD16⁺ cell"**). In particular, a cell expresses CD16 if the signal associated to surface CD16 staining (e.g. obtained with an antibody against CD16 coupled to a fluorescent marker) which is measured for said cell is clearly distinct and higher than the signal corresponding to the same staining of at least one cell being known as not expressing CD16, such as B lymphocytes. In other terms, the ratio between the surface CD16-associated signal measured for said cell and the surface CD16-associated signal measured for at least one cell being known as not expressing CD16 (e.g., a B lymphocyte) is superior to 10 (preferably superior to 100). On another hand, a cell is said to be **"CD16^{low}"** (or CD16- or CD16⁻ ) if the signal associated to surface CD16 staining (e.g., obtained with an antibody anti-CD16 coupled to a fluorescent marker) which is measured for said cell is similar or identical to the signal corresponding to the same staining of at least one cell being known as expressing low levels of CD16 (e.g., a B lymphocyte), or if the signal associated to surface CD16 staining is clearly distinct and lower than the CD16-associated signal measured for at least on cell being known as expressing high level of CD16 (such as a normal neutrophil). Preferably, CD16^{low} cells are such that the ratio between the surface CD16-associated signal measured for these cells and the surface CD16-associated signal measured for at least one cell being known as highly expressing CD16 is below 1/10. Cells that express high levels of CD16 at their surface are well known in the art. They are for example NK cells and neutrophils.

As used herein, a cell **"expresses CD14"** if CD14 is present at a significant level at its surface (such a cell being also defined as a **"CD14⁺ cell"** or "CD14^{high} cell"). In particular, a cell expresses CD14 if the signal associated to surface CD14 staining (e.g., obtained with an antibody anti-CD14 coupled to a fluorescent marker) which is measured for said cell is similar or identical to the signal corresponding to the same staining of at least one cell being known as expressing CD14. Preferably, CD14⁺ cells are such that the ratio between the surface CD14-associated signal measured for these cells and the surface CD14-associated signal measured for cells being known as not expressing CD14 is superior to 10. Cells that do not express CD14 at their surface are well known in the art. They are for example T lymphocytes. On another hand, a cell is said to be **"CD14^{low}"** (or CD14- or CD14⁻) if the signal associated to surface CD14 staining (e.g., obtained with an antibody anti-CD14 coupled to a fluorescent marker) which is measured for said cell is similar or identical to the signal corresponding to the same staining of at least one cell being known as expressing low levels of CD14. Cells expressing low levels of CD14 can be neutrophils or if the signal associated to surface CD14 staining is clearly distinct and lower than the CD14-associated signal measured for at least on cell being known as expressing high level of CD14 (such as a classical and intermediate monocytes). Preferably, CD14^{low} cells are such that the ratio between the surface CD14-associated signal measured for these cells and the surface CD14-associated signal measured for at least one cell being known as highly expressing CD14 is below about 1/10. Cells that expresses high levels of CD14 at their surface are well known in the art. They are for example classical and intermediate monocytes.

A cell is said to be **"CD10⁻"** or "CD10^{low}" if the signal associated to surface CD10 staining (e.g., obtained with an antibody anti-CD10 coupled to a fluorescent marker) which is measured for said cell is lower than the signal corresponding to the same staining of at least one cell being known as expressing CD10 (e.g. a normal neutrophil). Preferably, CD10⁻/ CD10^{low} cells are such that the ratio between the surface CD10-associated signal measured for these cells and the surface CD10-associated signal measured for at least one cell being known as not expressing CD10 is of about 1 or less. Preferably, the surface CD10-associated signal of the target cells is compared to an average surface CD10-associated signal measured on a population of cells being known as expressing CD10, e.g. normal neutrophils.

A cell is said to be **"CD101⁻"** or "CD101^{low}" if the signal associated to surface CD101 staining (e.g., obtained with an antibody anti-CD101 coupled to a fluorescent marker) which is measured for said cell is lower than the signal corresponding to the same staining of at least one cell being known as expressing CD101 (e.g. a normal neutrophil). Preferably, CD101⁻ cells are such that the ratio between the surface CD101 -associated signal measured for these cells and the surface CD101 -associated signal measured for at least one cell being known as not expressing CD101 is of about 1 or less. Preferably, the surface CD101 -associated signal of the target cells is compared to an average surface CD101-associated signal measured on a population of cells being known as expressing CD101, e.g normal neutrophils.

A cell is said to be **"HLA-DR^{low}"** if the signal associated to surface HLA-DR staining (e.g., obtained with an antibody anti-HLA-DR coupled to a fluorescent marker) which is measured for said cell is lower than the signal corresponding to the same staining of at least one cell being known as expressing normal levels of HLA-DR. Cells expressing normal levels of HLA-DR are classical monocytes. Preferably, HLA-DR^{low} cells are such that the ratio between the surface HLA-DR-associated signal measured for these cells and the surface HLA-DR -associated signal measured for at least one cell being known as not expressing HLA-DR is of about 1 or less, and the ratio between the surface HLA-DR -associated signal measured for these cells and the surface HLA-DR-associated signal measured for at least one cell being known as expressing high levels of HLA-DR is lower than 1/10. Preferably, the surface HLA-DR-associated signal of the target cells is compared to an average surface HLA-DR-associated signal measured on a population of cells being known as highly expressing HLA-DR, so that the ratio between the surface HLA-DR-associated signal measured for the target cells and the average surface HLA-DR-associated signal measured on a population of cells being known as highly expressing HLA-DR is e.g. lower than 1/10. Cells that expresses high levels of HLA-DR at their surface are well known in the art. They are for example classical monocytes

As used herein, **"S100A8/S100A9 calprotectin"** or **"calprotectin"** is a complex of the proteins S100A8 and S100A9 (S100A8/S100A9 heterodimer). Other names for calprotectin include alarmins, MRP8-MRP14, calgranulin A and B, cystic fibrosis antigen, L1, 60BB antigen, and 27E10 antigen. The human homolog of calprotectin is a 24 kDa dimer and is formed by the protein monomers S100A8 (10,835 Da) and S100A9 (13,242 Da). The primary structure of calprotectin can vary between species. For instance, the mouse homologue of S100A8 is 10,295 Da, while the S100A9 homologue is 13,049 Da. Calprotectin has a high affinity for calcium, zinc, iron, and manganese. In the presence of calcium, calprotectin is capable of sequestering the transition metals iron, manganese and zinc via chelation. This metal sequestration affords the complex antimicrobial properties. S100A8/A9 is also capable to interact with Toll-like receptor 4 (TLR4) and the receptor for advanced glycation end-products (RAGE). Calprotectin constitutes up to 60% of soluble protein content in the cytosol of neutrophil granulocytes, and it can be found at a lower concentration in monocytes, macrophages, and squamous epithelial cells. Calprotectin can be secreted in the plasma. Advantageously, the calprotectins are circulating calprotectins. Thus, as used herein, **"circulating S100A8/S100A9 calprotectin"** or **"circulating calprotectin"** are equivalent terms that mean the calprotectins that are secreted by the blood cells such as monocytes, macrophages, and also squamous epithelial cells and/or that are present in a free form in the plasma of the animal. A free form herein means that the calprotectin is not within a cell, but has been secreted in the medium (e.g. in the plasma).

The S100A8/S100A9 calprotectin protein level can be measured using any technique available in the art. In particular, any of the analytical technologies listed above for detecting, identifying, quantifying, expression of cell surface receptors and/or cell surface antigens (on any cells, including blood and/or plasma and/or bone marrow cells, such as monocytes, neutrophils, lymphocytes and leukocytes) can be used. For example, S100A8/S100A9 calprotectin expression may notably be assessed using cell membrane staining using biotinylation or other equivalent techniques followed by immunoprecipitation with specific antibodies, flow cytometry, western blot, ELISA, ELISPOT, antibodies microarrays, immunoprecipitation, immunohistology, dot blot, protein microarray, immunohistochemistry, tissue microarrays coupled to immunohistochemistry, western blot and any of the other techniques listed above. The S100A8/S100A9 calprotectin protein expression or protein level is preferably quantified using a technique selected from the group consisting of flow cytometry, ELISA, nucleic acid microarray, RNA-seq analysis (including 3'RNASeq, 5'RNA-seq, 3'scRNA-Seq, 5'scRNA-Seq), RT-PCT, RT-qPCR and more preferably a technique selected from the group consisting of ELISA, RNA-seq analysis (including 3'RNASeq, 5'RNA-seq, 3'scRNA-Seq, 5'scRNA-Seq), and RT-qPCR.

In the methods of the invention, it is also possible to measure the level of expression of the gene encoding calprotectin. This expression level can be measured at the nucleotide level, by measuring the amount of transcripts (mRNA or cDNA) of said gene, or can be measured at the peptide level, by measuring the amount of proteins derived from said transcripts. When marker expression is measured at the mRNA (or corresponding cDNA) level, any technology usually used by the skilled person can be implemented. These technologies for analyzing the level of gene expression, such as transcriptome analysis, include well-known methods such as PCR (Polymerase Chain Reaction, if starting from DNA), RT-PCR (Reverse Transcription-PCR, if starting from RNA) or quantitative RT-PCR (RT-qPCR), nucleic acid microarrays (including DNA chips and oligonucleotide chips), RNA-seq analysis (including 3'RNASeq, 5'RNA-seq, 3'scRNA-Seq, 5'scRNA-Seq), for a higher throughput. These technologies are routinely used by the skilled person and therefore there is no need to detail them here. Exemplary embodiments (mRNA or cDNA microarray) and RT-quantitative PCR are described in the experimental section. It is also possible to use hybridization with a labeled nucleic acid probe, such as northern blot (for mRNA) or Southern blot (for cDNA), but also by techniques such as the serial gene expression analysis method (SAGE) and its derivatives, such as LongSAGE, SuperSAGE, DeepSAGE, etc. It is also possible to use tissue chips (also known as TMAs: "tissue microarrays"). The tests usually used with tissue chips include immunohistochemistry and *in situ* fluorescent hybridization. For mRNA analysis, tissue chips can be coupled with *in situ* fluorescent hybridization. Finally, it is possible to use massive sequencing in parallel to determine the amount of mRNA in the sample (RNA-Seq or "Whole Transcriptome Shotgun Sequencing"). To this end, several massive parallel sequencing methods are available. Such methods are described in, for example, US 4,882,127; US 4,849,077; US 7,556,922; US 6,723,513; WO 03/066896; WO 2007/111924; US 2008/0020392; WO 2006/084132; US 2009/0186349; US 2009/0181860; US 2009/0181385; US 2006/0275782; EP-B1-1141399; Shendure & Ji, Nat Biotechnol., 26(10): 1135-45, 2008; Pihlak et al., Nat Biotechnol., 26(6): 676- 684, 2008; Fuller et al., Nature Biotechnol., 27(11): 1013-1023, 2009; Mardis, Genome Med., 1(4): 40, 2009; Metzker, Nature Rev. Genet., 11(1): 31-46, 2010.

**"S100 calcium-binding protein A8"** or **"S100A8"** or **"calgranulin A"** is a protein member of the S100 family of proteins containing 2 EF-hand calcium-binding motifs. S100A8 in human is encoded by the S100A8 gene (NCBI GeneID: 6279). In human, S100A8 protein has the NCBI reference sequence NP_001306130.1 or NP_002955.2 (isoform d) or NP_001306127.1 (isoform c) or NP_001306126.1 (isoform b) or NP_001306125.1 (isoform a) or CAG28602.1.

**"S100 calcium-binding protein A9"** or **"S100A9"** or **"calgranulin B"** or **"migration inhibitory factor-related protein 14 (MRP14)"** is a protein member of the S100 family of proteins containing 2 EF-hand calcium-binding motifs. S100A9 in human is encoded by the S100A9 gene (NCBI GeneID: 6280). In human, S100A8 protein has the NCBI reference sequence CAG47003.1 or CAG47020.1 or NP_002956.1.

As used herein, **"S100A8/S100A9 calprotectin inhibitor"** or **"calprotectin inhibitor"** is a molecule/compound/drug able to block at least partially the activity/function of calprotectin (e.g. disrupt at least 5%, 10%, 20%, 20%, 40%, 50%, 60%, 70%, 80%, 90% or more of at least on function/activity of calprotectin). An function of calprotectin comprises for example the ability to bind and/or chelate metal (calcium, zinc, iron, and manganese), the binding to TLR4 and RAGE, the interaction with myeloid progenitors. A calprotectin inhibitor may also bind calprotectin, and therefore prevents it from performing its natural function. Calprotectin inhibitors include for example, tasquinimod, paquinimod, etc.

As used herein, « **tasquinimod** » (or ABR-215050, or CID 54682876) is a quinoline-3-carboxamide variant synthesized using the drug roquinimex as a starting point. It is a drug currently being investigated for the treatment of solid tumors (Fizazi et al., 2017). Tasquinimod is a small-molecule inhibitor that has been shown to target the tumor microenvironment by controlling the accumulation and immunosuppressive, pro-angiogenic and pro-metastatic functions of regulatory myeloid cells. It binds to and inhibits the interactions of S100A9 with its target.

As used herein, « **paquinimod** » (or ABR-215757) is small-molecule inhibitor, ABR-215757 (paquinimod) that blocks the binding of S100A9 to TLR4 and RAGE (Kraakman et al., 2017; Raquil et al., 2008) and the preclinical anti-CD33 monoclonal antibodies (Walter, 2018) that may prevent S100A9 interaction with myeloid progenitors.

As used herein, **"cytokine"** refers to a broad and loose category of small proteins (∼5-20 kDa) important in cell signaling. Cytokines are peptides and cannot cross the lipid bilayer of cells to enter the cytoplasm. They act through cell surface receptors and are especially important in the immune system; cytokines modulate the balance between humoral and cell-based immune responses, and they regulate the maturation, growth, and responsiveness of particular cell populations. Immunological cytokines can be classified in two categories: those that enhance cellular immune responses, i.e. type 1 (such as TNFα, IFN-γ, etc.), and, type 2, i.e. those that favor antibody responses (such as TGF-B, IL-4, IL-10, IL-13, etc.). **"Chemokines"** are a family of small cytokines, or signaling proteins secreted by cells. Their name is derived from their ability to induce directed chemotaxis in nearby responsive cells; they are chemotactic cytokines.

The term **"antibody"** as used herein is intended to include monoclonal antibodies, polyclonal antibodies, and chimeric antibodies. Antibody fragments can also be used in the present diagnosis method. This term is intended to include Fab, Fab', F(ab')2, scFv, dsFv, ds-scFv, dimers, minibodies, nanobodies, diabodies, and multimers thereof and bispecific antibody fragments. Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, dsFv, ds-scFv, dimers, minibodies, diabodies, bispecific antibody fragments and other fragments can also be synthesized by recombinant techniques. The antibodies can be of different isotypes (namely IgA, IgD, IgE, IgG or IgM). They may be of recombinant sources and/or produced in transgenic animals. Conventional techniques of molecular biology, microbiology and recombinant DNA techniques are within the skill of the art. Such techniques are explained fully in the literature. Commercial antibodies recognizing specifically the antigens expressed by blood cells can be furthermore used. Some of them are listed in the experimental part below (said list being however not exhaustive nor limiting). These antibodies can be detected by direct labelling with detectable markers. Alternatively, unlabelled primary antibody can be used in conjunction with a labelled secondary antibody, comprising antisera, polyclonal antisera or a monoclonal antibody specific for the primary antibody. Immunohistochemistry protocols and kits are well known in the art and are commercially available.

Advantageously, the antibodies are tagged with a detectable marker, preferably a fluorescent or a luminescent marker. Examples of detectable markers / labels include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase examples of suitable prosthetic group complexes include streptavidin/biotin and avidin/biotin, examples of suitable fluorescent materials include umbelliferone, fluorescein, fluorescein isothiocyanate, rhodamine, dichlorot[pi]azinylamine fluorescein, dansyl chloride or phycoerythrin, an example of a luminescent material includes luminol, examples of bioluminescent materials include luciferase, luciferin, and aequorin, and examples of suitable radioactive material include ¹²⁵I, ¹³¹I, ³⁵S or ³H.

The terms **"pharmaceutically acceptable"** are herein intended to mean what is useful to the preparation of a pharmaceutical composition, and what is generally safe and non-toxic, for a pharmaceutical use.

The terms **"pharmaceutically acceptable salt"**, as used herein, mean a salt of a compound which is pharmaceutically acceptable, as defined above, and which possesses the pharmacological activity of the corresponding compound. The pharmaceutically acceptable salts comprise:
(i) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(ii) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

As used herein, **"remdesivir"** or "GS-5734" is a broad-spectrum antiviral compound, acting as a nucleoside analogue, specifically an adenosine analogue. Its presence misleads the viral polymerase and causes a reduction in viral RNA synthesis. Remdesivir has the formula C₂₇H₃₅N₆O₈P. It was initially developed for the treatment of Ebola virus infections.

**"Lopinavir"** is a viral protease inhibitor, previously used against the human immunodeficiency virus (HIV). Lopinavir inhibits the production of functional proteins by the new virions, thereby blocking the spread of the virus. The IUPAC (International Union of Pure and Applied Chemistry) name for lopinavir is (2S)-N-[(2S,4S,5S)-5-{[2-(2,6-diméthylphénoxy)acétyl]amino]-4-hydroxy-1,6-diphénylhexan-2-yl}-3-méthyl-2-(2-oxo-1,3-diazinan-1-yl)butanamide. Lopinavir is rapidly degraded in the body. For this reason, it is usually administered in fixed combination with **"Ritonavir"**, which inhibit cytochrome P450 monooxygenases, thereby slowing the degradation of Lopinavir by these enzymes.

**"Ritonavir"** is an antiretroviral drug used to treat HIV infection. It is a protease inhibitor. The IUPAC name for ritonavir is N-[(2S,3S,5S)-3-hydroxy-5-[(2S)-3-méthyl-2-{[méthyl({[2-(propan-2-yl)-1,3-thiazol-4-yl]méthyl})carbamoyl]amino}butanamido]-1,6-diphénylhexan-2-yl]carbamate de 1,3-thiazol-5-ylméthyle.

**"Chloroquine"** or "chloroquinine" is an antimalarial drug of the 4-aminoquinoline family. The IUPAC name for chloroquine is (RS)-N-(7-chloroquinolin-4-yl)-N,N-diéthyl-pentane-1,4-diamine.

**"Hydroxychloroquine"**, initially known for its anti-malaria activity, has been shown to have an apparent efficacy in the treatment of Covid-19 (Yao et al., 2020). The IUPAC name for hydroxychloroquine is (RS)-2-[{4-[(7-chloroquinolin-4-yl)amino]pentyl}(éthyl)amino]ethanol.

**"Beta-1a Interferon"** is a 166 amino acid recombinant glycoprotein having the same sequence and glycosylation state as natural human interferon beta. It is usually used for treating on multiple sclerosis and acts as an immunomodulator.

### Methods of the invention

Increasing evidences suggest a dysregulation of blood myeloid cells in the coronavirus disease 2019 (COVID-19) caused by severe acute respiratory syndrome-coronavirus 2 (SARS-CoV-2). Yet, no clear immune cell signatures distinguishing patients with mild versus severe COVID-19 or predicting the evolution of the disease have been ever identified. In this context, the present inventors performed high dimensional single cell analyses at the protein level of peripheral blood cells of 154 COVID-19 patients as well as single cell RNA-sequencing on 3 infected patients at two time points, with both patients with mild versus severe COVID-19. A distinctive type I interferon response was detected in monocytes and granulocytes of SARS-CoV-2⁺ mild outpatients, correlating with an increased IFNa2a plasma level. In contrast, in severe COVID-19, this response was absent while non-classical CD14^{low}CD16⁺ monocytes disappeared and HLA-DR^{low} classical monocytes accumulated in the peripheral blood. Importantly, in the latter, massive amounts of calprotectin (S100A8/S100A9) and inflammatory cytokines and chemokines were released, together with CD10⁻CD101⁻CXCR4^{+/-} immature neutrophils and erythroid cells whose genotype and phenotype suggests immunosuppressive cells. Egress of immature myeloid cells from the bone marrow, including CD34⁺ cells with enhanced phosphorylated RelA/p65, suggested emergency myelopoeisis.

The data show that these rapid modulations of innate immune cells are a characteristic feature of COVID that has not been reported in other viral infections so far. More importantly, the present data show that these modulations are a characteristic biological signature of an imminent switch to an aggressive form of COVID disease. In addition, the Inventors have demonstrated that these modulations correlate with the occurrence of a burst of calprotectin in the blood of COVID patients. They propose that this calprotectin burst triggers an emergency myelopoiesis that can be assessed by analysing the level of specific innate immune cells.

In this context, the Inventors have thus designed a new method for the prognosis, for the monitoring, for the stratification, and/or for determining the outcome of a betacoronavirus disease in a subject. This method comprises the step of measuring, in a biological sample from said subject, an innate immune response that could be driven by a calprotectin burst.

As explained above, this innate immune response is characterized by specific levels of CD14^{low} /CD16⁺ monocytes, of HLA-DR^{low} classical monocytes; and/or of CD16^{low} neutrophils that are a signature of an imminent switch into severe form. In a preferred embodiment, the signature may also contain detecting the level of circulating calprotectin in the tested subject. This signature is typically characterized by a decrease in CD14^{low} /CD16⁺ non-classical monocytes, an increase of HLA-DR^{low} classical monocytes; and/or an increase in CD16^{low} neutrophils, in the blood of the patient. Other markers have also been identified, associated with an enhanced myelopoiesis and the subsequent release of immature cells having an immunosuppressive phenotype, as explained in the examples part below. This signature can be assessed by any means, and preferably by FACS.

In particular, the methods of the invention can comprise the steps of:
a) Quantifying, in a biological sample from the subject, the level of at least one biomarker selected from the group consisting of:
   - CD14^{low}/CD16⁺ monocytes;
   - HLA-DR^{low} classical monocytes;
   - S100A8/S100A9 calprotectins (preferably the level of circulating S100A8/S100A9 calprotectins),
   - CD16^{low} neutrophils (preferably CD101⁻CD10⁻CD16^{low} neutrophils); and
   - Any combination thereof;
b) Comparing the level(s) obtained in step a) to (a) reference level(s); and
c) Determining the prognosis of the betacoronavirus disease, monitoring the COVID and/or betacoronavirus infection, stratifying the COVID and/or betacoronavirus infection, determining the outcome of the COVID and/or betacoronavirus infection, or any combination thereof, based on the comparison of step b).

When used for detecting the outcome of the disease, this method can serve also for assessing the risk, for a subject suffering from a betacoronavirus disease of switching to a severe form of the disease. It can also serve for identifying if a subject suffering from a betacoronavirus disease and/or a viral infection due to at least one betacoronavirus is at risk of switching to a severe form of the disease.

This prognosis method can also be used for selecting a therapy for a subject suffering from a COVID and/or a viral infection due to at least one betacoronavirus, depending on the levels of the tested biomarkers, or for assessing the efficacy of a therapy in a treated patient.

It can also be used for adapting a COVID therapy for a COVID-suffering subject. In this case, the method comprises the steps of :
a) Assessing the efficacy of the therapy according to the method disclosed above; and
b) Adapting the therapy based on the result of step a), if the patient is at risk of undergoing the switch toward the severe form of the disease, despite the treatment.

In a particular embodiment, the methods of the invention contain the following steps:
a) Detecting a monocyte population in a biological sample from the subject;
b) Quantifying, in a biological sample from the subject, the level of CD14^{low}/CD16⁺ monocytes and/or HLA-DR^{low} classical monocytes;
c) Comparing the level(s) obtained in step b) to reference level(s) of these biomarkers; and
d) Determining the prognosis of the disease, monitoring the disease, stratifying the patients, determining the outcome of the disease, or any combination thereof, based on the comparison of step c).

In another particular embodiment, the methods of the invention contain the following steps:
a) Detecting a neutrophil population in a biological sample from the subject;
b) Quantifying, in a biological sample from the subject, the level of CD16^{low} neutrophils (preferably CD101⁻CD10⁻CD16^{low} neutrophils);
c) Comparing the level obtained in step b) to a reference level; and
d) Determining the prognosis of the disease, monitoring the disease, stratifying the patients, determining the outcome of the disease, or any combination thereof, based on the comparison of step c).

In another particular embodiment, the methods of the invention contain the following steps:
a) Quantifying, in a biological sample from the subject, the level of S100A8/S100A9 calprotectins (preferably the level of circulating S100A8/S100A9 calprotectins),
b) Comparing the level obtained in step a) to a reference level; and
c) Determining the prognosis of the disease, monitoring the disease, stratifying the patients, determining the outcome of the disease, or any combination thereof, based on the comparison of step b).

In one embodiment of the present methods :
(i) If the level of CD14^{low}/CD16⁺ monocytes quantified in step a) is lower than the reference level of step b), wherein said reference level is the mean level of CD14^{low}/CD16⁺ monocytes quantified in a healthy subject or in a subject suffering from an asymptomatic or a mild COVID;
(ii) If the level of HLA-DR^{low} classical monocytes quantified in step a) is higher than the reference level of step b), wherein said reference level is the mean level of HLA-DR^{low} classical monocytes quantified in a healthy subject or in a subject suffering from an asymptomatic or a mild COVID;
(iii) If the level of S100A8/S100A9 calprotectins quantified in step a) is higher than the reference level of step b), wherein said reference level is the mean level of S100A8/S100A9 calprotectins quantified in a healthy subject or in a subject suffering from an asymptomatic or a mild COVID; and/or
(iv) If the level of CD16^{low} neutrophils quantified in step a) is higher than the reference level of step b), wherein said reference level is the mean level of CD16^{low} neutrophils quantified in a healthy subject or in a subject suffering from an asymptomatic or a mild COVID;

Then: the betacoronavirus disease is exacerbated and/or has switched to a severe form, worsening the outcome and the prognosis of the disease. The patient is indeed progressing into a severe form that will eventually require intensive care. In other terms, the risk of switching to a severe COVID form becomes high, and the subject is identified as being at risk of switching to a severe COVID form.

Advantageously, said reference level is the mean level of said biomarker quantified in at least two healthy subjects or at least two subjects suffering from an asymptomatic or a mild COVID.

Classical therapy for treating betacoronavirus infected patients is usually Remdesivir, Lopinavir, Rotinavir, chloroquine, hydroxychloroquine, or beta-1a Interferon (or any pharmaceutically acceptable salts thereof). As a matter of fact, these compounds have been shown to have a therapeutic effect in preventing and/or treating COVID. Yet, this therapy is sometimes not sufficient to impair the switch into a COVID severe form. In the context of the invention, it can be concluded that, in any of the above mentioned (i) to (iv) case, the efficacy of the therapy administered to the subject, if any, is unsatisfactory. The treatment has to be changed, or the doses should be increased. The methods of the invention can then be performed again to verify that the newly administered dose or treatment indeed ameliorates the prognosis of the treated patient.

In one embodiment, the methods further comprise the step of detecting a monocyte population, preferably prior to the step of quantifying the level of CD14^{low}/CD16⁺ monocytes and/or the level of HLA-DR^{low} classical monocytes. This monocyte population is preferably detected by an exclusion gating strategy by flow cytometry.

Advantageously, detecting the monocyte population comprises contacting the biological sample(s) with antibodies recognizing antigens expressed by cells selected from the group consisting of: granulocytes, T lymphocytes, B lymphocytes, NK cells, and any combination thereof; wherein said antibodies are preferably selected from the group consisting of: anti-CD56 antibodies, anti-CD2 antibodies, anti-CD24 antibodies, anti-CD15 antibodies, anti-CD16 antibodies and any combination thereof. the monocyte population comprises, or consists essentially of, or consists of, cells selected from the group consisting of: CD45⁺ cells, CD15⁻ cells, CD2⁻ cells, CD56⁻ cells, CD24⁻ cells, and any combination thereof; and/or cells selected from the group consisting of: CD2⁺ cells, CD56⁺ cells, CD24⁺ cells, CD14⁻CD16⁻ cells, and any combination thereof, are excluded.

In one embodiment, the methods further comprise the step of quantifying all the monocytes in the sample(s). This step thus comprises detecting or purifying the monocyte population in the biological sample of the tested subject.

In a particular embodiment, it is advantageous to analyze only the CD45 expressing-cells, in order to eliminate the contaminant blasts and to select mature cells, including all the monocytes. In this embodiment, the monocytes are detected in the CD45⁺/SSC^{intermediate} population of cell present in the biological sample. After exclusion of other contaminating populations, the CD14 and CD16 expression can be assessed. Thus, the step of quantifying all the monocytes in the sample(s) advantageously comprises the detection and the measurement of CD45 expression at the cell surface and of the side scatter parameter (SSC) of the cells present in the biological sample.

In a particular embodiment, it is advantageous to detect a substantially pure monocyte population, that is, a population of monocytes that is devoid of contaminant cells. The remaining white blood cells can be identified and counter-selected on the basis of the expression of specific markers. Using anti-CD15, anti-CD16, anti-CD56, anti-CD2 or anti-CD24 antibodies enables to detect and therefore exclude, if needed, the cells expressing CD2, CD56 and CD24 proteins, notably the CD2⁺ T lymphocytes, the CD2⁺ NK cells, the CD56⁺ NK cells, the CD24⁺ immature granulocytes as well as the CD15⁺ or CD16⁺⁺ granulocytes.

Therefore the step of quantifying all the monocytes in the sample(s) advantageously comprises the steps of:
- Excluding the CD2+ cells from the analysis (in order to eliminate the contaminant T lymphocytes and a part of the NK cells);
- Excluding the CD56+ cells from the analysis (in order to eliminate the remaining contaminant NK cells);
- Excluding the CD16++ or the CD15+ cells from the analysis (in order to eliminate the granulocyte cells); and / or
- Excluding the CD24+ cells from the analysis (said cells corresponding to granulocytes and B lymphocytes).

Advantageously, the methods also further comprise the step of calculating the ratio of CD14^{low}/CD16⁺ monocytes to all monocytes and/or to detect a significant fraction of HLA-DR^{low} cells among classical monocytes. Accordingly, this step comprises quantifying all the monocytes (that is, calculating the number or the concentration of cells of the MO1, MO2 and MO3 populations) in the sample and calculating the ratio of CD14^{low}/CD16⁺ monocytes (MO3) to all monocytes and to detect the presence of HLA-DR^{low} cells among classical monocytes. In the methods of the invention, it is possible to conclude that a switch to the severe COVID form is likely if a significant fraction of HLA-DR^{low} cells among classical monocytes is detected.To be significant, the fraction of HLA-DR^{low} classical monocytes should be e.g. superior to 1%, with respect to the total number of monocytes.

The Inventors have surprisingly shown that a decrease in non-classical monocyte fraction below 4% can be used to discriminate between the subjects suffering from a severe COVID and other reference subjects. Thus, it can be said that when the ratio of CD14^{low}/CD16⁺ monocytes to all monocytes is lower than 4% of the total monocytes, then the prognosis of the subject is poor because the betacoronavirus disease is exacerbated and/or has switched to a severe form, so that the outcome of the COVID disease is likely switching into a severe COVID form. In other terms, the subject is identified as being at risk of switching to a severe COVID form when the ratio of CD14^{low}/CD16⁺ monocytes to all monocytes is lower than 4% of the total monocytes.

In one embodiment, the present methods further comprise the step of detecting a neutrophil population, preferably prior to the step of quantifying the level of CD16^{low} neutrophils. The neutrophil population is preferably detected by an exclusion gating strategy by flow cytometry. In an advantageous embodiment, the methods further comprise the steps of quantifying all the neutrophils in the sample(s). In the methods of the invention, it is possible to conclude that a switch to the severe COVID form is likely if the sample contains a significant fraction of CD16^{low} neutrophils. To be significant, the fraction of CD16^{low} neutrophils should be e.g. superior to 1%, with respect to the total number of neutrophils. Advantageously, the methods also further comprise the step of calculating the ratio of CD16^{low} neutrophils to all neutrophils. To conclude that a switch to the severe COVID form is likely, his ratio is preferably superior to 1%.

In one embodiment of the invention, the methods of the invention require to compare the level of S100A8/S100A9 calprotectins to a reference level, in order to determine if the betacoronavirus disease is likely to become exacerbated and/or has switched to a severe form. This reference level of calprotectin is for example comprised between 150 and 400 ng/mL, what correspond to the mean calprotectin level measured in healthy subjects blood. The results of the inventors show that the patients suffering from mild COVID disease have a mean calprotectin level of about 181 ng/mL (IQR 134-242) in their blood, whereas patients suffering from severe COVID disease have a mean calprotectin level of about 3641 ng/mL (IQR 2030-5664) in their blood. Therefore, it can be said that the COVID disease has a bad prognosis if the calprotectin level in the blood of a patient is superior to 400 ng/mL, preferably superior to 600 ng/mL, more preferably superior to 1000 ng/mL.

In an advantageous embodiment of the present methods, the level of CD14^{low}/CD16⁺ monocytes is quantified by a method comprising a step of contacting the sample with an anti-CD14 antibody and/or an anti-CD16 antibody; the level of HLA-DR^{low} classical monocytes is quantified by a method comprising a step of contacting the sample with an anti-HLA-DR antibody, the level of S100A8/S100A9 calprotectins (preferably the level of circulating S100A8/S100A9 calprotectins) is quantified by a method comprising a step of contacting the sample with an anti-S100A8 antibody and/or an anti-S100A9 antibody; the levels of CD16^{low} neutrophils (preferably CD101⁻CD10⁻CD16^{low} neutrophils) is quantified by a method comprising a step of contacting the sample with an anti-CD16 antibody (and optionally with an anti-CD10 antibody and/or an anti-CD101 antibody); and any combination thereof.

The level of CD14^{low}/CD16⁺ monocytes, the level of HLA-DR^{low} classical monocytes, the level of S100A8/S100A9 calprotectins (preferably the level of circulating S100A8/S100A9 calprotectins), the levels of CD16^{low} neutrophils (preferably CD101⁻CD10⁻CD16^{low} neutrophils), and any combination thereof is preferably quantified by flow cytometry.

Even more preferably, the level of S100A8/S100A9 calprotectins (preferably the level of circulating S100A8/S100A9 calprotectins) is(are) quantified using a technique selected from the group consisting of flow cytometry, ELISA (preferably multiplex), nucleic acid microarray, RNA-seq analysis (including 3'RNASeq, 5'RNA-seq, 3'scRNA-Seq, 5'scRNA-Seq), RT-PCT, RT-qPCR and more preferably a technique selected from the group consisting of ELISA, RNA-seq analysis (including 3'RNASeq, 5'RNA-seq, 3'scRNA-Seq, 5'scRNA-Seq), and RT-qPCR.

In the present methods, the biological sample is preferably a blood sample, a plasma sample, or a bone marrow sample. Since monocytes, neutrophils, calprotectins and cytokines are mostly found in the blood and plasma, it is particularly advantageous to use blood and/or plasma as a biological sample for the method of the invention. Indeed, such a blood and/or plasma sample may be obtained by a completely harmless, non-invasive blood and/or plasma collection from the subject. The blood and/or plasma sample used in the present methods is preferably depleted of most, if not all erythrocytes, by common red blood cell lysis procedures. The detection is performed on the remaining blood cells, which are white blood cells (e.g., neutrophils, monocytes, lymphocytes, basophiles, etc.) and platelets.

Any volume used commonly by the person of skills in the art for hematological analyses will be convenient for the present methods. For example, the volume of the blood sample can be of 100 µL, 200 µL, 300 µL, 400 µL, 500 µL, 600 µL, 700 µL, 800 µL, 900 µL, or 1000 µL (1mL).

The inventors have shown that there is an egress of immature myeloid cells from the bone marrow, including CD34⁺ cells with enhanced phosphorylated RelA/p65. Therefore, the methods can advantageously further comprise a step of quantifying the level of CD34⁺ phosphorylated RelA/p65 cells in the sample(s).

In one embodiment of the invention, the innate immune cell signature of the invention includes the level of CD34⁺ phosphorylated RelA/p65 cells. The methods of the invention can indeed contain the step of assessing the level of CD34⁺ phosphorylated RelA/p65 cells in the biological sample of the tested subject. If the level of CD34⁺ phosphorylated RelA/p65 cells is lower in the sample of the subject than the reference level (wherein said reference level is for example the mean level of CD34⁺ phosphorylated RelA/p65 cells quantified in a healthy subject or in a subject suffering from an asymptomatic or a mild COVID), then the prognosis of the disease is poor (Riva M. et al, 2012).

This biomarker can be used in any of the methods mentioned above, e.g., for assessing the efficiency of a therapy or stratifying the patients. It is preferably combined with any of the other biomarker disclosed above.

All the definitions mentioned above apply, *mutatis mutandis*, to the terms used in all the methods of the invention.

### Uses as biomarkers

The Inventors have shown that the levels of CD14^{low}/CD16⁺ monocytes, HLA-DR^{low} classical monocytes, S100A8/S100A9 calprotectins, and/or CD16^{low} neutrophils can be used as biomarkers for the prognosis, for the monitoring, for the stratification, for determining the outcome, or any combination thereof, of a betacoronavirus disease (COVID). They can also be used as biomarkers for assessing the risk, for a subject suffering from a betacoronavirus disease, of switching to a severe form. The data also suggest that the levels of CD14^{low}/CD16⁺ monocytes, HLA-DR^{low} classical monocytes, S100A8/S100A9 calprotectins, and/or CD16^{low} neutrophils are useful for selecting a therapy for a subject suffering from a COVID, for assessing the efficacy of a therapy in a subject suffering from a COVID and for adapting a COVID therapy for a COVID-suffering subject.

The present invention thus relates to the use of at least one selected from the group consisting of:
- CD14^{low}/CD16⁺ monocytes;
- HLA-DR^{low} classical monocytes;
- S100A8/S100A9 calprotectins (preferably the level of circulating S100A8/S100A9 calprotectins),
- CD16^{low} neutrophils (preferably CD101⁻CD10⁻CD16^{low} neutrophils); and
- Any combination thereof;
as biomarkers, preferably as biomarkers of a betacoronavirus disease (COVID) and/or of a viral infection due to a betacoronavirus in a subject.

More precisely, these biomarkers can be used for the prognosis, for the monitoring, for the stratification, or for determining the outcome of a betacoronavirus disease in a subject; and/or
for assessing the risk, for a subject suffering from a betacoronavirus disease of switching to a severe form of said disease and/or for identifying a subject at risk of switching to a severe form of said disease; and/or for selecting a therapy for a subject suffering from said disease; and/or for assessing the efficacy of a therapy in a subject suffering from said disease; and/or for adapting a COVID therapy.

### Therapeutic uses

The Inventors have demonstrated the surprising occurrence of a burst of calprotectin in COVID patients. Their data show that calprotectin plasma level is also a biomarker of COVID-19 switch to severe diseases. This strongly suggests that calprotectins can be used as therapeutic targets to prevent and/or treat COVID and/or betacoronavirus infections.

In a further aspect, the invention thus relates to a S100A8/S100A9 calprotectin inhibitor for use for preventing a worsening and/or for treating a betacoronavirus disease.

The invention thus also relates to the use of a S100A8/S100A9 calprotectin inhibitor for preventing a worsening and/or for treating a betacoronavirus disease and/or a viral infection due to betacoronavirus. In other terms, the invention relates to the use of a S100A8/S100A9 calprotectin inhibitor for the manufacture of a medicament intended to treat a betacoronavirus disease and/or a viral infection due to a betacoronavirus, and to a method for preventing and/or treating a betacoronavirus disease, comprising the administration of a S100A8/S100A9 calprotectin inhibitor in a subject in need thereof.

The S100A8/S100A9 calprotectin inhibitor is preferably selected from the group consisting of tasquinimod, paquinimod, pharmaceutically acceptable salts thereof, and any combination thereof.

Advantageously, the betacoronavirus is a Severe Acute Respiratory Syndrome-related coronavirus (SARS-CoV), in particular SARS-CoV-2; and/or the betacoronavirus disease is cororonavirus disease 19 (COVID-19), more particularly a severe COVID-19.

### DESCRIPTION OF THE FIGURES

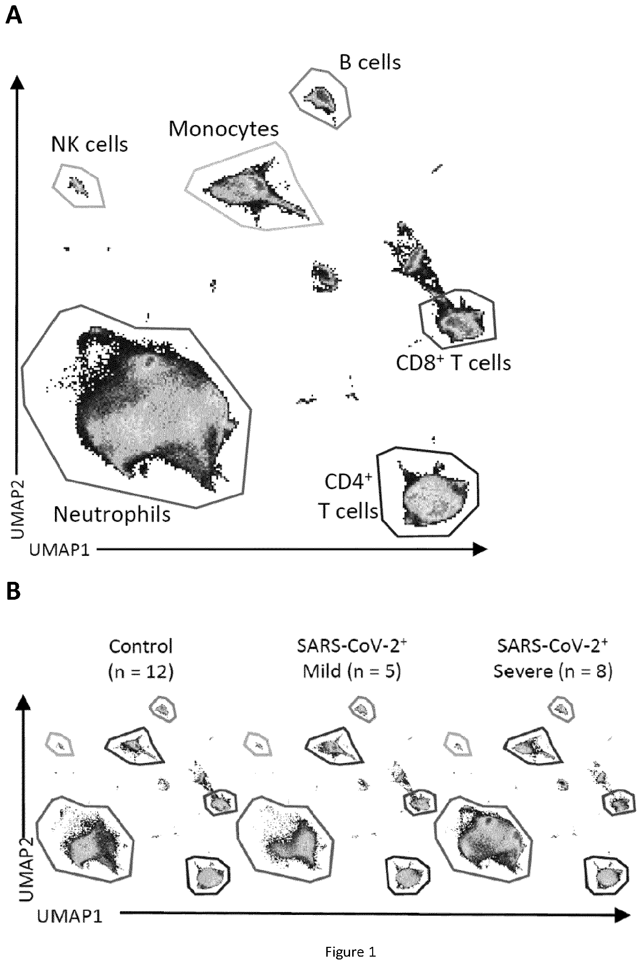

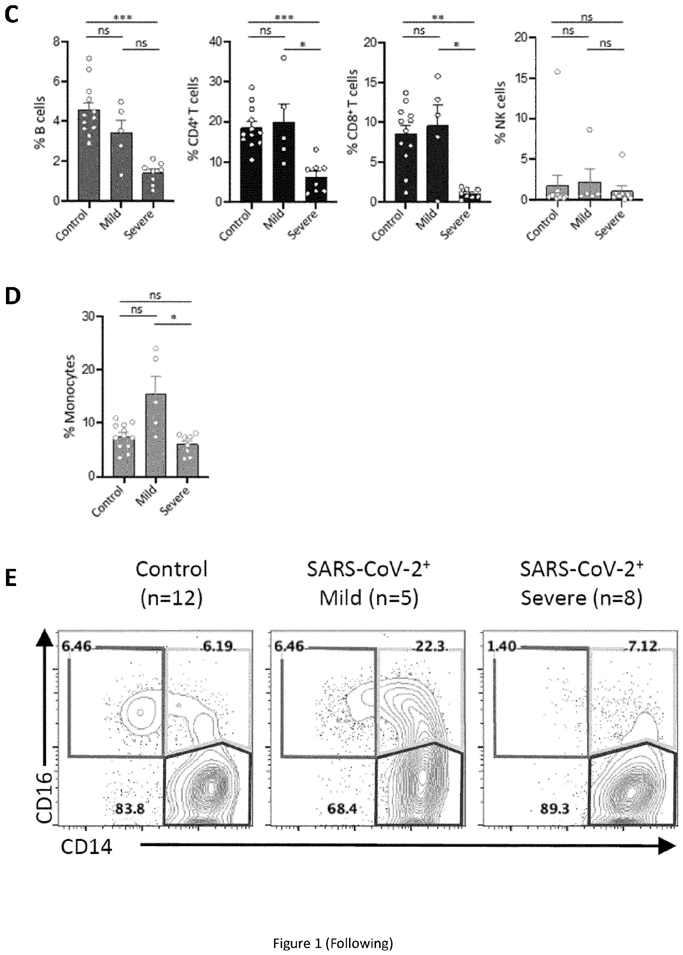

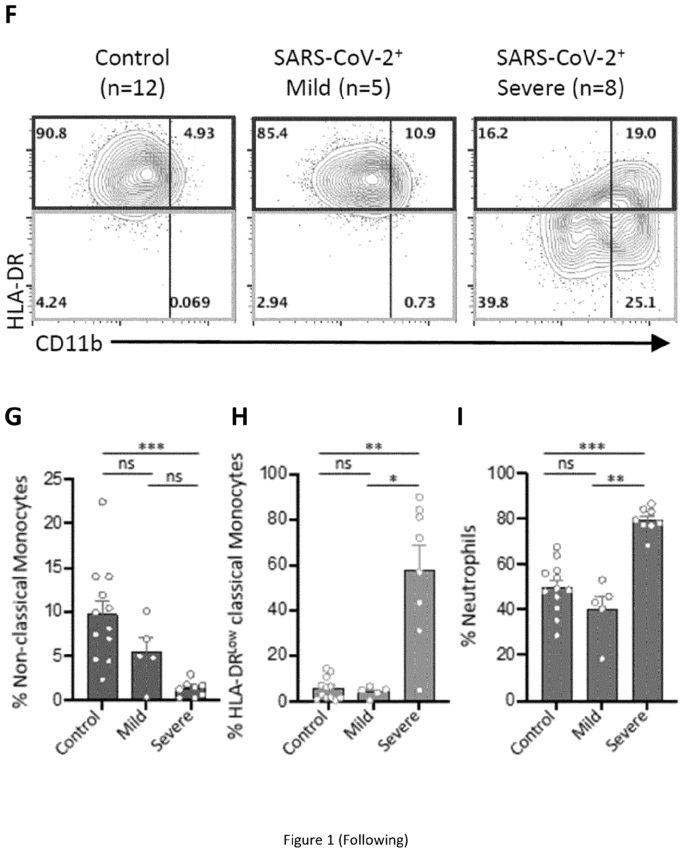

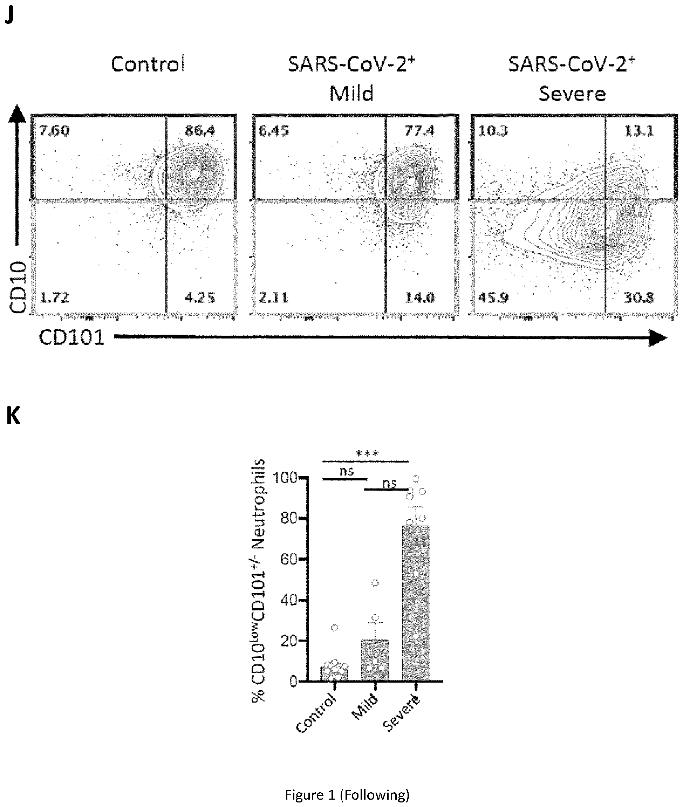

**Figure 1****: Spectral flow analysis of peripheral blood cells in a learning cohort of controls and COVID-19 patients. A.** Non-supervised UMAP analysis of data from 25 patient blood samples (controls=12; mild=5; critical=8); **B.** Non-supervised UMAP analysis of patient blood samples in control, mild and severe groups; C. Percentage of indicated cell populations (B cells, CD4⁺ T cells, CD8⁺ T cells, CD56⁺ NK cells) within total cells in each individual sample (circles) in control, mild and severe groups; **D.** Percentage of CD14⁺ monocytes within total cells in each individual sample analyzed as in panel C. **E.** Partition of monocyte subsets in each patient group (data combined from all patients), based on CD14 and CD16 expression **F.** CD11b and HLA-DR expression on classical monocytes in patient of the three studied groups (data pooled); **G.** % of non-classical monocytes within total monocytes analyzed in each individual sample as in panel C; **H.** Percentage of HLA-DR^{low} classical monocytes among CD14^{High}CD16^{Low} classical monocytes analyzed in each individual sample (circles) as in C; **I.** Percentage of neutrophils in each individual sample as in C; **J.** Partition of neutrophil subsets, based on CD101 and CD10 expression, in patient categories defined in panel D; **K.** Percentage of CD10^{Low}CD101^{+/-} neutrophils among total neutrophils analyzed in each individual sample as in panel C; Kruskal-Wallis test, * *P*<0.05; ** *P* <0.01; *** *P*<0.001; ns, non-significant.

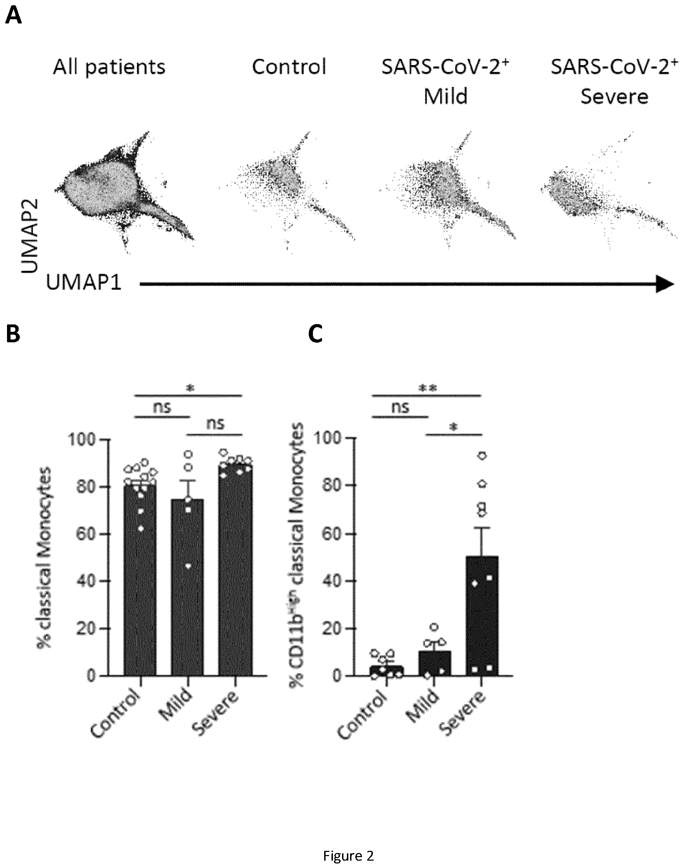

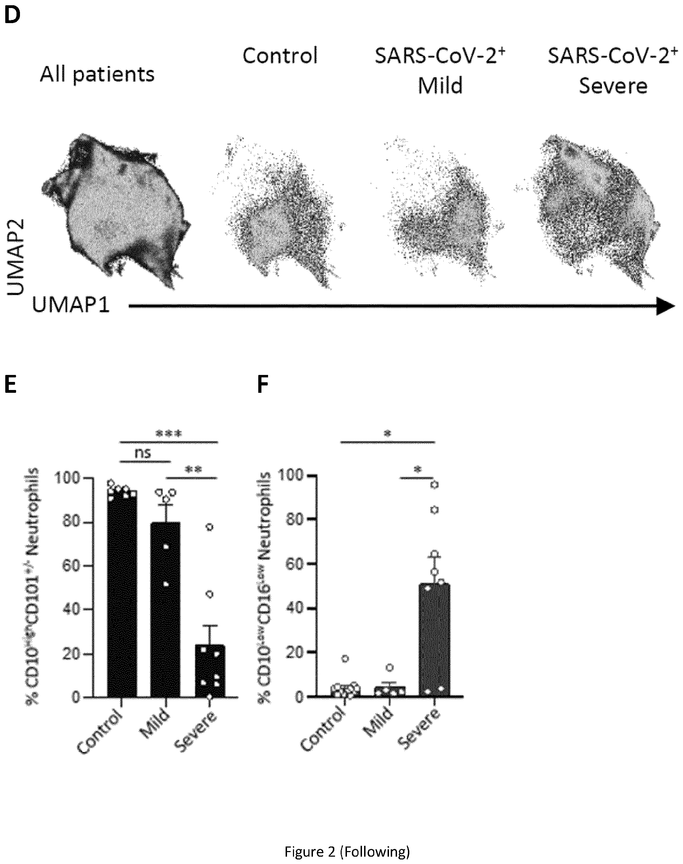

**Figure 2****: Spectral flow analysis of peripheral blood cells in a learning cohort of controls and COVID-19 patients. A.** UMAP profile from pooled data on monocytes in all patients or indicated group of patients; **B,C.** Percentage of CD14⁺ (**B**) or CD11b^{High} (**C**) monocytes among total monocytes in all individuals of indicated groups; **D.** UMAP profile from pooled data on neutrophils in all patients or indicated group of patients; **E.F.** Percentage of CD10^{High} (**E**) or CD16^{Low} neutrophils (**F**) among total neutrophils in all individuals of indicated groups; Kruskal-Wallis test, * *P*<0.05; ** *P* <0.01; *** *P*<0.001; ns, non-significant.

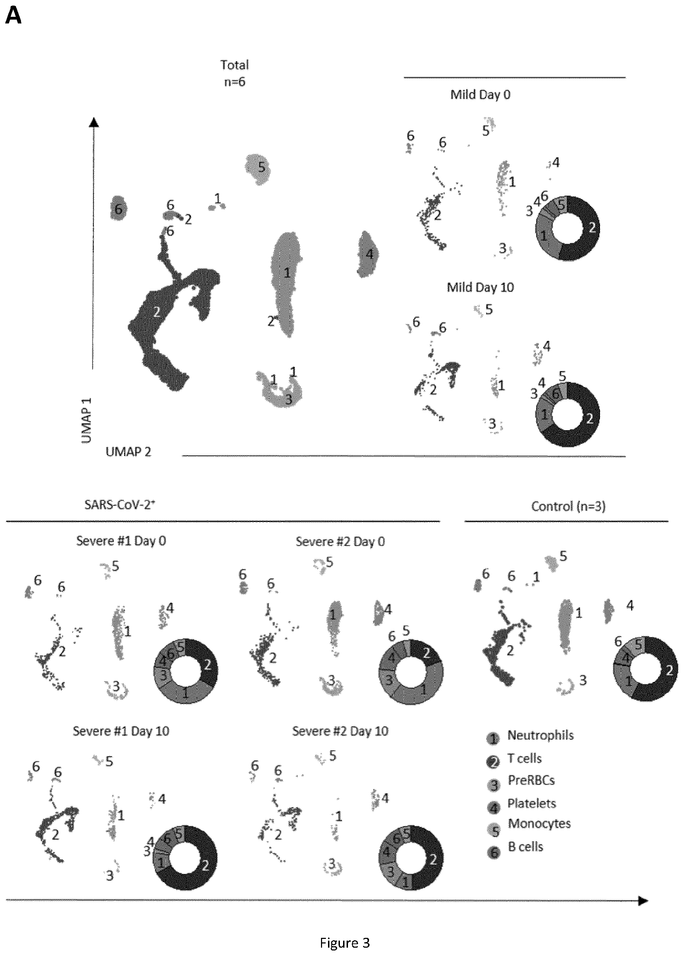

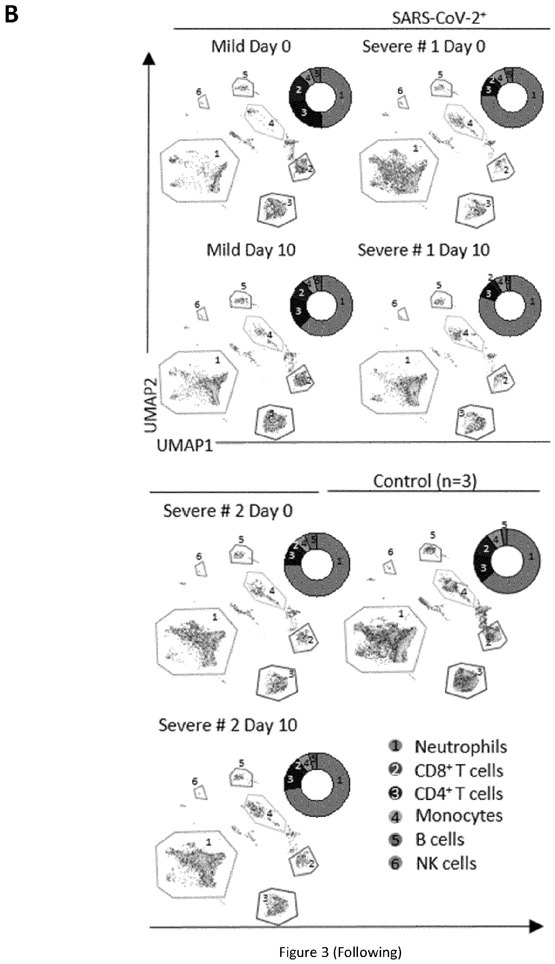

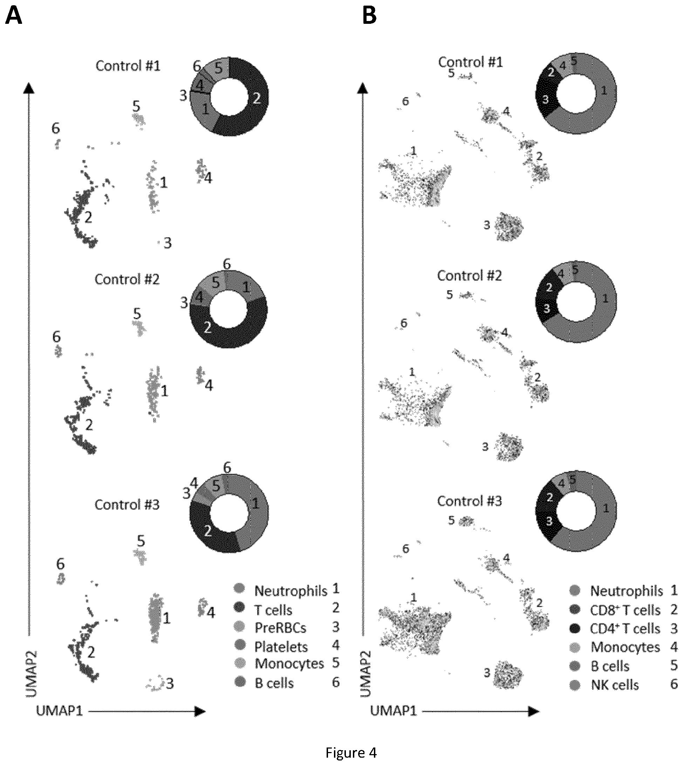

**Figure 3****. Single cell RNA sequencing of peripheral blood cells in SARS-CoV-negative and positive patients. A.** UMAP analysis of the 9 sequenced samples showing the repartition of indicated cell populations. Patient samples were analyzed individually at days 0 and 10; control patient data have been combined (individual analyses shown in **Figure 4**); **B.** UMAP analysis of spectral flow cytometry data in each patient at day 0 and at day 10 and the three combined controls (individual analyses shown in Figure S2), showing the repartition of indicated cell populations.
**Figure 4****. Single cell analysis of peripheral blood cells. A-B.** Individual UMAP analysis of each control sample analyzed by scRNAseq (**A**) and spectral flow cytometry (**B**).

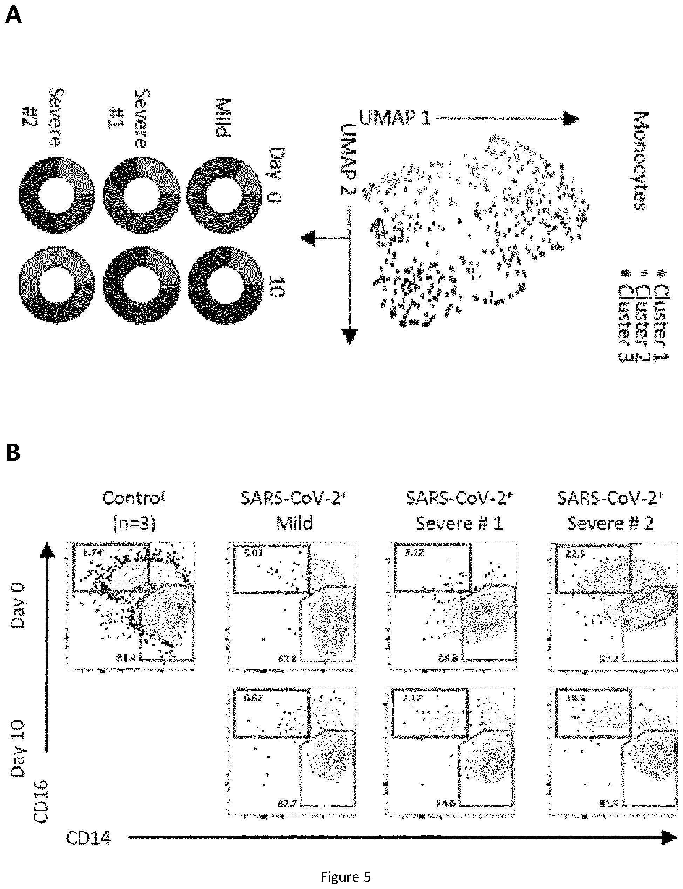

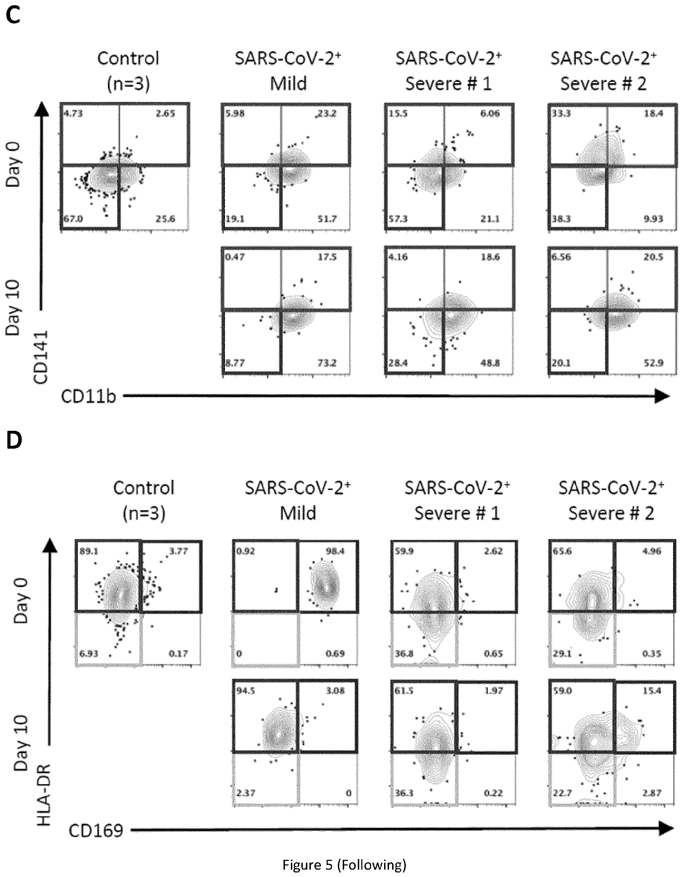

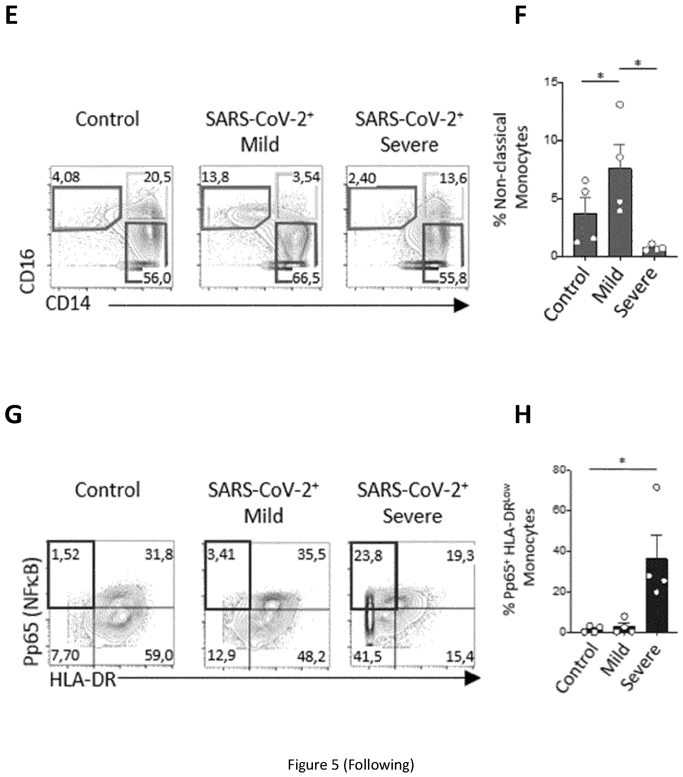

**Figure 5****. Single cell analysis of monocytes by single cell RNA sequencing, spectral flow cytometry and mass spectrometry. A.** UMAP profile of monocytes in the 9 samples analyzed by scRNAseq, and repartition of cell clusters in patient samples collected at day 0 and day 10,; **B-D**. Analysis of monocyte subset partition based on CD14 and CD16 expression among all monocytes (**B**), CD11b and CD141 expression among classical monocytes (**C**), and CD169 and HLA-DR expression among classical monocytes (**D**), in combined controls and each patient at sampling points on days 0 and 10; **E-H.** Mass spectrometry analysis of monocyte subsets in 4 controls, 4 mild and 4 severe COVID-19 patients; monocyte subset partition analysis in each group (individual data pooled), based on CD14 and CD16 expression (**E**); Percentage of non-classical monocytes among total monocyte in each group (**F**); p65/NF-□B expression in HLA-DR^{low} classical monocyte subsets in each group (individual data pooled) (**G**); Percentage of p65/NF-□B^{high}HLA-DR^{low} classical monocytes among classical monocytes in each group (**H**).

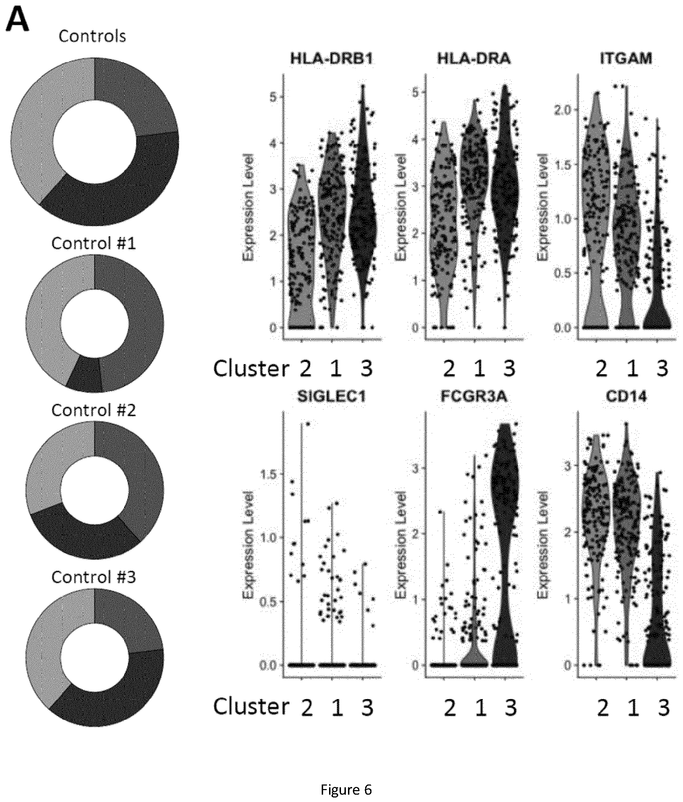

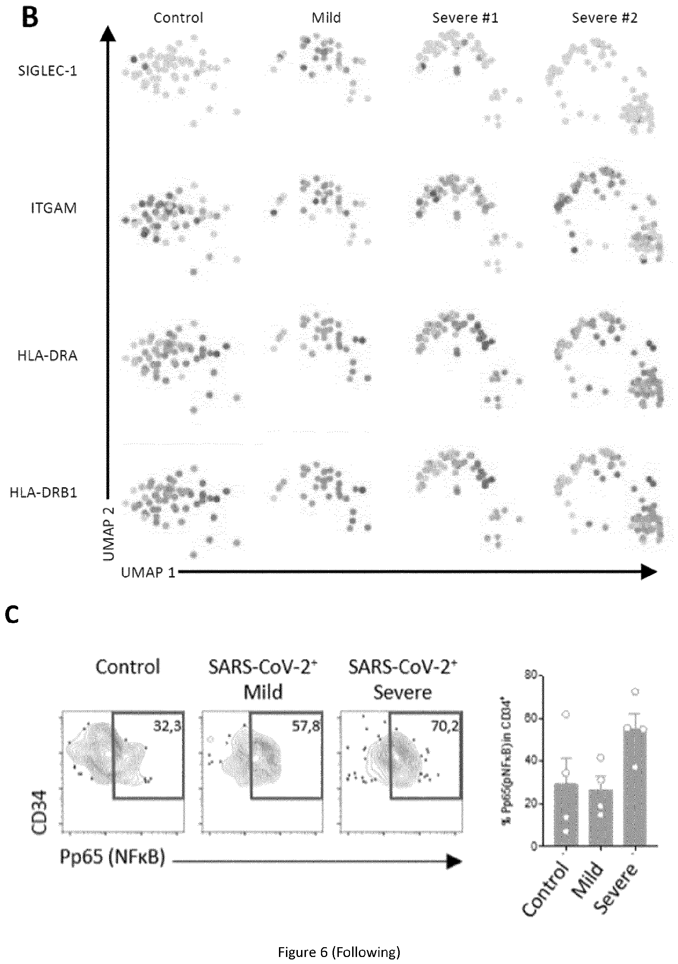

**Figure 6****. Monocyte analysis by single cell RNA sequencing, spectral flow cytometry and mass spectrometry. A.** UMAP analysis of monocyte clusters defined by scRNAseq analysis in the three controls together and each individual control; differential expression of indicated genes in monocyte clusters suggests non-classical (cluster 3, high expression of *FCGR3A*, low *CD14),* intermediate (expression of both *FCGR3A* and *CD14)* and classical (cluster 2, high expression of CD14, no expression of FCGR3A) monocyte subsets; **B.** Expression of indicated genes in monocyte clusters of the three controls combined and in each individual patient; **C.** Combined (left panel) and individual analysis (right panel) of p65/NF-□B expression in circulating CD34⁺ cells in each group.

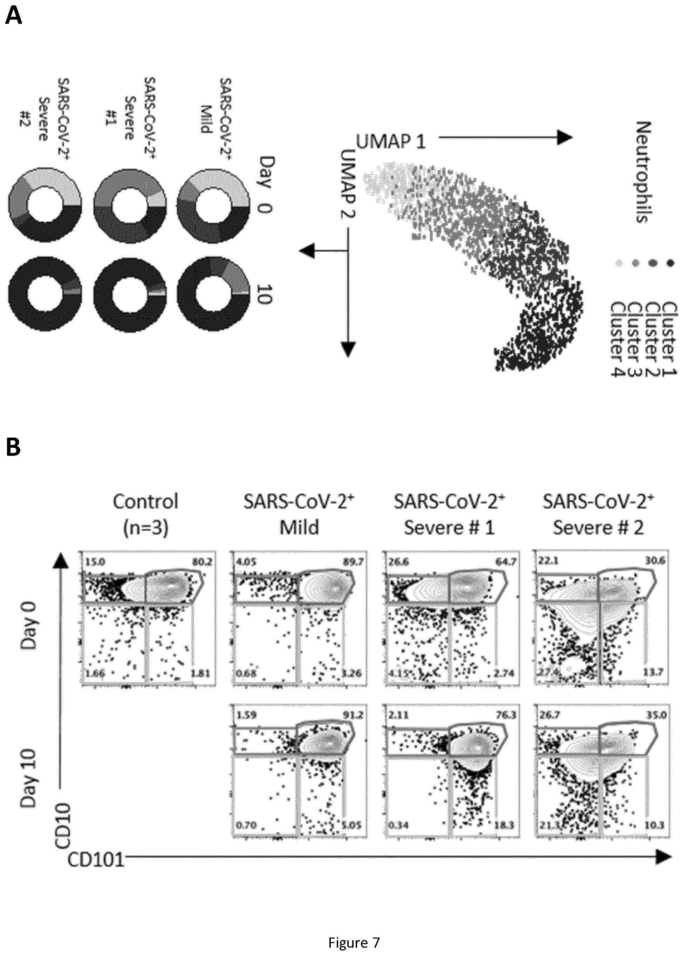

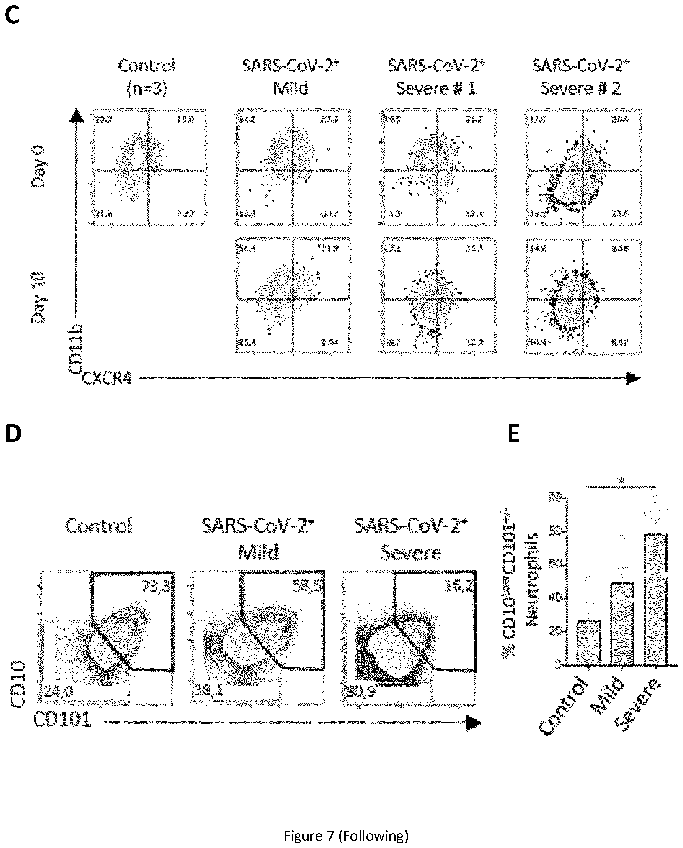

**Figure 7****. Single cell analysis of neutrophils by single cell RNA sequencing, spectral flow cytometry and mass spectrometry. A.** UMAP profile of neutrophils in the 9 samples analyzed by scRNAseq, and partition of cell clusters in patient samples collected at days 0 and 10; **B-C** Analysis of neutrophil subsets, based on CD10 and CD101 expression (**B**), and CXCR4 and CD11b expression among CD10^{Low}CD101⁻ neutrophils (**C**) in the indicated samples (data from the three controls combined). **D-E.** Mass spectrometry analysis of neutrophil subsets showing combined data from the 4 controls, 4 mild and 4 severe COVID-19 patients, based on CD10 and CD101 expression (**D**) and percentage of CD10^{Low}CD101⁻ neutrophils among neutrophils in each group (**E**).

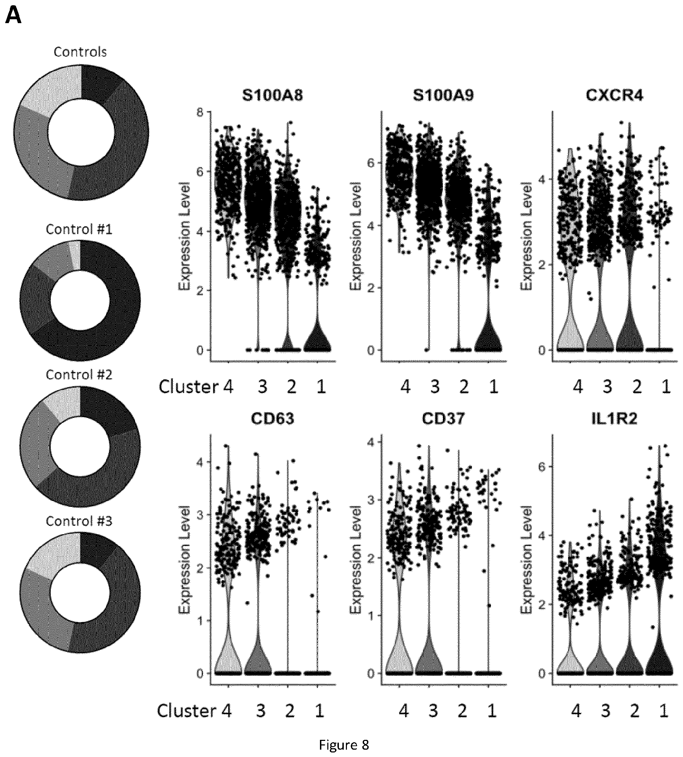

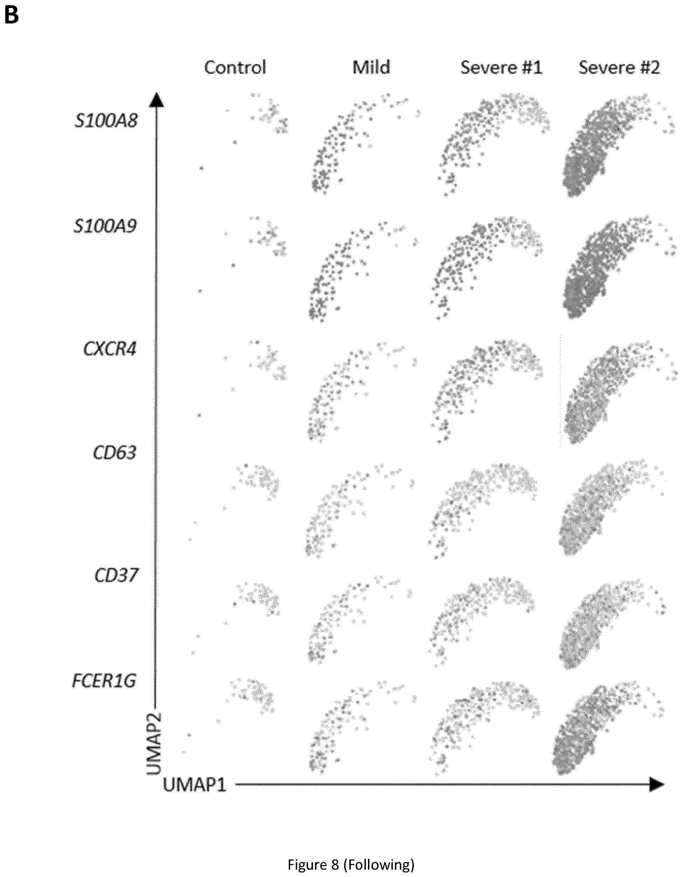

**Figure 8****. Neutrophil analysis by single cell RNA sequencing, spectral flow cytometry and mass spectrometry. A.** UMAP analysis of neutrophil clusters defined by scRNAseq analysis in the three controls together and each individual control; differential expression of indicated genes in neutrophil clusters; **B.** Expression of indicated genes in neutrophil clusters of the three controls and each individual patient.

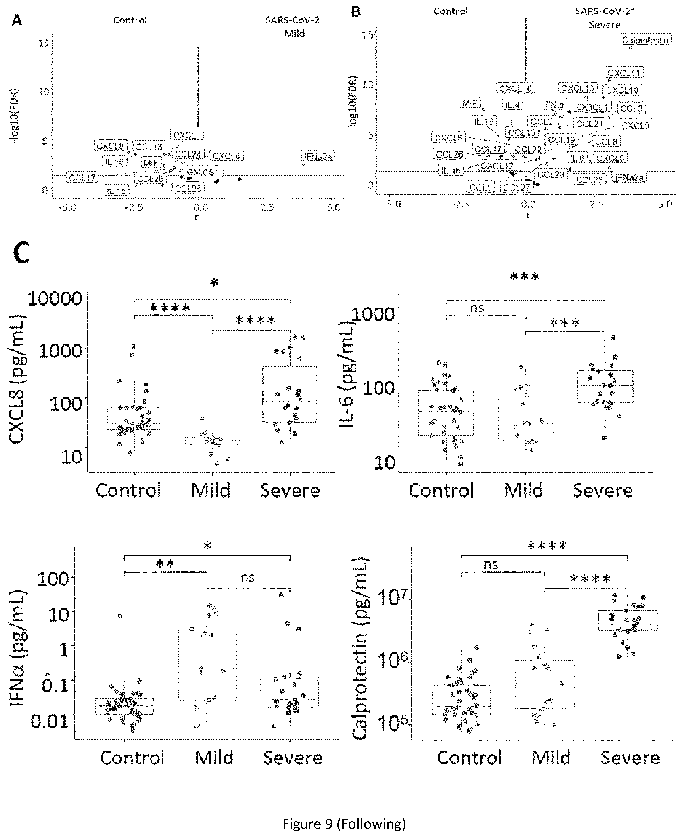

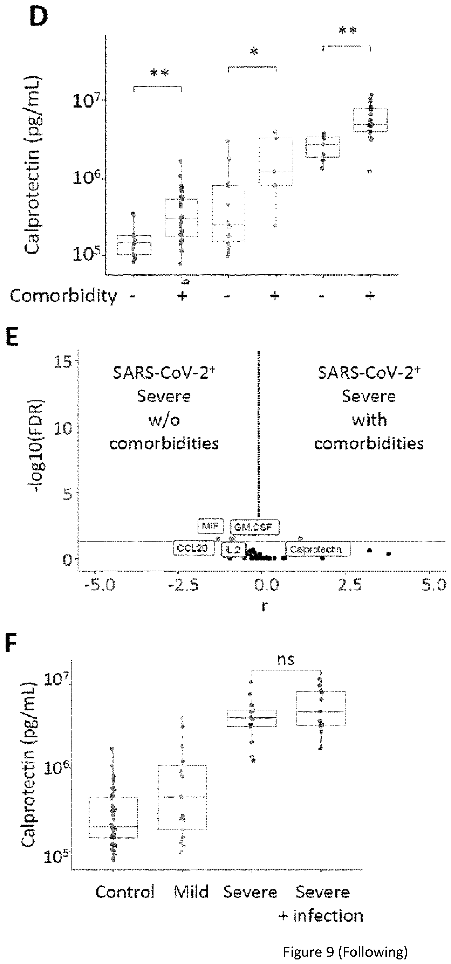

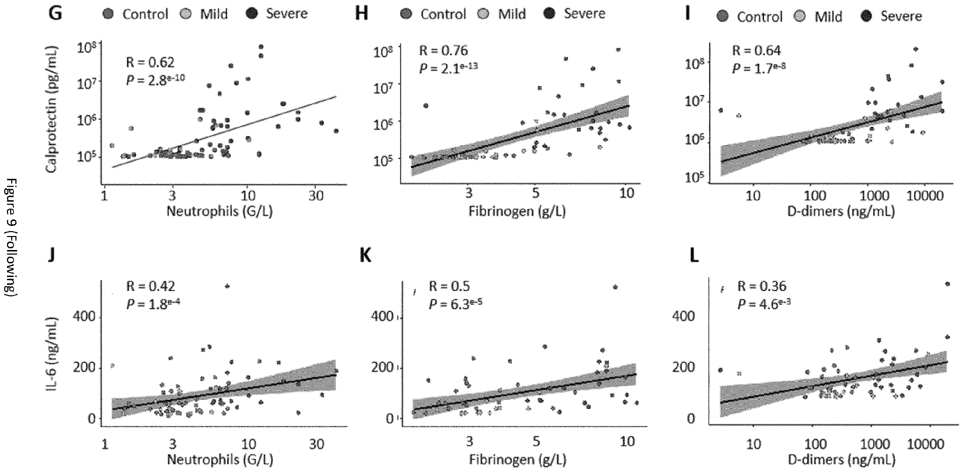

**Figure 9****. Calprotectin is the most abundant immune mediator detected in the plasma of severe COVID-19 patients.** Protein levels of calprotectin (S100A8/S100A9), Interferon alpha (IFN□2a) in addition to 40 cytokines and chemokines were measured in the plasma collected from 84 patients, comprising controls (n=40), outpatients with mild COVID-19 (n=18) and COVID-19 patients with moderate or severe disease (n=25). **A.** Volcanoplot representation of cytokine levels in mild COVID-19 compared to control patients; **B.** Volcanoplot representation of cytokine levels in severe COVID-19 compared to control patients; **C.** Circulating levels of CXCL8, IFN□2a, calprotectin and IL-6 measured in the three studied groups; **D.** Impact of comorbidities (listed in **Table 9**) on calprotectin level in the three studied groups; **E.** Volcanoplot representation of cytokine levels in severe COVID-19 patients with and without comorbidities; **F.** Calprotectin plasma levels in control, mild and severe COVID-19 without and with bacterial infection; **G-I.** Spearman correlations between calprotectin plasma level and **G.** neutrophil count, **H.** fibrinogen, and **I.** D-dimers; **J-L.** Spearman correlations between IL-6 plasma level and **J.** neutrophil count, **K.** fibrinogen, and **L.** D-dimers. Wilcoxon rank-sum test, * *P*<0.05; ** *P* <0.01; *** *P*<0.001; **** *P*<0.0001; ns, non-significant.

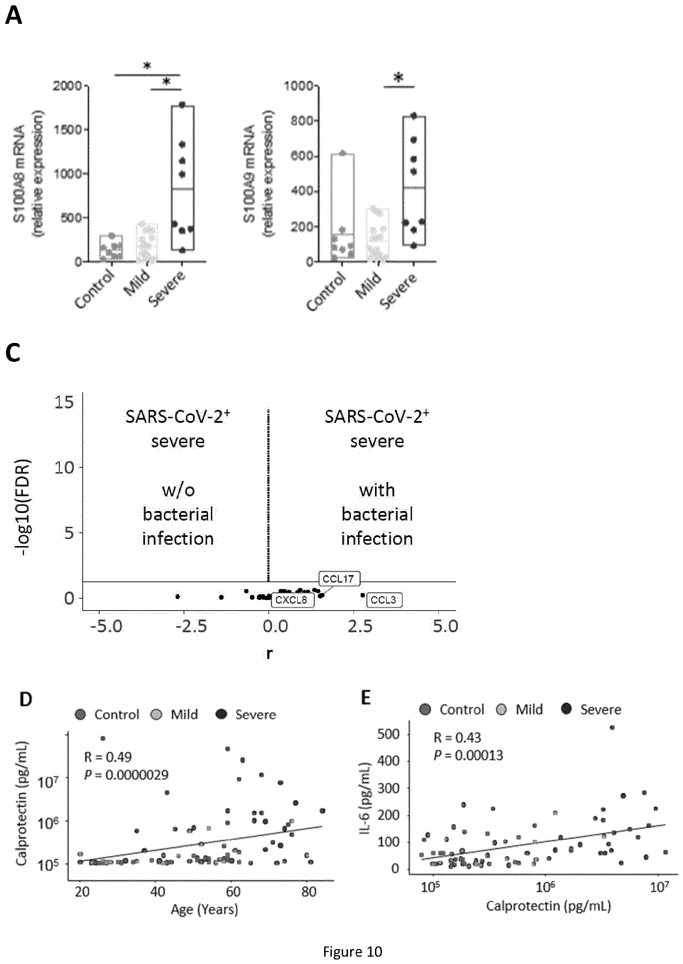

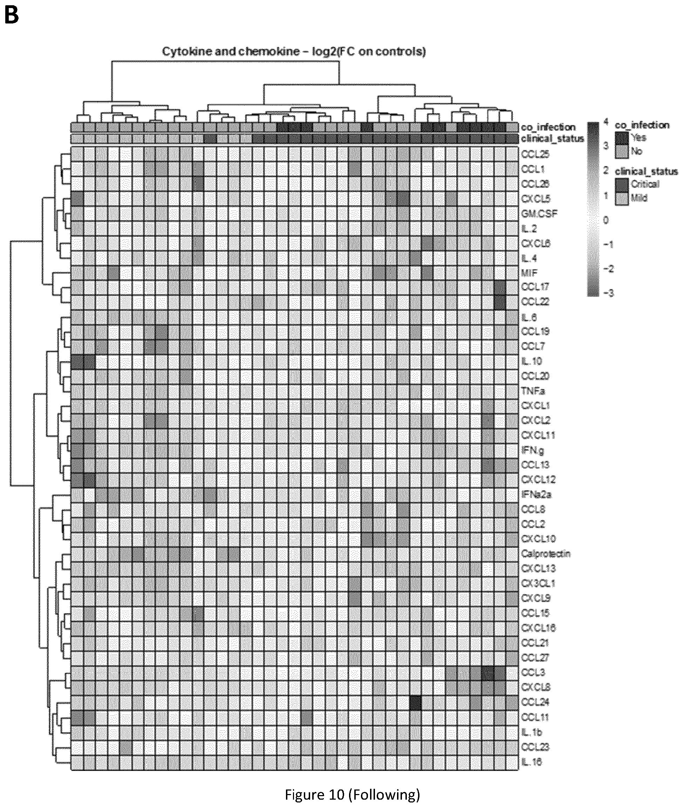

**Figure 10****. Calprotectin is the most abundant immune mediator/immune protein detected in the plasma of severe COVID-19 patients. A.** RT-qPCR analysis of S100A8 and S100A9 gene expression in the three groups of patients, using HPRT as a control gene; **B.** Heatmap of cytokines, chemokines, IFN□ and calprotectin plasma levels in 37 COVID-19 patients compared to 40 controls. SARS-CoV-2 positive patients included 14 mild and 23 severe patients. Associated bacterial infection at sample collection is indicated in purple. The heatmap shows z score-normalized concentrations, each column represents one patient and each row one protein; the color gradient from blue to red indicates increasing concentrations. Rows and columns are clustered using correlation distance and average linkage; **C.** Volcano-plot representation of cytokine levels in severe SARS-CoV-2 patients with or without bacterial infection at the time of sample collection; **D.** Spearman correlation between calprotectin concentration and age showing control patients in green, mild in orange and severe in red; **E.** Spearman correlation between IL-6 and calprotectin concentrations (color code as in D).

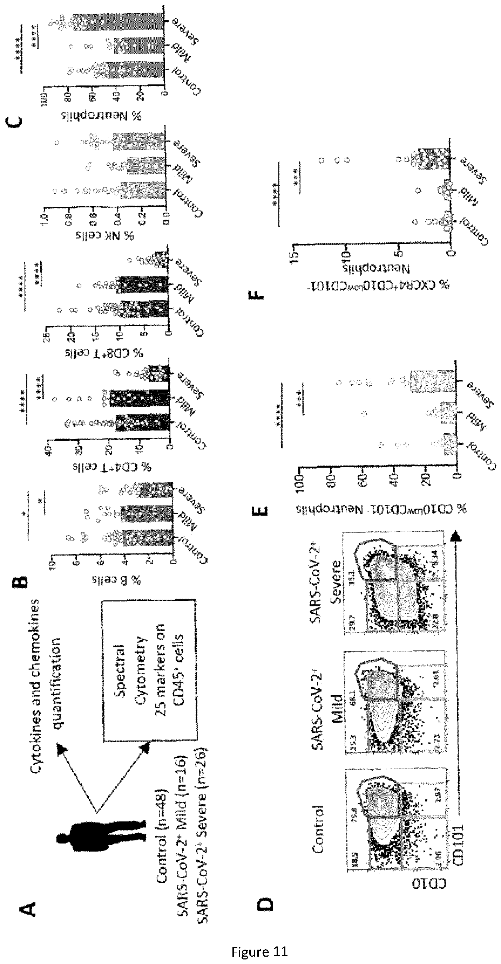

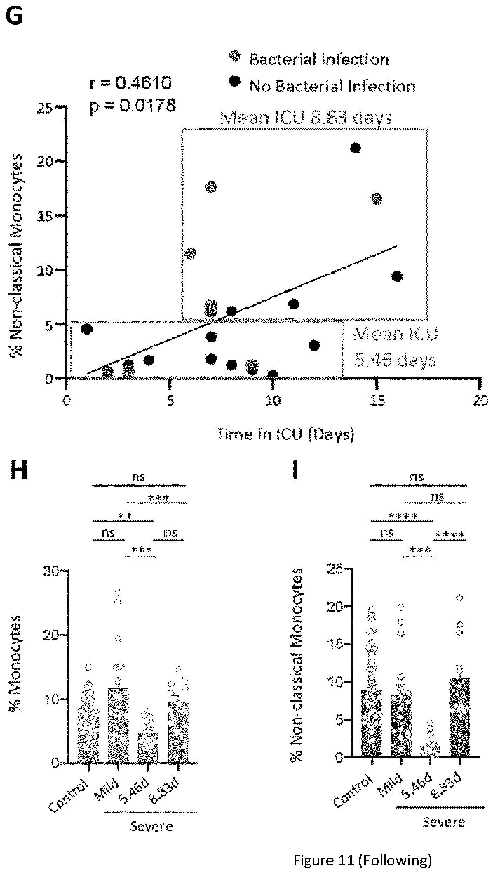

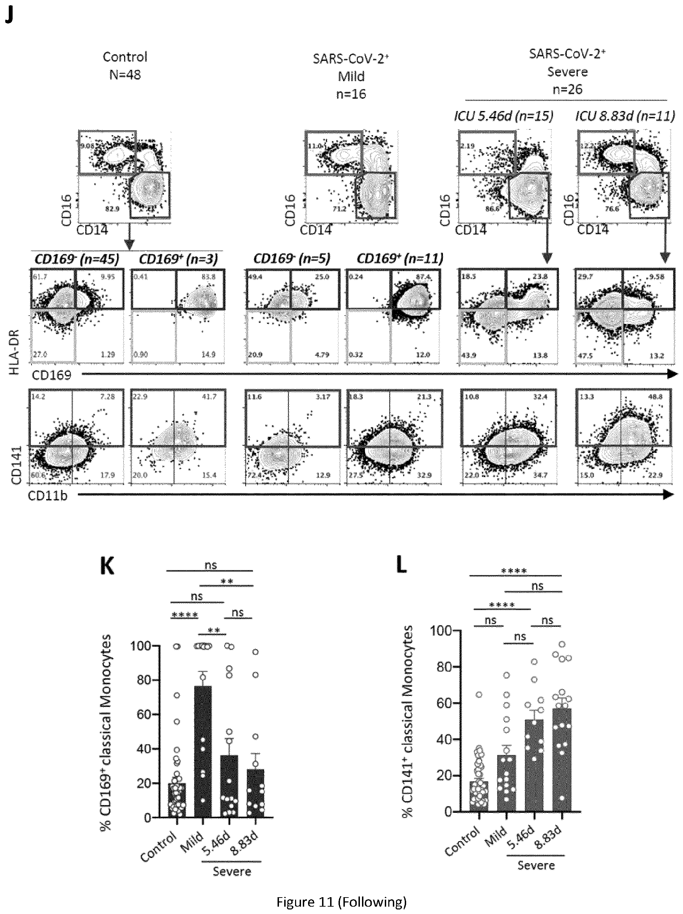

**Figure 11****. Validation of severe COVID-19 innate immune signature. A.** Spectral flow analysis of peripheral blood cells from controls and patients with mild or severe disease. **B.** Percentage of **B** cells, CD4⁺ T cells, CD8⁺ T cells and NK cells among total cells in each individual sample (circles) of the three groups; **C.** Percentage of neutrophils in individual samples from each group; **D.** Combined data from the three patient groups showing neutrophil subsets identified by CD101 and CD10 expression; **E.** Percentage of CD10^{Low}CD101⁻ neutrophils among total neutrophils in individual samples from each group; **F.** Percentage of CXCR4⁺ neutrophils among CD10^{Low}CD101⁻ neutrophils in individual samples from each group; **G.** Spearman correlation of time spent in ICU and percentage of non-classical monocytes among total monocytes; patients with a bacterial infection are shown as red dots. Mean time spent in ICU, 5.46 days in patients with a low (≤5%) and 8.83 days in those with a high (<5%) percentage of CD14^{low}CD16^{High} monocytes; **H.** Percentage of classical monocytes among white blood cells in each individual sample (circles) in the three groups; **I.** Percentage of non-classical monocytes among total monocytes in each individual sample (circles) in the three groups; J. Combined data showing monocyte subset partition in the three groups of patients, the critical group being split in two groups, based on mean time spent in ICU; upper panel shows monocyte subsets identified by CD14 and CD16 expression; lower panels analyze the expression of HLA-DR and CD169 or CD11b and CD141 in classical CD14⁺CD16⁻ monocyte subset; **K.** Percentage of monocytes expressing CD169 among classical monocytes in each individual sample (circles) in the three groups; **L.** Percentage of monocytes expressing CD141 among classical monocytes in each individual sample (circles) in the three groups; Kruskal-Wallis test, * *P*<0.05; ** *P* <0.01; *** *P*<0.001; ns, non-significant.

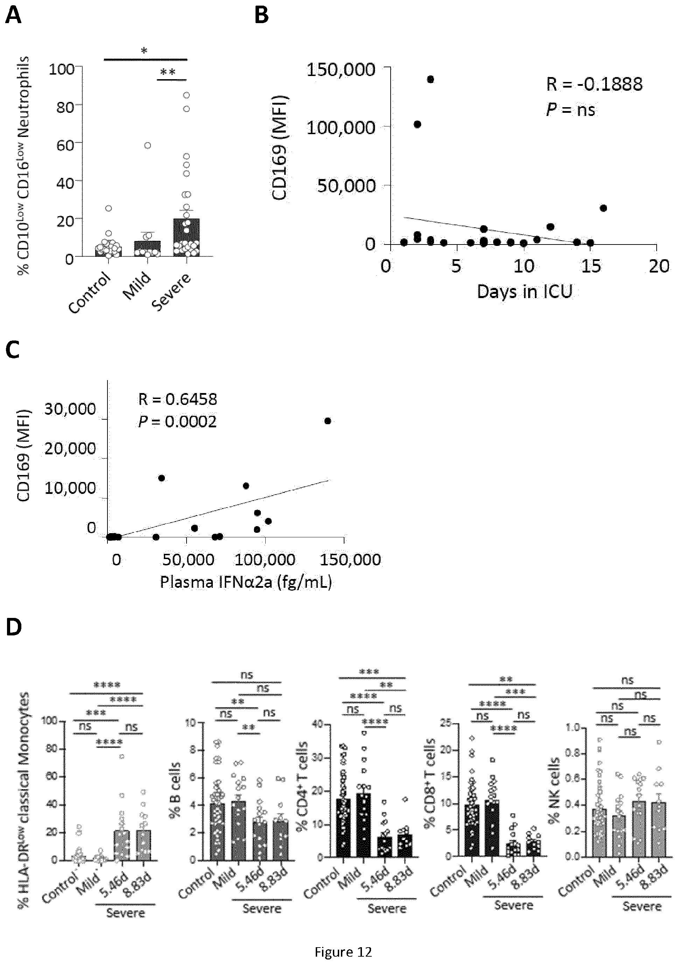

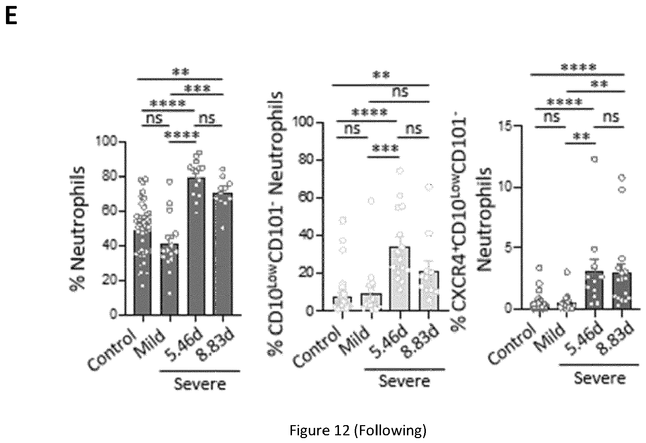

**Figure 12****. Validation of innate immune signature of severe COVID-19. A** Bar plots representing the percentage of CD10^{Low}CD16^{Low} neutrophils among all neutrophils in individual patients from each group in the validation cohort (n=90). **B.** Spearman correlation between CD169 (*SIGLEC-1*) mean fluorescence intensity (MFI) and days spent in ICU. **C.** Spearman correlation between CD169 (*SIGLEC-1*) mean fluorescence intensity (MFI) and plasma IFN□ concentration. **D.E.** Bar plots representing the percentage of HLA-DR^{Low} classical monocytes, **B** cells, CD4⁺ and CD8⁺ T cells and NK cells (**D**) and neutrophils among CD45+ cells, CD10^{Low}CD101⁻ neutrophils among all neutrophils and CD10^{Low}CXCR4⁺ neutrophils among CD10^{Low}CD101⁻ neutrophils (**E**) in individual patients from each group in the validation cohort (n=90).

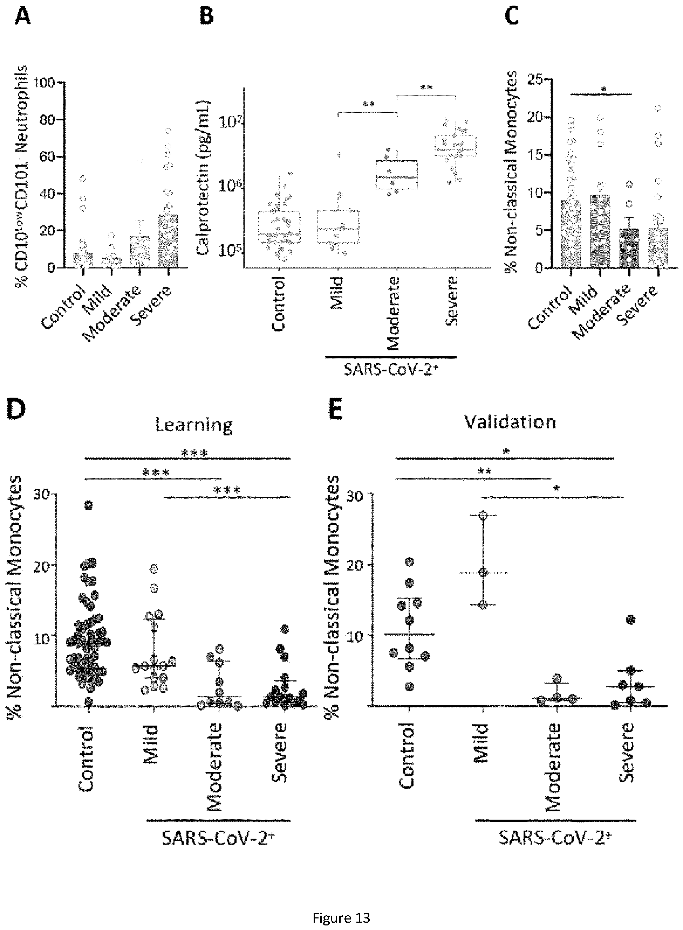

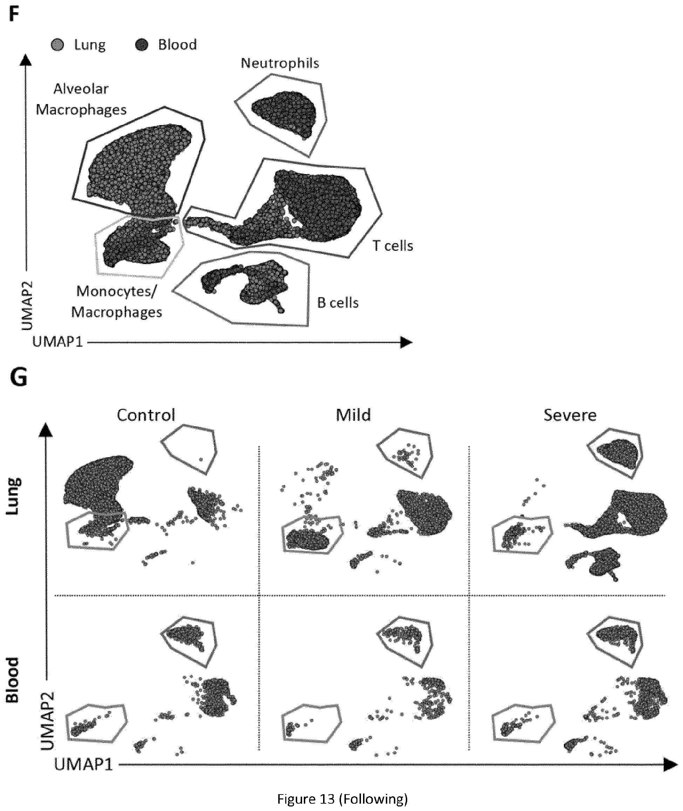

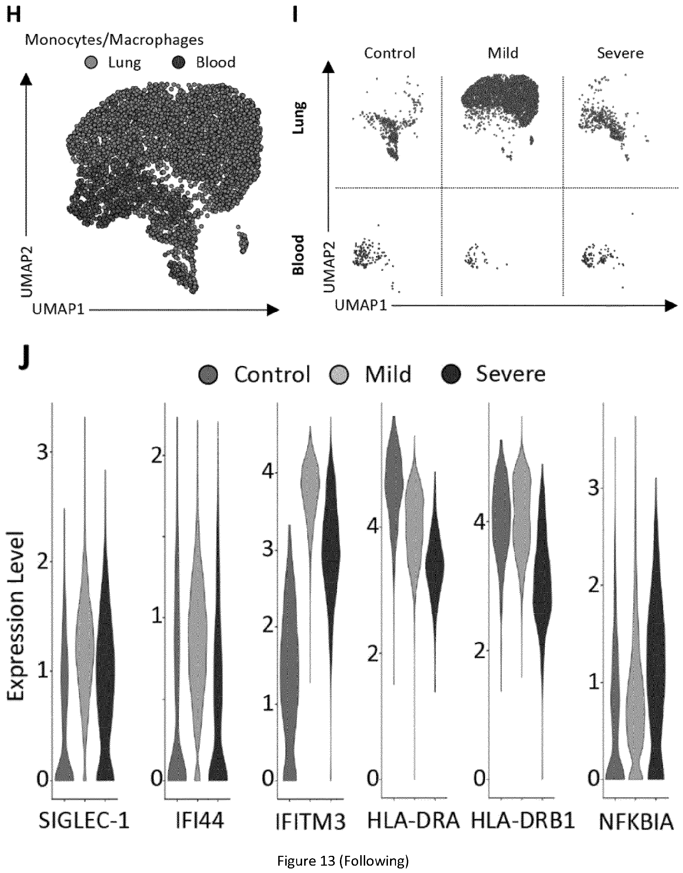

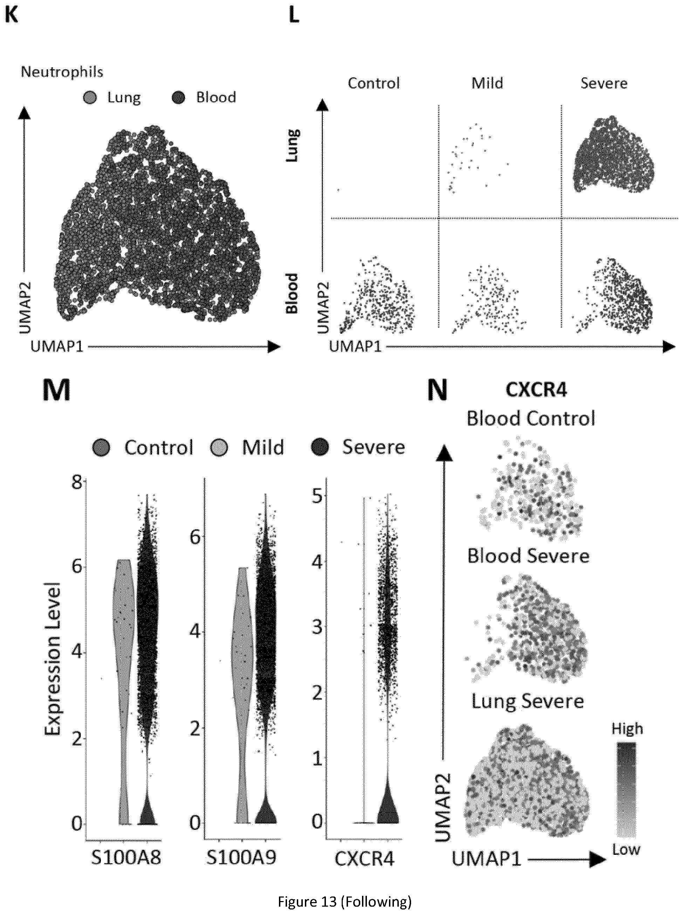

**Figure 13****. Low dimensional flow analysis of non-classical monocyte subsets in COVID-19. A.** Percentage of CD10^{Low}CD101⁻ neutrophils among total neutrophils in individual samples from each group; **B.** Concentration of calprotectin in plasma of moderate COVID-19 patients (dark grey dots) compared to the three other groups; **C.** Percentage of non-classical monocytes among total monocytes in samples with moderate disease (dark grey bar plot) compared to the other three groups; **D-E.** Percentage of non-classical monocytes among monocytes in a learning cohort of 98 patients (controls, n=56; mild, n=16; moderate, n=10; severe, n =16) (**D.**); and a validation cohort of 24 patients (controls, n=10; mild n=3; moderate n=4, severe n=7) (**E**); * Mann Whitney test, * *P*<0.05; ** *P* <0.01; *** *P*<0.001; **F-M.** Integration of scRNAseq data from cells of blood and lung (bronchoalveolar lavage fluid) in COVID-19 patients. Blood scRNAseq samples are detailed in **Table 4;** lung scRNAseq data are from Liao et al, and comprises data from 3 control patients, 3 mild COVID-19 patients and 6 severe COVID-19 patients; **F.** UMAP analysis of integrated scRNAseq from blood and lung samples. **G.** UMAP analysis of blood and lung samples in each patient group; **H.** UMAP analysis of integrated scRNAseq data from monocytes/macrophages from blood and lung; **I.** UMAP analysis of blood and lung monocytes/macrophages in each patient group; **J.** Violin plot representation of gene expression in lung monocytes/macrophages of control, mild and severe COVID-19 patients; **K.** UMAP analysis of integrated scRNAseq data of neutrophils from blood and lung samples; **L.** UMAP analysis of blood and lung neutrophils in each patient group. **M.** Violin plot representation of gene expression in lung neutrophils of control, mild and severe COVID-19 patients; **N.** UMAP analysis of neutrophils with *CXCR4* gene expression level projection (low expression = grey dots; high expression = dark blue dots).

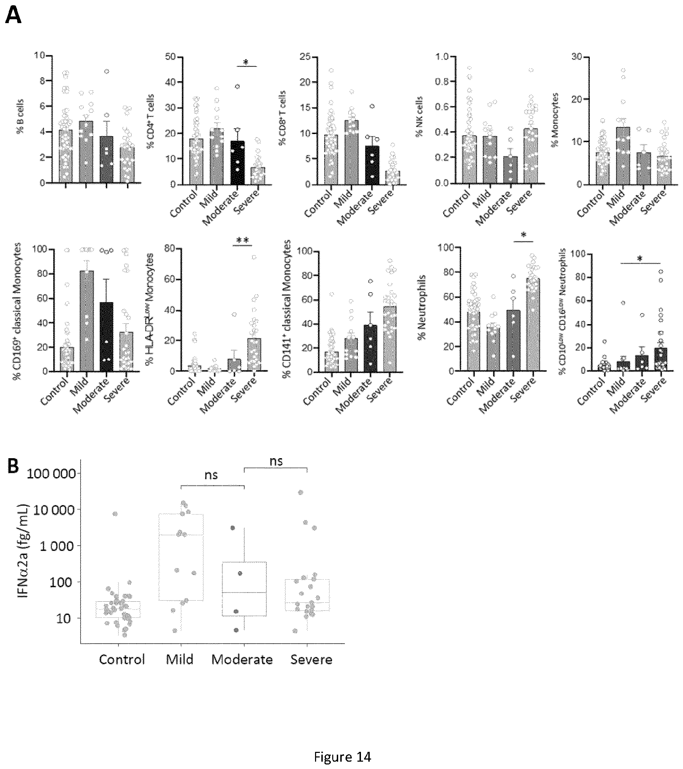

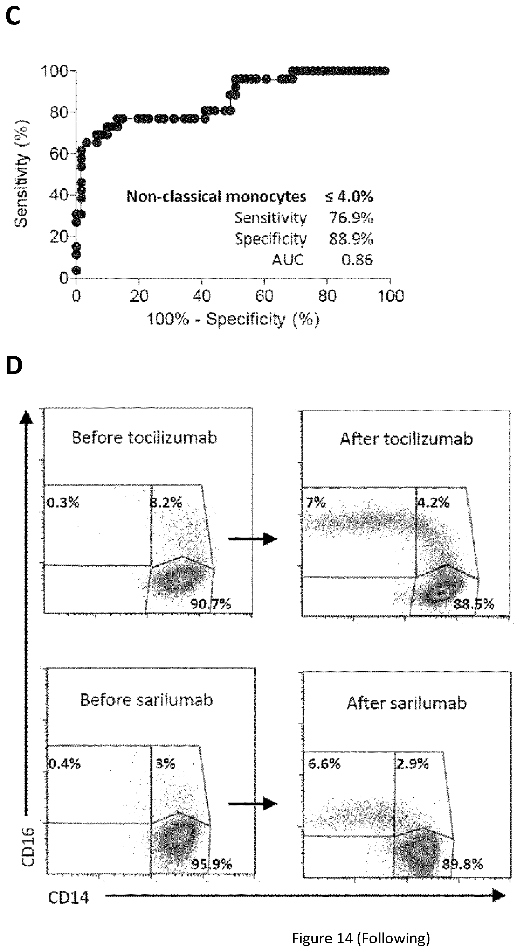

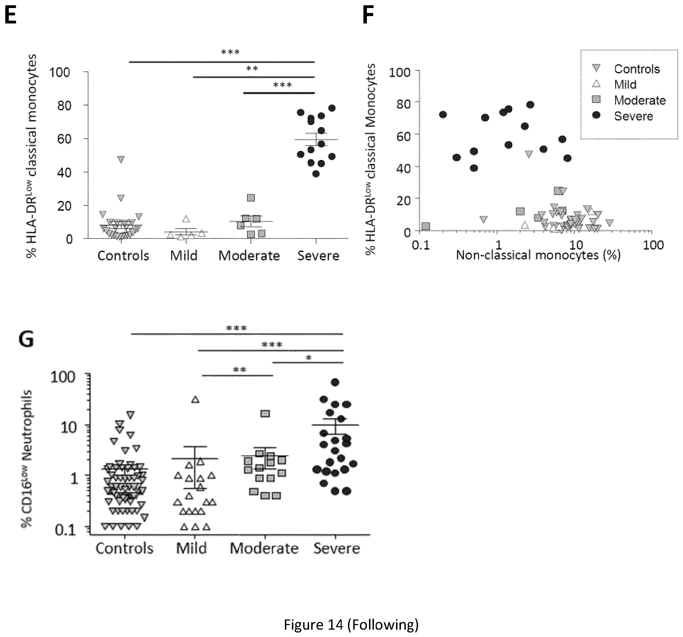

**Figure 14****. Changes in innate immune cell phenotype are detected in moderate COVID-19 patients. A.** Bar plots representing the percentage of B cells, CD4⁺ T cells, CD8⁺ T cells, NK cells, total monocytes, CD169⁺, HLA-DR^{Low} and CD141⁺ classical monocyte subsets, total neutrophils among CD45⁺ cells, and CD10^{Low}CD101⁻ and CD10^{Low}CD16^{Low} neutrophil subset among all neutrophils in individual patients from each group, with the moderate category (6 patients) highlighted. **B.** Plasma concentration of IFN□ in moderate COVID-19 patients compared to the three other groups. **C.** ROC analysis showing performance of a diagnostic test using percentage of non-classical monocytes among total monocytes to distinguish controls and mild COVID patients from moderate and severe COVID patients; **D.** Monocyte subset analysis in the peripheral blood of 2 severe patients, before (left panels) and after (right panels) treatment with the indicated anti-IL-6 antibodies; **E.** Percentage of HLA-DR^{low} classical monocytes among classical monocytes in a cohort of 22 patients and 17 controls grouped into 4 clinical categories; **F.** Correlation between the percentage of HLA-DR^{low} classical monocytes and non-classical monocytes; **G.** Percentage of CD16^{low} neutrophils among neutrophils in control and COVID-19 patients of the learning cohort described in **Figure 14****.** Mann Whitney test, * *P*<0.05; ** *P* <0.01; *** *P*<0.001.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

The examples below show that calprotectin-driven emergency myelopoisis generates an innate immune cell signature of severe COVID-19.

### 1. Patients and Methods

### 1.1. Patients

This non-interventional study received approval by institutional review boards of Cochin-Port Royal (Paris, France) and Gustave Roussy (Villejuif, France) hospitals and the ethical committee of Cochin-Port Royal Hospital (CLEP Decision N°: AAA-2020-08023) and conformed to the principles outlined in the Declaration of Helsinki. Controls (n = 72) were symptomatic patients who were seen at Hotel-Dieu or Gustave Roussy COVID-19 screening unit and were negative for SARS-CoV-2 RT-PCR on pharyngeal swab. Mild COVID-19 patients (n = 27) were defined by having limited clinical symptoms (fever, cough, diarrhea, myalgia, anosmia/ageusia) that did not require CT-scan or hospitalization. Moderate cases (n = 16) were defined as symptomatic patients with dyspnea and radiological findings of pneumonia on thoracic CT scan, requiring hospitalization and a maximum of 9 L/min of oxygen. In the larger part of this study, mild and moderate cases were analyzed together and grouped under "mild category". Severe patients (n = 43) were those hospitalized in the ICU with respiratory distress requiring more 10L/min of oxygen or more without endotracheal intubation, and mechanical ventilation.

**Table 1 - Main characteristics of all included patients**

| | All patients | SARS-CoV-2 negative Controls | SARS-CoV-2 positive Mild | SARS-CoV-2 positive Moderate | SARS-CoV-2 positive Severe |
|---|---|---|---|---|---|
| Number | 158 | 72 (46) | 27 (17) | 16 (10) | 43 (27) |
| Age (median, IQR) | 53 (37-63) | 50 (31-58) | 42 (29-56) | 64 (53-72) | 62 (50-69) |
| Age > 60 year-old | 48 (30) | 12 (17) | 3 (11) | 11 (69) | 22 (51) |
| Male, n (%) | 69 (44) | 27 (38) | 8 (30) | 5 (31) | 29 (67) |

| Comorbidities, n (%) | | | | | |
|---|---|---|---|---|---|
| Overweight | 78 (49) | 33 (46) | 9 (33) | 5 (31) | 31 (72) |
| Cardiovascular | 55 (35) | 20 (28) | 5 (19) | 10 (62) | 20 (47) |
| Diabetes | 29 (18) | 9 (13) | 1 (4) | 6 (38) | 13 (30) |
| Respiratory | 31 (20) | 17 (24) | 1 (4) | 3 (19) | 10 (23) |
| Cancer | 34 (22) | 13 (18) | 5 (19) | 7 (44) | 9 (21) |

| Initial symptoms, n (%) | | | | | |
|---|---|---|---|---|---|
| Fever | 56 (35) | 10 (14) | 4 (15) | 11 (69) | 31 (72) |
| Cough | 86 (54) | 31 (43) | 16 (59) | 10 (62) | 29 (67) |
| Dyspnea | 46 (29) | 3 (4) | 2 (7) | 7 (44) | 34 (79) |
| Myalgia | 49 (31) | 27 (38) | 11 (41) | 1 (6) | 10 (23) |
| Diarrhea | 35 (22) | 14 (19) | 6 (22) | 5 (31) | 10 (23) |
| Anosmia/Ageusia | 30 (19) | 11 (15) | 10(37) | 2 (13) | 7 (16) |

| Laboratory findings median, IQR) | | | | | |
|---|---|---|---|---|---|
| Leukocytes (G/L) | 7.1 (5.6-10.3) | 6.7 (5.6-8.5) | 5.6 (4.3-6.8) | 5.7 (4.7-8.9) | 10.4 (7.9-14.6) |
| Neutrophils (G/L) | 4.4 (3.0-7.4) | 3.7 (2.9-5.2) | 2.8 (2.3-3.5) | 4.6 (3.4-6.1) | 7.8 (6.4-11.2) |
| Lymphocytes | 1.6 (1.0-2.1) | 1.9 (1.5-2.3) | 1.6 (1.0-2.1) | 1.0 (0.7-1.3) | 1.1 (0.7-1.7) |
| Monocytes (G/L) | 0.5 (0.4-0.8) | 0.5 (0.4-0.6) | 0.5 (0.4-0.7) | 0.4 (0.2-0.7) | 0.8 (0.4-1.1) |
| Hemoglobin (g/dL) | 12.6 (9.9-13.9) | 13.7 (12.8-14.7) | 13.0 (11.3-13.6) | 10.5 (9.6-13.0) | 9.8 (8.1-11.9) |
| Platelets (G/L) | 255 (205-320) | 257 (227-303) | 210 (171-296) | 191 (123-323) | 273 (210-407) |
| Fibrinogen (g/L) | | | | 5.4 (5.0-6.3) | 7.5 (5.5-8.0) |
| D-dimers (ng/mL) | | | | 1089 (666-1954) | 2145 (1285-5331) |
| CRP (mg/L) | | | | 50.5 (30.6-98.0) | 142.8 (91.1-194.5) |
| IL-6 (ng/L) | | | | 36.0 (15.4-48.5) | 89.3 (48.8-157.6) |
| Ferritin (mg/L) | | | | 858 (598-858) | 959 (454-1828) |
| LDH (Ul/L) | | | | 240 (203-310) | 400 (328-460) |

| Chest CT findings, n (%) | | | | | |
|---|---|---|---|---|---|
| <10% | 3 (4) | | | 1 (6) | 0 (0) |
| 10-25% | 12 (8) | | | 6 (38) | 4 (2) |
| 25-50% | 29 (18) | | | 7 (44) | 18 (42) |
| >50% | 20 (13) | | | 1 (6) | 18 (42) |
| NA | 103 (65) | 72 (100) | 27 (100) | 1 (6) | 3 (7) |

| Anti-inflammatory drugs, n | | | | | |
|---|---|---|---|---|---|
| Dexamethasone | 6 | | | 2 | 4 |
| anti-IL1 (anakinra) | 3 | | | 1 | 3 |
| anti-IL6 (sari, toci) | 9 | | | 2 | 7 |

| Severity criteria | | | | | |
|---|---|---|---|---|---|
| PE, n (%) | 11 (7) | | | 3 (19) | 8 (19) |
| Maximal O2 | | | | 2 (0-9) | 15 (6-50) = 12/13 |
| Mechanical ventilation, n | | | | | 30 |
| PaO2/FiO2, med (range) | | | | | 157 (75-453) |
| SOFA, med (range) | | | | | 5.5 (2-12) |
| SOFA ≥ 8, n (%) | | | | | 10 (33) |
| Death, n (%) | 10 (6) | 0 (0) | 1 (96)** | 0 (0) | 9 (21) |

| Sampling delay (days), n (%) | | | | | |
|---|---|---|---|---|---|
| 1^{st} day hosp ≤ 10 d | 150 (95) | 71 (99) | 27 (100) | 16 (100) | 36 (84) |

| | | | | | |
|---|---|---|---|---|---|
| A total number of 158 patients were included. Samples collected from a given patients have been used in several experiments. Sub-cohorts are described in supplemental tables. IQR, interquartile range; CRP, C reactive protein; LDH, Lactate deshydrogenase; CT: computed tomography scan; NA, not available; sari, sarilumab ; toci, tocilizumab; PE, pulmonary embolism; SOFA, Sequential Organ Failure Assessment score; PaO2/FiO2 (mmHg) at sampling; med, median (range); "1st day hosp ≤ 10 d" indicates the number of patients including in the study in the first 10 days after admission to the hospital. | | | | | |

### 1.2. Sampling

Whole human peripheral blood was collected in sterile vacutainer tubes containing EDTA or heparin. Except for single cell RNA sequencing, tubes were centrifuged at 300 g for 5 min at room temperature and plasma was collected. Whole blood was mixed at a ratio 1:1 with Whole Blood Cell Stabilizer (Cytodelics), incubated at room temperature for 10 min and transferred to -80°C freezer awaiting analysis. These samples were secondarily thawed in a water bath set to +37°C. Cells were fixed at a ratio 1:1 with Fixation Buffer (Cytodelics, ratio 1:1) and incubated for 10 min at room temperature. Red blood cells were lysed by addition of 2 mL of Lysis Buffer (Cytodelics, ratio 1:4) at room temperature for 10 min. White blood cells were washed with 2 mL of Wash Buffer (Cytodelics, ratio 1:5).

### 1.3. Spectral flow Cytometry

Cells were resuspended in 100 µL extra-cellular antibody cocktail and incubated at room temperature for 15 min. For intra-cellular staining, a step of permeabilization was performed using 200 µL of BD Cytofix/Cytoperm Kit (BD), cells were incubated for 40 min at +4°C, washed in Perm Buffer (BD) and resuspended in intra-cellular antibody cocktail. After incubation, cells were washed in Flow Cytometry Buffer (1% BSA, 0.5% Na-Azide and 0.5M EDTA in PBS) and resuspended to proceed to the acquisition. All antibodies used are listed in **Table 3** Samples were acquired on CyTEK Aurora flow cytometer (Cytek Biosciences). Fcs files were exported and analyzed using FlowJo software.

### 1.4. 3' scRNA-seq analysis of human blood cells.

To fully capture peripheral blood cell heterogeneity, fresh samples were analyzed without cell sorting and freezing and without Ficoll enrichment, minimizing time of incubation and processing. Sample preparation was done at room temperature. After red cell lysis, single-cell suspensions were loaded onto a Chromium Single Cell Chip (10x Genomics) according to the manufacturer's instructions for co-encapsulation with barcoded Gel Beads at a target capture rate of ∼7000 individual cells per sample. To analyze neutrophils, RNase inhibitor (RNAse OUT Recombinant Ribonuclease Inhibitor Invitrogen, 40U/mL) was added into the loading buffer. Captured mRNAs were barcoded during cDNA synthesis using the Chromium Single Cell 3' Solution v3 (10x Genomics) according to the manufacturer's instructions. Of note, we increase the PCR cycles by 2 extra cycles during cDNA amplification. All samples (at D0 and D10) were processed simultaneously with the Chromium Controller (10x Genomics) and the resulting libraries were prepared in parallel in a single batch. We pooled all of the libraries for sequencing in a single SP Illumina flow cell. All of the libraries were sequenced with an 8-base index read, a 28-base Read1 containing cell-identifying barcodes and unique molecular identifiers (UMIs), and a 91-base Read2 containing transcript sequences on an Illumina NovaSeq 6000.

### 1.5. Integration of scRNAseq dataset from bronchoalveolar lavage fluid of COVID-19 patients.

The bronchoalveolar dataset was downloaded from the NIH GEO database (Liao et al, dataset GSE145926) and integrated with our own blood scRNAseq data using the Seurat V3 anchoring method (Stuart et al., 2019). Briefly, the datasets were normalized independently and the highly variable genes were identified for each dataset using the Seurat pipeline. A corrected data matrix with both datasets was then generated using the Seurat v3 anchoring procedure to allow for joint analysis. The matrix was scaled and a Principal Component Analysis (PCA) was performed using the Seurat v3 pipeline. A UMAP was performed on the 30 first Principal Components (PCs) (Becht et al., 2019).

### 1.6. RT-qPCR analysis

Total RNA was extracted with RNeasy Mini Kit (Qiagen) and reverse transcribed with SuperScript^{™} IV VILO^{™} Master Mix with ezDNase^{™} Enzyme (Invitrogen). Real-time quantitative polymerase chain reaction (RT-qPCR) was performed using Power SYBR^{™} Green PCR Master Mix in a BioRad CFX96 thermocycler using the standard SyBR Green detection protocol as outlined by the manufacturer (Applied Biosystems). Briefly, 12 ng of total cDNA, 50nM (each) primers, and 1× SyBR Green mixture were used in a total volume of 20 µL.

Human primer sequences are the following: GUS (F: GAAAATATGTGGTTGGAGAGCTCATT (SEQ ID NO:1); R: CCGAGTGAAGATCCCCT TTTTA (SEQ ID NO:2)); HPRT (F: GGACAGGACTGAACGTCTTGC (SEQ ID NO:3); R: CTTGAGCACACAGAGGGCTACA (SEQ ID NO:4); S100A8 (F: CAACACTG ATGGTGCAGTTAACTTC (SEQ ID NO:5); R: CTGCCACGCCCATCTTTATC (SEQ ID NO:6)); S100A9 (F: CTGAGCTTCGAGG AGTTCATCA (SEQ ID NO:7); R: CGTCACCCTCGTGCATCTTC (SEQ ID NO:8)).

### 1.7. Cytokine and chemokine measurements

Plasma samples were centrifuged 15 min at 1,000 g, diluted 1:4, then monitored using the Bio-Plex ProTM Human Chemokine Panel 40-plex Assay (Bio-rad, ref: 171AK99MR2) according to the manufacturer's instructions. 40-plex cytokines and chemokines provided are: CCL1, CCL11, CCL13, CCL15, CCL17, CCL19, CCL2, CCL20, CCL21, CCL22, CCL23, CCL24, CCL25, CCL26, CCL27, CCL3, CCL7, CCL8, CX3CL1, CXCL1, CXCL10, CXCL11, CXCL12, CXCL13, CXCL16, CXCL2, CXCL5, CXCL6, CXCL8, CXCL9, GM-CSF, IFN-□, IL-10, IL-16, IL-1b, IL-2, IL-4, IL-6, MIF, TNF-α. Acquisitions and analyses were performed on a Bio-Plex 200 system (Bio-rad) and a Bio-Plex Manager 6.1 Software (Bio-rad), respectively. Soluble Calprotectin (diluted 1:100) and IFN-α2a (diluted 1:2) were analyzed using a R-plex Human Calprotectin Antibody Set (Meso Scale Discovery, ref: F21YB-3) and the ultra-sensitive assay S-plex Human IFN-α2a kit (Meso Scale Discovery, ref: K151P3S-1), respectively, following manufacturer's instructions. Acquisitions and analyses of soluble Calprotectin and IFN-α2a were performed on a MESO^{™} QuickPlex SQ120 reader and the MSD's Discovery Workbench 4.0. Each plasma sample was assayed twice with the average value taken as the final result. Data representation was performed with software R v3.3.3 using tidyverse, dplyr, ggplot2, ggpubr, pheatmap, corrplot or Hmisc packages.

### 1.8. Mass Cytometry

Cells were barcoded using the 20-Plex Pd barcoding kit (Fluidigm). Briefly, they were washed in Barcode Perm Buffer, resuspended in 800 µL of Barcode Perm Buffer and 100 µL of each barcode were transferred to the appropriate sample. Cell suspensions were incubated for 30 min at room temperature, washed twice with Cell Staining Buffer (Fluidigm) and pooled, suspended in 100 µL filtered antibody cocktail, and incubated for 30 min at +4°C. All antibodies used are listed in **Table 3.** After staining, cells were washed with Cell Staining Buffer and permeabilized with 200 µL of Fix/Perm from Foxp3/Transcription Factor Staining Buffer kit (eBiosciences), 40 min at +4°C. After incubation, cells were washed in Perm Buffer from Foxp3/Transcription Factor Staining Buffer kit (eBiosciences), resuspended in 100 µL filtered antibody cocktail, incubated for 30 min at +4°C, washed in Cell Staining Buffer and resuspended in 50 µL of CytoFix/Perm for 5 min at room temperature. Then, 400 µL of PBS containing 1.6%PFA + Iridium (1:4000) were added for 35 min at room temperature. Finally, cells were washed in Cell Staining Buffer, resuspended in 50 µL and stored at +4°C until acquisition. Cells were counted, washed and resuspended in Maxpar Cell Acquisition Solution at 0.5x 106 / mL and mixed with 10% EQ Beads immediately before acquisition on Helios mass cytometer using noise reduction, event length limits of 10-150 pushes. An average of 500,000 events were acquired per sample at a flow rate of 0.03mL/min. Mass cytometry standard files were normalized to a global standard determined for each log of EQ beads using CyTOF Software v.6.7.1014 (Fluidigm). Fcs files were exported and analysed using FlowJo software.

### 1.9. Routine multiparameter flow analysis test

Whole-blood samples (200µL) were stained with anti-CD14-PC7 (clone RMO52); CD16-PB (clone 3G8); CD2-FITC (clone 39C1.5); CD56-PC5.5 (clone N901); CD24-PE (clone ALB9); CD45-KO (clone J33) and HLA-DR-APC (clone Immu-357) antibodies, all purchased from Beckman-Coulter. Red blood cells were lysed with 1 mL VersalyseTM (Beckman Coulter) before sample analysis with a Navios Cytometer (Beckman Coulter) as described (Tarfi et al., 2019). Monocytes were selected as CD45^{high}/SSC^{int} cells among living cells and singlets before excluding T cells as CD2⁺/SSC^{low}, NK cells as CD56⁺/SSC^{low/int}, B cells as CD24⁺/SSC^{low}, immature and mature granulocytes as CD24⁺/SSC^{int/high}, CD16^{bright} residual granulocytes, and remaining CD14⁻CD16⁻ cells corresponding mainly to basophils and NK cells not previously excluded. Monocyte subsets were detected on a CD45/SCC dot plot, using a CD14/CD16 scattergram that separates CD14^{++/High}/CD16^{-/Low} (classical), CD14^{++/High}/CD16^{+/High} (intermediate) and CD14^{low/-}/CD16^{+/High} (non-classical) subsets. Finally, the proportion of monocytes HLA-DR^{dim/-} was evaluated on a HLA-DR/CD14 scattergram.

### 1.10. Data analysis

Calculations and statistical tests were performed using R v3.3.3. Unless stated, p-values are two-sided with 95% confidence intervals for the reported statistic of interest. Individual data points representing the measurement from one patient are systematically calculated from the corresponding distribution. Wilcoxon rank-sum test was applied to assess differences in concentration between two different groups. When indicated, the false discovery rate (FDR, p > 0.05) was controlled using the Benjamini-Hochberg procedure. Spearman correlations were computed using the Hmisc R package and cytokine results were shown using R package Pheatmap. Soluble factorsfold ratios were calculated as log2 transformation of values of mild and severe patients versus median value of all controls patients and were converted to z scores. Hierarchical clustering of the patients based Gene ontology networks were made by subjecting the DEGs from previous scRNA sequencing analysis to the Cytoscape addon ClueGO. The selected DEGs were specific to those with increased expression by monocytes and neutrophils from mild or severe SARS-CoV-2 positive patients. Biological Process gene ontologies selected had an FDR < 0.05. Other statistical analyses were performed using GraphPad Prism 7on the zscore of 42 soluble factors was performed using euclidean distance and ward.D clustering.

### 2. Results

### 2.1. Introduction to the studied cohort of patients

This non-interventional study enrolled 158 patients (**Table 1**), including 72 subjects referred to the hospital with various flu-like symptoms but negative for SARS-CoV-2 PCR on pharyngeal swab, and 86 COVID-19 patients, as defined by a positive PCR. COVID-19 patients were stratified according to disease severity: mild disease (n=27) was defined as having limited clinical symptoms, and not requiring CT-scan or hospitalization; moderate disease (n=16) was defined as being symptomatic with dyspnea and radiological findings of pneumonia on thoracic CT scan, requiring hospitalization but with a maximum of 9 L/min of oxygen; and severe disease (n=43) which was defined as respiratory distress requiring admission into the intensive care unit (ICU). Mild and moderate cases were mixed in the discovery part of the study and considered separately at the end of the study to explore the ability of a routine flow assay to discriminate patients that require hospitalization from those with a mild disease.

### 2.1. Circulating innate immune cells in mild and severe COVID-19 patients exhibit distinct phenotypes.

Peripheral blood samples were collected from 13 patients positive for SARS-CoV-2 by RT-PCR and 12 patients suffering from flu-like symptoms but negative for SARS-CoV-2. The former group included 5 patients with mild disease and 8 patients with severe disease admitted to the intensive care unit (ICU) (**Table 2**). After red blood cell lysis, peripheral blood cells were labelled with a panel of 25 antibodies recognizing immune cell surface markers (**Table 3**) and analyzed them by spectral flow cytometry. By pooling the data from the 25 control and COVID-19 patients and subjecting them to dimensionality reduction using the non-supervised UMAP algorithm (Becht et al, 2018), populations of CD3⁺CD4⁺ T cells, CD3⁺CD8⁺ T cells, CD19⁺ B cells, CD14^{High} monocytes and CD15⁺CD66b⁺ neutrophils were identified (**Figures 1A**). HLA-DR^{High}CD11b⁺ and CD16^{High} monocytes were also identified as well as neutrophils expressing CD11b, CD15, CD16 and CD64 (**Figure 1A****).**
UMAP dimensionality reduction was applied to the cell surface marker (HLA-DR, CD10, CD101, CD11b, CD16, CD64, CD117) expression datasets from control, SARS-CoV-2 mild and SARS-CoV-2 severe groups separately, marked differences were uncovered in COVID-19 patient monocyte and neutrophil populations (**Figure 1B****).** Severe patients exhibited significantly lower frequencies of B cells compared to controls (p<0.001), and of CD4⁺ (p<0.001) and CD8⁺
(p<0.01) T cells relative to both controls and mild patients; while CD56⁺ NK cell frequencies were comparable across all groups (**Figure 1C**). An increased fraction of monocytes was also observed in patients with mild COVID-19 compared to severe patients (**Figure 1D****),** without significant change in their absolute number (**Table 2**).
The change in monocyte abundance was dissected, the fraction of CD14^{High}CD16^{High} intermediate monocytes was significantly greater in mild COVID-19 patients (16.95+/- 6.75%) than in control (5.84+/-1.02%) or severe (6.77+/-1.10%) groups, while the non-classical CD14^{Low}CD16^{High} monocyte fraction was lower in severe COVID-19 patients (1.31+/-0.35%) than in mild (5.46+/-1.57%) or control groups (6.68+/-1.14%) (**Figures 1E****,** **1G**). Within the CD14^{High}CD16^{Low} classical monocyte subset (**Figures 2B**), higher frequencies of CD11b^{High} monocytes was detected with increased disease severity, while the intensity of HLA-DR expression was lower across both CD11b⁺ and CD11b⁻ monocyte populations of severe COVID-19 patients (**Figures 1F, 1H****,** **2C**). Finally, an expansion in the proportion of circulating neutrophils within the peripheral blood cell population in severe disease has been shown (**Figure 1I**), which was associated with an increase in their absolute number as published (Huang et al., 2020). Focusing on neutrophil subsets, a slight increase was noticed in the fraction of CD101⁺CD10^{Low} neutrophils in mild COVID-19 patients, whereas the fraction of CD101⁻CD10⁻ neutrophils was remarkably amplified in severe patients, suggesting an accumulation of immature subsets of neutrophils (Ng et al., 2019) in the peripheral blood of these patients (**Figures 1J**, **1K**, **2D-F**).

Altogether, these data identify significant SARS-CoV-2-induced changes in the relative abundance of monocyte and neutrophil subsets within the peripheral blood cell population, with loss of non-classical CD14^{Low}CD16^{High} monocytes, reduced expression of HLA-DR on classical monocytes and drop in CD101 and CD10 expression on neutrophils, characterizing severe cases.

**Table 2: Characteristics of patients of the learning cohort analyzed by spectral cytometry (results shown on Figure 1 and Figure 2)**

| | **All patients** | **SARS-CoV-2 negative Controls** | **SARS-CoV-2 positive Mild / Moderate** | **SARS-CoV-2 positive Severe** |
|---|---|---|---|---|
| Number | 25 | 12 (48) | 5 (20) | 8 (32) |
| Age (median, IQR) | 54 (31-73) | 48 (30-63) | 35 (28-56) | 73 (45-74) |
| Age > 60 | 8 (32) | 3 (25) | 0 (0) | 5 (38) |
| Male, n (%) | 13 (52) | 7 (58) | 0(0) | 6 (25) |

| **Comorbidities, n (%)** | | | | |
|---|---|---|---|---|
| Overweight | 12 (48) | 3 (25) | 1 (20) | 8 (100) |
| Cardiovascular | 7 (28) | 3 (25) | 0 (0) | 4 (50) |
| Diabetes | 5 (20) | 1 (8) | 0 (0) | 4 (50) |
| Respiratory | 6 (24) | 3 (25) | 1 (20) | 2 (25) |
| Cancer | 1 (4) | 0 (0) | 0 (0) | 1 (13) |

| **Initial symptoms, n (%)** | | | | |
|---|---|---|---|---|
| Fever | 9 (36) | 1 (8) | 0 (0) | 8 (100) |
| Cough | 13 (52) | 5 (42) | 3 (60) | 5 (63) |
| Dyspnea | 6 (24) | 0 (0) | 0 (0) | 6 (75) |
| Myalgia | 13 (52) | 6 (50) | 3 (60) | 4 (50) |
| Diarrhea | 4 (16) | 0 (0) | 4 (80) | 0 (0) |
| Anosmia / Ageusia | 3 (12) | 0 (0) | 2 (40) | 1 (13) |

| **Laboratory findings, median (IQR)** | | | | |
|---|---|---|---|---|
| Leukocytes (G/L) | 6.4 (5.0-10.0) | 5.8 (4.8-6.6) | 6.0 (4.4-7.1) | 13.3 (9.6-18.1) |
| Neutrophils (G/L) | 3.7 (2.9-6.8) | 3.3 (2.8-3.5) | 2.9 (2.8-4.0) | 10.9 (7.2-14.0) |
| Lymphocytes (G/L) | 1.8 (1.6-2.3) | 1.8 (1.6-2.5) | 2.3 (1.7-2.3) | 1.8 (0.9-2.0) |
| Monocytes (G/L) | 0.5 (0.4-0.6) | 0.4 (0.3-0.5) | 0.6 (0.5-0.7) | 0.6 (0.5-1.3) |
| Hemoglobin (g/dL) | 12.9 (11.7-14.4) | 14.5 (13.0-15.5) | 13.1 (12.8-13.1) | 11.1 (8.9-12.0) |
| Platelet count (G/L) | 248 (204-304) | 235 (202-261) | 206 (204-246) | 393 (268-522) |
| Fibrinogen (g/L) | 3.0 (2.6-6.3) | 2.5 (2.3-2.9) | 2.8 (2.8-3.4) | 7.8 (6.3-8.8) |
| D-dimers (ng/mL) | 339 (184-568) | 184 (85-204) | 339 (249-568) | 3738 (836-6489) |
| CRP (mg/L) | | | | 116.0 (102.5-241.7) |
| IL-6 (ng/L) | | | | 189.0 (59.2-677.0) |
| Ferritin (mg/L) | | | | 1133 (972-1633) |
| LDH (UI/L) | | | | 418 (352-446) |

| **Chest CT findings, n (%)** | | | | |
|---|---|---|---|---|
| <10% | 0 (0) | | 0 (0) | 0 (0) |
| 10-25% | 2 (8) | | 0 (0) | 2 (25) |
| 25-50% | 3 (12) | | 0 (0) | 3 (38) |
| >50% | 2 (8) | | 0 (0) | 2 (25) |
| <10% | 0 (0) | | 0 (0) | 0 (0) |
| NA | 18 (72) | 12 (100) | 5 (100) | 1 (13) |

| **Anti-inflammatory drugs, n** | | | | |
|---|---|---|---|---|
| Dexamethasone | 0 | | 0 | 0 |
| anti-IL 1 (anakinra) | 2 | | 1 | 1 |
| anti-IL6 (sari, toci) | 2 | | 1 | 1 |

| **Severity criteria** | | | | |
|---|---|---|---|---|
| PE, n (%) | 3 (12) | 0 | 0 | 3 (38) |
| MV, n (%) | | | | 8 (100) |
| PaO2/FiO2, med (range) | | | | 138 (95-177) |
| SOFA, med (range) | | | | 8 (3-14) |
| SOFA ≥ 8, n (%) | | | | 4 (50) |
| Death, n (%) | | | | 4 (50) |

| **Sampling, n (%)** | | | | |
|---|---|---|---|---|
| Delay since 1^{st} day hospital ≤ 10 days | 24 (96) | 12 (100) | 5 (100) | 7 (88) |

| | | | | |
|---|---|---|---|---|
| IQR, interquartile range; LDH, Lactate deshydrogenase; NA, not available; CT: computed tomography scan; PE, pulmonary embolism; SOFA, Sequential Organ Failure Assessment score; Sari, sarilumab ; toci, tocilizumab; MV, mechanical ventilation; PaO2/FiO2 (mmHg) at sampling; med, median (range) | | | | |

**Table 3 Panel of antibodies used for spectral cytometry analyses (CYTEK, Panel 1, results shown on Figure 1 to 8; CYTEK, panel 2, results shown on Figure 11 and Figure 12; CYTOF, results shown on Figure 5 to 7)**

| **Markers** | **Fluorochromes Metals** | **Localisation** | **Company** | **Concentration** | **Catalogue number** | **Panel** |
|---|---|---|---|---|---|---|
| CD195/CCR5 | PerCp/Cy5.5 | Extracellular | BD | 1 µL for 100 uL | 560635 | Cytek - Panel 1 |
| CD10 | PE-Cy5 | Extracellular | eBiosciences | 1 µL for 100 µL | 15-0106-42 | Cytek - Panel 1/2 |
| CD101 | AF647 | Extracellular | BioLegend | 1 µL for 100 µL | 331010 | Cytek - Panel 1/2 |
| CD117 (ckit) | BV480 | Extracellular | BD | 1 µL for 100 µL | 746848 | Cytek - Panel 1/2 |
| CD11b | BV786 | Extracellular | BD | 1 µL for 100 µL | 740965 | Cytek - Panel 1/2 |
| CD11c | BV711 | Extracellular | BD | 1 µL for 100 µL | 563130 | Cytek - Panel 2 |
| CD14 | BV421 | Extracellular | BioLegend | 1 µL for 100 µL | 301830 | Cytek - Panel 1/2 |
| CD141 | BV650 | Extracellular | BioLegend | 1 µL for 100 µL | 740604 | Cytek - Panel 2 |
| CD15 | Pacific Blue | Extracellular | BioLegend | 1 µL for 100 µL | 323022 | Cytek - Panel 1/2 |
| CD16 | BUV496 | Extracellular | BD | 1 µL for 100 µL | 564653 | Cytek - Panel 1/2 |
| CD163 | PE-CF593 | Extracellular | BD | 1 µL for 100 µL | 562670 | Cytek - Panel 1 |
| CD169 | PE | Extracellular | BioLegend | 1 µL for 100 µL | 346004 | Cytek - Panel 2 |
| CD19 | BUV563 | Extracellular | BD | 1 µL for 100 µL | 612916 | Cytek - Panel 1/2 |
| CD3 | BB700 | Extracellular | BD | 1 µL for 100 µL | 566575 | Cytek - Panel 1/2 |
| CD36 | APC | Extracellular | BioLegend | 1 µL for 100 µL | 336208 | Cytek - Panel 2 |
| CD37 | FITC | Extracellular | BioLegend | 1 µL for 100 µL | 356304 | Cytek - Panel 2 |
| CD38 | BV605 | Extracellular | BD | 1 µL for 100 µL | 562665 | Cytek - Panel 1/2 |
| CD4 | BUV805 | Extracellular | BD | 1 µL for 100 µL | 612887 | Cytek - Panel 1/2 |
| CD40 | APC-R700 | Extracellular | BD | 1 µL for 100 µL | 565179 | Cytek - Panel 1 |
| CD45 | BUV395 | Extracellular | BD | 1 µL for 100 µL | 563792 | Cytek - Panel 1/2 |
| CD56 | BB700 | Extracellular | BD | 1 µL for 100 µL | 566400 | Cytek - Panel 1/2 |
| CD63 | Pe-Cy7 | Extracellular | BioLegend | 1 µL for 100 µL | 353010 | Cytek - Panel 2 |
| CD64 | AF700 | Extracellular | R&D | 1 µL for 100 µL | FAB12571N-100UG | Cytek - Panel 2 |
| CD66b | FITC | Extracellular | Beckman Coulter | 1 µL for 100 µL | IM0531U | Cytek - Panel 1 |
| CD8 | BV510 | Extracellular | BD | 1 µL for 100 µL | 563919 | Cytek - Panel 1/2 |
| CD95 | BV650 | Extracellular | BD | 1 µL for 100 µL | 740589 | Cytek - Panel 1 |
| CD181/CXCR1 | PE | Extracellular | BioLegend | 1 µL for 100 µL | 320608 | Cytek - Panel 1 |
| CD184/CXCR4 | PE-Dazzle 594 | Extracellular | BioLegend | 1 µL for 100 µL | 306526 | Cytek - Panel 2 |
| FcεRI | BUV737 | Extracellular | BD | 1 µL for 100 µL | 749338 | Cytek - Panel 1/2 |
| CD130/GP130 | APC | Extracellular | BioLegend | 1 µL for 100 µL | 362005 | Cytek - Panel 1 |
| HLA-DR | APC-H7 | Extracellular | BD | 1 µL for 100 µL | 561358 | Cytek - Panel 1/2 |
| CD126/IL-6Rc | PECy7 | Extracellular | BioLegend | 1 µL for 100 µL | 352810 | Cytek - Panel 1 |
| CD284/TLR4 | BV711 | Extracellular | BD | 1 µL for 100 µL | 564404 | Cytek - Panel 1 |
| CD41 | 89Y | Extracellular | Fluidigm | 1 µL for 100 µL | 3089004B | CyTOF |
| CD45 | 141Pr | Extracellular | Fluidigm | 1 µL for 100 µL | 3141009B | CyTOF |
| CD19 | 142Nd | Extracellular | Fluidigm | 1 µL for 100 µL | 3142001B | CyTOF |
| CD45RA | 143Nd | Extracellular | Fluidigm | 1 µL for 100 µL | 3143006C | CyTOF |
| CD42 | 144Nd | Extracellular | Fluidigm | 1 µL for 100 µL | 3144020B | CyTOF |
| CD4 | 145Nd | Extracellular | Fluidigm | 1 µL for 100 µL | 3145001B | CyTOF |
| CD8 | 146Nd | Extracellular | Fluidigm | 1 µL for 100 µL | 3146001B | CyTOF |
| CD303 | 147Sm | Extracellular | Fluidigm | 1 µL for 100 µL | 3147009B | CyTOF |
| CD16 | 148Nd | Extracellular | Fluidigm | 1 µL for 100 µL | 3148004B | CyTOF |
| CD34 | 149Sm | Extracellular | Fluidigm | 1 µL for 100 µL | 3149013B | CyTOF |
| CD123/IL-3R | 151Eu | Extracellular | Fluidigm | 1 µL for 100 µL | 3151001B | CyTOF |
| CD66b | 152Sm | Extracellular | Fluidigm | 1 µL for 100 µL | 3152011B | CyTOF |
| CD192/CCR2 | 153Eu | Extracellular | Fluidigm | 1 µL for 100 µL | 3153023B | CyTOF |
| CD3 | 154Sm | Extracellular | Fluidigm | 1 µL for 100 µL | 3154003B | CyTOF |
| CD36 | 155Gd | Extracellular | Fluidigm | 1 µL for 100 µL | 3155012B | CyTOF |
| CD10 | 156Gd | Extracellular | Fluidigm | 1 µL for 100 µL | 3156001B | CyTOF |
| CD101 | 158Gd | Extracellular | Fluidigm | 1 µL for 100 µL | 3158020C | CyTOF |
| CD11c | 159Tb | Extracellular | Fluidigm | 1 µL for 100 µL | 3159001B | CyTOF |
| CD14 | 160Gd | Extracellular | Fluidigm | 1 µL for 100 µL | 3160006B | CyTOF |
| CD90 | 161Dy | Extracellular | Fluidigm | 1 µL for 100 µL | 3161009B | CyTOF |
| CD33 | 163Dy | Extracellular | Fluidigm | 1 µL for 100 µL | 3163023B | CyTOF |
| CD95/Fas | 164Dy | Extracellular | Fluidigm | 1 µL for 100 µL | 3164008B | CyTOF |
| CD163 | 165Ho | Extracellular | Fluidigm | 1 µL for 100 µL | 3165017B | CyTOF |
| Pp65/PNFkB | 166Er | Intracellular | Fluidigm | 1 µL for 100 µL | 3166006A | CyTOF |
| CD38 | 167Er | Extracellular | Fluidigm | 1 µL for 100 µL | 3167001B | CyTOF |
| CD71 | 168Er | Extracellular | Fluidigm | 1 µL for 100 µL | 3168014B | CyTOF |
| CD40 | 170Er* | Extracellular | BioLegend | 1 µL for 100 µL | 334302 | CyTOF |
| CX3CR1 | 172Yb | Extracellular | Fluidigm | 1 µL for 100 µL | 3172017B | CyTOF |
| Granzyme B | 173Yb | Intracellular | Fluidigm | 1 µL for 100 µL | 3173006C | CyTOF |
| HLA-DR | 174Yb | Extracellular | Fluidigm | 1 µL for 100 µL | 3174001B | CyTOF |
| CD56 | 176Yb | Extracellular | Fluidigm | 1 µL for 100 µL | 3176008B | CyTOF |
| CD11b | 209Bi | Extracellular | Fluidigm | 1 µL for 100 µL | 3209003B | CyTOF |

| | | | | | | |
|---|---|---|---|---|---|---|
| *Maxpar Antibody Labeling Kit - Fluidigm | | | | | | |

### 2.2. Serial single cell analysis of mild versus severe patient blood cells identifies dynamic changes in monocyte subsets

To gain insights into the factors affecting innate immune cell phenotype in COVID-19, patient blood cells were subjected to single cell RNA sequencing (scRNAseq) using the 10X Genomics Chromium platform. Peripheral blood samples were collected from three control patients with flu-like symptoms but testing negative for SARS-CoV-2 by RT-PCR of oropharyngeal swabs, and three SARS2-CoV-2 positive infected patients: one outpatient with mild disease and two patients with severe disease admitted to ICU (**Table 4**). All samples were sequenced immediately after collection and red blood cell lysis, without additional sorting or freezing in order to preserve fragile cell populations, mainly neutrophils and plasma cells. Unsupervised clustering based on gene expression identified B and T cells as well as neutrophils, monocytes, erythroid cells and platelets (**Figure 3A**). UMAP analysis suggested a smaller fraction of lymphocytes and a higher proportion of neutrophils in the two severe patients when compared to controls and the mild patient (**Figure 3A**, **4A**). Samples analyzed by scRNAseq were simultaneously analyzed by spectral flow cytometry for comparison. Again, UMAP analysis showed lower proportions of CD4 and CD8 T cell fractions while the neutrophil fraction was greater in severe patients compared to controls and to the mild patient (**Figure 3B**, **4B**). The three SARS2-CoV-2-infected patients were sampled again 10 days later to monitor progression of the immune response in relation to clinical status (**Figures 3A**, **3B**).

UMAP visualization of monocytes at both sampling timepoints analyzed by scRNAseq identified three clusters (**Figure 5A**), defined by the expression of FCGR3A (CD16 or type III Fcγ receptor in cluster 3), ITGAM (CD11b, in clusters 1 and 2) and HLA-DRA and HLA-DRB1 (cluster 1) genes (**Figure 6A**). Alarmin S100A8 and S100A9 genes were expressed in cells from the three clusters. The proportion of the three monocyte clusters differed between mild and severe patients (**Figure 5A** **and** **6B**): for example, compared to other patients, the two patients with severe disease showed a higher proportion of monocytes expressing ITGAM gene, while the expression of HLA-DRA and HLA-DRB1 was lower (**Figure 6B**). Differential expressed genes (DEG) and pathway analyses delineated a distinct type I interferon signature in the mild COVID-19 patient monocytes, with expression of a signature of interferon-stimulated genes (ISGs) (IFI44L, IFI44, MX1, ISG15, SIGLEC1) (**Table 5**). This signature was less pronounced in the two severe COVID-19 samples and controls, contrasting with the elevated expression of genes involved in the production of reactive oxygen species (ROS) and nitric oxygen species (NOS) (**Table 5**), suggestive of a chronic inflammation induced immunosuppression (Gabrilovich and Nagaraj, 2009). Accordingly, genes involved in iNOS (*IFNGR2, RELA, TRAF6, MYD88)* and eNOS (*AQP9, CALM1, PIK3CA, PIK3CG, PRKAG1, PRKAB2, HSPA6, AKT1*) pathways were highly expressed in these samples. We also performed a gene ontology network analysis that confirmed high expression of type I interferon signaling pathway and related networks in the monocytes of the mild patient, while the regulation of the ROS metabolic process network and NF-κB signaling were highly expressed in the two severe patients (**Figure 6C**).

To validate these findings, the data collected by spectral flow cytometry of the same samples were analyzed. CD14^{High}CD16^{Low} classical monocytes were distinguished from CD14^{Low}CD16^{High} non-classical monocytes using a non-supervised UMAP analysis. This highlighted clear variation in monocyte subset repartition among patients: compared to controls and the mild patient, severe patient #1 showed a lower fraction of CD14^{Low}CD16^{High} non-classical monocytes at day 0, as observed in other severe patients (**Figure 1**), while the other severe patient showed a high level of this monocyte fraction (**Figures 5B**). Regarding classical CD14^{High}CD16^{Low} monocytes, the two severe patients showed markedly higher levels of CD141 (THBD) at their surface (**Figure 5C**), in accordance with scRNAseq analysis.

In the mild patient, one of the most highly expressed genes in classical monocytes was the ISG SIGLEC-1 (CD169), which was further validated by the strong expression of CD169, the corresponding protein, at the surface of classical monocytes at day 0 (**Figure 5D**). Ten days later in the same patient, *SIGLEC-1* gene expression was down-regulated and CD169 expression was undetectable at the surface of HLA-DR^{High} classical monocytes (**Figure 5D**). Additionally, SIGLEC-1 gene was not overexpressed, and CD169 expression at the surface of HLA-DR^{High} classical monocytes remained low in the two severe patients (**Figure 5D**). These results suggest a transient IFNα response in mild patients lasting fewer than 10 days. Finally, the two severe patients exhibited low expression of HLA-DR protein on their monocyte surface at day 0, without significant change at day 10 (**Figure 5D**).

Mass spectrometry analysis of an independent cohort of 12 patients (four controls, four mild and four severe COVID patients)(**Table 6**) showed similar results, with a lower fraction of CD14^{Low}CD16^{High} non-classical monocytes in severe patients compared to mild patients, and a higher fraction in mild patients compared to both severe patients and controls (**Figure 5E and 5F**). As pathway analysis of our scRNAseq highlighted NF-κB activation (e.g. *NFKBIA, NFKBIZ* and *SOD1* gene up-regulation) as a prominent feature in monocytes of severe patients, mass cytometry was used to explore the expression of the phosphorylated transcription factor RelA/p65 (P-p65), a critical effector of the canonical NF-κB pathway, in HLA-DR^{Low} monocytes of severe COVID-19 patients. Significantly higher levels of P-p65 was observed in HLA-DR^{Low}CD14⁺ classical monocytes from severe patients compared to controls (**Figure 5G and 5H**). P-p65 expression was also measured in circulating CD34⁺ cells, identifying an increased expression in severe disease (**Figure 6C**).

**Table 4: Characteristics of patients analyzed by single cell RNA sequencing (results shown on Figure 3 to 8). M, male; F, female; NA, not available; PE, pulmonary embolism.**

| | **SARS-CoV2 pos Severe # 1** | | **SARS-CoV2 pos Severe # 2** | | **SARS-CoV2 pos Mild** | | **Control # 1** | **Control # 2** | **Control # 3** |
|---|---|---|---|---|---|---|---|---|---|
| Age | 69 | | 26 | | 21 | | 54 | 27 | 58 |
| Gender | M | | F | | F | | F | F | F |
| SARS-CoV2 RT-PCR | positive | | positive | | positive | | negative | negative | negative |

| **Comorbidities** | | | | | | | | | |
|---|---|---|---|---|---|---|---|---|---|
| Overweight | Yes | | Yes | | No | | Yes | No | Yes |
| Cardiovascular | No | | No | | No | | Yes | No | No |
| Diabetes | Yes | | Yes | | No | | No | No | Yes |
| Respiratory | No | | No | | No | | No | No | No |
| Cancer | No | | No | | No | | No | No | No |

| **Initial symptoms** | | | | | | | | | |
|---|---|---|---|---|---|---|---|---|---|
| Fever | Yes | | No | | No | | No | No | No |
| Cough | Yes | | No | | Yes | | Yes | Yes | No |
| Dyspnea | Yes | | Yes | | No | | No | No | No |
| Myalgia | No | | Yes | | Yes | | No | Yes | No |
| Diarrhea | Yes | | Yes | | No | | No | No | No |
| Anosmia / Ageusia | No | | No | | Yes | | No | Yes | No |

| **Sampling** | | | | | | | | | |
|---|---|---|---|---|---|---|---|---|---|
| | Sample 1 (d0) | Sample 2 (d10) | Sample 1 (d0) | Sample 2 (d10) | Sample 1 (d0) | Sample 2 (d10) | Sample 1 | Sample 1 | Sample 1 |
| Delay from 1st day at hospital (days) | 14 | 24 | 10 | 20 | 0 | 0 | 0 | 0 | 0 |

| **Laboratory findings and chest CT** | | | | | | | | | |
|---|---|---|---|---|---|---|---|---|---|
| Leukocytes (G/L) | 10.2 | 10.9 | 11.4 | 7.4 | 4.9 | 6.6 | 5.7 | 6.7 | 7.1 |
| Neutrophils (G/L) | 6.9 | 8.3 | 7.7 | 4.9 | 2.2 | 3.4 | 3.4 | 4.0 | 4.2 |
| Lymphocytes (G/L) | 1.8 | NA | 2.5 | NA | 2.3 | NA | 1.7 | 1.9 | 2.2 |
| Monocytes (G/L) | 0.7 | NA | 0.68 | NA | 0.27 | NA | 0.48 | 0.48 | 0.43 |
| Hemoglobin (g/dL) | 9.8 | 10.5 | 6.7 | 6.8 | 10.4 | 10.6 | 12.6 | 12.4 | 12.8 |
| Platelets (G/L) | 186 | 136 | 282 | 287 | 281 | 348 | 302 | 236 | 230 |
| CRP (mg/L) | 16.7 | | 193 | | 1.1 | | < 1.5 | < 1.0 | 4.4 |
| IL-6 (ng/L) | 11.1 | | 67.3 | | < 1.5 | | 3.5 | < 1.5 | < 1.5 |
| CT damage (%) | 50 | | 25-50 | | | | | | |

| **Severity criteria** | | | | | | | | | |
|---|---|---|---|---|---|---|---|---|---|
| PE | No | | Yes | | No | | No | No | No |
| Maximal 02 (L/min) | MV | 1 | MV | MV | 0 | 0 | 0 | 0 | 0 |
| PaO2/FiO2 (mmHg) | 124 | 273 | 225 | 233 | NA | NA | NA | NA | NA |
| SOFA at sampling | 7 | 6 | 6 | 3 | NA | NA | NA | NA | NA |
| Death, n (%) | No | | No | | No | | No | No | No |

| | | | | | | | | | |
|---|---|---|---|---|---|---|---|---|---|
| Note : Samples were collected from four patients examined at Hotel-Dieu COVID-19 screening unit for cough without or with fever. Only one of these patients was SARS-CoV2 RT-PCR-positive on nasopharyngeal swab. Given her mild symptoms, this 21-year old nurse was not hospitalized. She recovered at home and was seen 10 days later for a second blood screen. | | | | | | | | | |

Patient "Severe #1" is a 69-year old man with type 2 diabetes mellitus (body mass index, 26.2) who was hospitalized on April 4, 2020 because of fever, dyspnea, diarrhea and poor general condition. SARS-CoV2 RT-PCR on buccal swab was positive. He was rapidly transferred to Cochin hospital ICU and mechanically ventilated (MV), starting on April 5 (SOFA: 12, PaO2/FiO2: 182 at admission). The sample used for scRNAseq was collected on April 20, 2020 (SOFA: 7; PaO2/FiO2: 124). He progressively recovered, was released from mechanical ventilation on April 29 and transferred to a medical unit on April 30 (PaO2/FiO2: 273), when the sample used for the second scRNAseq analysis was collected. On May 5, the patient was recovering with mild oxygenotherapy (1L/min) in Cochin hospital pneumology care unit.

Patient "Severe #2" is a 26-year old woman with insulin-dependent type 1 diabetes mellitus and obesity (body mass index, 30.6) who was hospitalized on April 3, 2020 because of fever, dyspnea and diarrhea. On April 8, SARS-CoV2 RT-PCR on pharyngeal swab was positive. On April 10, she was hospitalized in Cochin hospital emergency unit with acido-cetosis after she had interrupted her daily insulin therapy. She required oxygenotherapy and was transferred to ICU on April 12 (SOFA score 3). The sample used for scRNAseq was collected on April 20, 2020 (SOFA: 6; PaO2/FiO2: 225). She developed a bacterial infection (P. aeruginosa, S. aureus in bronchoalveolar aspirate) on April 20, partially recovered and was transferred in Cochin hospital pneumology care unit on April 25, then returned to ICU on April 28 with degraded pulmonary condition due to K. pneumonia pneumopathy and pulmonary embolism. The sample used for the second scRNAseq analysis was collected on April 30th (SOFA 3; PaO2/FiO2: 232.8). On May 4, she was still in ICU with progressive recovery of severity parameters (SOFA:1; PaO2/FiO2: 312).

**Table 5: List of genes involved in pathways identified in patient granulocytes**

| **Monocyte HLADR-** | **Patient Mild** | **Patient Severe** | **Patient Critical** |
|---|---|---|---|
| **Interferon Signaling** | IFI6, IFITM1, IFITM2, IFITM3, ISG15 | | |
| **IL-8 Signaling** | AKT1, GNB2, GNG5, IQGAP1, NRAS, PLD2, SRC | GNB2, HBEGF, HMOX1, ITGB2, MAPK9, PIK3CA, PIK3CG, PLD3, RAC1, RAP2B, RELA, RHOG, TRAF6 | CSTB, CXCL8, GNG5, HBEGF, MYL12B, NRAS, RAC1, RHOC, RHOG |
| **CXCR4 Signaling** | AKT1, EGR1, GNB2, GNG5, NRAS, SRC | | GNG5, MYL12A, MYL12B, MYL6, NRAS, RAC1, RHOC, RHOG |
| **Production of Nitic Oxide and Reactive Oxygen Species in Macrophages** | | IFNGR2, LYZ, MAP3K8, MAPK9, PIK3CA, PIK3CG, PPARA, RAC1, RELA, RHOG, SPI1, TNFRSF1A | CLU, CYBA, IFNGR2, LYZ, MAP3K8, RAC1, RHOC, RHOG, S100A8 |
| **IL-12 Signaling and Production in Macrophages** | | CEBPB, LYZ, MAP3K8, MAPK9, PIK3CA, PIK3CG, RELA, SPI1, TRAF6 | |
| **IL-6 Signaling** | | CD14, CEBPB, MAPK9, PIK3CA, PIK3CG, RAP2B, RELA, TNFRSF1A, TRAF6 | CD14, CEBPB, CXCL8, NRAS, STAT3 |

**Table 6: Characteristics of patients whose cells were analyzed by mass spectrometry (results shown on Figure 5 to 8)**

| | **SARS-CoV-2 negative Controls** | **SARS-CoV-2 positive Mild / Moderate** | **SARS-CoV-2 positive Severe** |
|---|---|---|---|
| Number | 4 (33) | 4 (33) | 4 (33) |
| Age (median IQR) | 58 (46-62) | 42 (27-58) | 65 (61-69) |
| Age>60 | 2 (50) | 0 (0) | 3 (75) |
| Male, n(%) | 2 (50) | 1 (25) | 3 (75) |

| **Comorbidities, n (%)** | | | |
|---|---|---|---|
| Overweight | 1 (25) | 0 (0) | 4 (100) |
| Cardiovascular | 0 (0) | 0 (0) | 1 (25) |
| Diabetes | 0 (0) | 0 (0) | 0 (0) |
| Respiratory | 1 (25) | 1 (25) | 0(0) |
| Cancer | 0 (0) | 0 (0) | 0 (0) |

| **Initial symptoms, n (%)** | | | |
|---|---|---|---|
| Fever | 0 (0) | 1 (25) | 3 (75) |
| Cough | 2 (50) | 4 (100) | 3 (75) |
| Dyspnea | 0 (0) | 1 (25) | 4 (100) |
| Myalgia | 2 (50) | 2 (50) | 0 (0) |
| Diarrhea | 2 (50) | 3 (75) | 0 (0) |
| Anosmia / Ageusia | 2 (50) | 1 (25) | 0 (0) |

| **Laboratory findings, median (IQR)** | | | |
|---|---|---|---|
| Leukocytes (G/L) | 6.7 (5.9-8.0) | 6.4 (4.0-8.9) | 8.9 (6.4-12.1) |
| Neutrophils (G/L) | 4.0 (3.0-6.6) | 3.7 (2.5-5.3) | 6.9 (5.3-8.8) |
| Lymphocytes (G/L) | 1.8 (1.7-2.0) | 1.7 (0.9-2.5) | 1.2 (0.7-1.7) |
| Monocytes (G/L) | 0.5 (0.4-0.7) | 0.5 (0.5-0.6) | 0.7 (0.5-1.0) |
| Hemoglobin (g/dL) | 14.2 (12.7-15.5) | 13.8 (11.8-16.3) | 10.4 (9.3-11.2) |
| Platelet count (G/L) | 217.5 (190.0-267.8) | 186.5 (170.0-197.0) | 323 (210-442) |
| Fibrinogen (g/L) | 3.0 (2.6-3.7) | 3.0 (2.7-3.5) | 7.2 (6.2-7.6) |
| D-dimers (ng/mL) | 313 (210-410) | 244 (219-254) | 2300 (2042-6747) |
| CRP (mg/L) | | | 177.2 (146.4-306.6) |
| IL-6 (ng/L) | | | 113.2 (58.4-186.1) |
| Ferritin (mg/L) | | | 1888 (1423-2343) |
| LDH (UI/L) | | | 405 (355-412) |

| **Chest CT findings, n (%)** | | | |
|---|---|---|---|
| <10% | | | 0 (0) |
| 10-25% | | | 4 (100) |
| 25-50% | | | 0 (0) |
| >50% | | | 0 (0) |
| NA | | | 0 (0) |

| **Anti-inflammatory drugs, n** | | | |
|---|---|---|---|
| Dexamethasone | | 0 | 0 |
| anti-IL1 (anakinra) | | 0 | 0 |
| anti-IL6 (sari, toci) | | 0 | 0 |

| **Severity criteria** | | | |
|---|---|---|---|
| PE, n (%) | 0 (0) | 0 (0) | 2 (50) |
| MV, n (%) | | | 4 (100) |
| PaO2/FiO2, med (range) | | | 157 (95-232) |
| SOFA, med (range) | | | 3 (2-8) |
| SOFA ≥ 8, n (%) | | | 1 (25) |
| Death, n (%) | 0 (0) | 0 (0) | 1 (25) |

| **Sampling, n (%)** | | | |
|---|---|---|---|
| Delay from 1^{st} day in hospital ≤ 10 days | 4 (100) | 4 (100) | 4 (100) |

| | | | |
|---|---|---|---|
| IQR, interquartile range; LDH, Lactate deshydrogenase; NA, not available; CT: computed tomography scan; sari, sarilumab ; toci, tocilizumab; PE, pulmonary embolism; SOFA, Sequential Organ Failure Assessment score; Sari, sarilumab ; toci, tocilizumab; MV, mechanical ventilation; PaO2/FiO2 (mmHg) at sampling; med, median (range); | | | |

### 2.3. Serial single cell analysis of mild versus severe patient blood cells identifies also changes in neutrophil subsets

Having uncovered significant changes in the fraction of neutrophils in blood of COVID-19 patients, neutrophil subset partition during the disease was investigated. UMAP of neutrophil populations identified four clusters (**Figure 7A**), defined by the expression of S100A8 and S100A9 alarmin genes (cluster 4), CXCR4 (cluster 3 and 4) and IL1R2 (cluster 1) (**Figure 8A**). The relative proportions of each neutrophil cluster differed between mild and severe patients (**Figure 7A** **and** **8A**). Severe disease was associated with a greater proportion of neutrophils from cluster 3 and 4 and higher expression levels of S100A8, S100A9, FCER1G (encoding the high affinity IgE receptor), CD63 and CD37, two tetraspanin-encoding genes, and the CXCR4 gene (encoding the trans-membrane chemokine receptor C-X-C Motif Chemokine Receptor 4 binding CXCL12), compared to the mild patient and controls (**Figure 8B**).

Analysis of neutrophil DEGs at day 0 in the mild patient suggested a typical type I interferon response (IFI44L, IFI44, IFIT3, IFIT1, MX1, ISG15 and TNFAIP3) (**Table 7**), in accordance with our observations in monocytes, that was lost by day 10 in the neutrophils of the mild patient. This signature was absent in the two severe patient samples and controls, demonstrating high expression of genes involved in the production of ROS (TSPO, SOD2, ITGB2, ICAM1, RAC1, RAB27A, ZC3H12A, GNAS) and the inducible NOS pathway (IRAK3, IFNGR2, MAPK14) (**Table 7**). In severe patient #2, neutrophils also exhibited high expression of IL-1 signaling pathway genes (GNAI3, GNAS, GNG2, GNG5 and IRAK3) (**Table 7**). These expression signatures observed at day 0 in the two severe patients were decreased at day 10. Finally, gene ontology network analysis further confirmed the activation of a type I interferon pathway in the neutrophils of the mild patient, while severe patients showed high levels of expression of genes involved in oxidative metabolism, IL-1 signaling and NF-κB activation pathways.

The data collected by spectral flow cytometry analysis of the same samples was analyzed to validate these findings (**Figure 7B**). As observed in **Figure 1**, CD10⁺CD101⁺ mature neutrophils were distinguished from CD10^{Low}CD101⁻ immature neutrophils using a non-supervised UMAP analysis (**Figure 7C**). Severe patients at day 0 had more circulating CD10^{Low}CD101⁻ immature form of neutrophils compared to controls or the mild patient (grey gate on UMAP **Figure 7B**) at day 0. Severe patient #1 neutrophils had increased the expression of CD101 on their surface at day 10 while severe patient #2 neutrophils retained their immature phenotype at day 10. Focusing on the expression of a pre-neutrophil hallmark CXCR4 at the surface of CD10^{Low}CD101⁻ immature neutrophils (Ng et al, 2019), an increase in the proportion of neutrophils with a CD10LowCD101-CXCR4+ phenotype was observed, described as pre-neutrophils (**Figure 7C**).

Mass spectrometry analysis of an independent cohort of 12 patients (four controls, four mild and four severe COVID patients) again suggested a higher fraction of CD10LowCD101-immature neutrophils in severe patients compared to control patients (**Figure 7D and 7E**).

Altogether, these results suggested that mild disease patients could develop a potentially protective transient type I interferon response, while instead severe patients exhibited phenotypically-immature subsets of monocytes, and neutrophils expressing S100A8 and S100A9 alarmin genes products, as well as immunosuppressive gene patterns involving reactive oxygen species.

**Table 7: List of genes involved in pathways identified in patient neutrophils**

| **Neutrophil CXCR4+** | **Patient Mild** | **Patient Severe** | **Patient Critical** |
|---|---|---|---|
| **Interferon Signaling** | IFIT1, IFIT3, IFITM1, IFITM3, ISG15, MX1, TAP1 | | |
| **CXCR4 Signaling** | | GNB2, GNG2, MAP2K3 | GNAI3, GNAS, GNG2, GNG5, LYN, MYL12A |
| **IL-1 Signaling** | | | GNAI3, GNAS, GNG2, GNG5, IRAK3 |
| **IL-8 Signaling** | | GNB2, GNG2, ITGB2 | GNAI3, GNAS, GNG2, GNG5, IRAK3 |
| **Thrombin Signaling** | | | GNAI3, GNAS, GNG2, GNG5, MYL12A |
| **Production of Nitic Oxide and Reactive Oxygen Species in Macrophages** | | | CYBA, LYZ, NCF4, S100A8 |

### 2.4. Interferon and calprotectin plasma levels distinguish mild from severe COVID-19 patients.

ScRNAseq analyses suggested that S100A8 and S100A9 alarmin encoding genes were significantly more highly expressed in several patients. S100A8 and S100A9 alarmins, representing -45% of the cytoplasmic proteins in neutrophils, are released under inflammatory conditions and form a stable heterodimer known as "calprotectin" with chemokine-like functions (reviewed in Wang S et al., 2018). RT-qPCR analysis of S100A9 and S100A9 gene expression levels confirmed significantly higher expression of these genes in peripheral blood nucleated cells (buffy coats) of patients with severe COVID-19 (n=8) compared to controls (n=8) and patients with a mild disease (n=16) (**Figure 10A** **and Table 8**). This led to measure the plasma level of calprotectin (S100A9/S100A9 heterodimer), together with type I interferon (IFNα2a) and 40 other cytokines and chemokines in samples from a cohort of 84 patients (**Table 9**). We compared the expression profile of these cytokines and chemokines between SARS-CoV-2-positive patients and controls (**Figure 9A and 9B**). As observed in **Figure 9A**, patients with mild disease showed significantly less CXCL8 (**Figures** **9C** **and** **10B**) and significantly more type I IFNα2a (**Figure 9A and 9C**) compared to controls; while calprotectin plasma levels were comparable between mild and control patients (**Figure 9C**). In contrast, severe patients exhibited a dramatic increase in calprotectin levels compared to mild COVID-19 or controls, without further increase in interferon plasma level above mild disease levels (**Figures 9B and 9C and 10C**). Calprotectin was the most significantly increased circulating biomarker in severe patients, accompanied by a rise in 23 out of the 40 other tested chemokines and cytokines, including CXCL-8, CXCL-12 and IL-6 **(****Figures 9B, 9C** **and** **10B**).

Age and comorbidities (including obesity, diabetes mellitus, cardiovascular and respiratory diseases and cancer) are predictor of severe COVID-19 disease (Richardson et al., 2020). We found that plasma calprotectin levels were significantly higher in control patients with comorbidities, as well as in mild or severe COVID-19 patients with comorbidities (**Figure 9D**). Nevertheless, the increase in calprotectin in severe COVID-19 far exceeds that correlations associated with comorbidities. None of the other measured circulating proteins were significantly increased in patients with comorbidities (**Figure 9E**). Bacterial infections that can occur in severe COVID-19 (Chen et al., 2020b; Llitjos et al., 2020) and were present in some of our severe patients did not significantly modify the profile of released proteins (**Figures 10B** **and** **10D**), including calprotectin (**Figure 9F**). Increasing age was associated with elevated calprotectin level in controls (**Figure 10D**). However, no correlation between calprotectin and age was observed in the severe group of patients (R = -0.26; p-value = 0.09). Calprotectin concentration correlated with neutrophil counts (**Figure 9G**), fibrinogen plasma levels (**Figure 9H**) and D dimers (**Figure 9I**), the latter being fibrin degradation products reflecting a hypercoagulability state. We also uncovered a weak correlation between IL-6 plasma concentration and levels of calprotectin (**Figure 10E**), blood neutrophils (**Figure 9J**), fibrinogen (**Figure 9K**) and D dimers (**Figure 9L**).

These results indicate that high plasma levels of calprotectin and low plasma levels of IFNα2a are seen in severe cases, while the opposite is true in mild disease, corroborating the evidence from peripheral blood immune phenotypes. Importantly, correlations are independent from confounding factors for prognosis such as advanced age, co-morbidities or concurrent bacterial infection which have only minor effects on plasma calprotectin and interferon levels.

**Table 8: Characteristic of patients whose buffy coat was analyzed by RT-qPCR (results shown on Figure 10)**

| | **All patients** | **SARS-CoV-2 negative Controls** | **SARS-CoV-2 positive Mild** / **Moderate** | **SARS-CoV-2 positive Severe** |
|---|---|---|---|---|
| Number | 32 | 8 (25) | 16 (50) | 8 (25) |
| Age (median, IQR) | 46 (31-61) | 30 (26-44) | 49 (36-61) | 64 (47-69) |
| Age > 60 | 9 (28) | 0(0) | 5 (31) | 4 (50) |
| Male, n (%) | 12 (38) | 2 (25) | 5 (31) | 5 (50) |

| **Comorbidities, n (%)** | | | | |
|---|---|---|---|---|
| Overweight | 18 (56) | 3 (38) | 8 (50) | 7 (88) |
| Cardiovascular | 9 (28) | 1 (18) | 5 (31) | 3 (38) |
| Diabetes | 7 (22) | 0(0) | 3 (19) | 4 (50) |
| Respiratory | 4 (13) | 2 (25) | 1 (6) | 1 (13) |
| Cancer | 0(0) | 0(0) | 0(0) | 0(0) |

| **Initial symptoms, n (%)** | | | | |
|---|---|---|---|---|
| Fever | 13 (41) | 0(0) | 6 (38) | 7 (88) |
| Cough | 21 (67) | 4 (50) | 12 (75) | 5 (63) |
| Dyspnea | 13 (41) | 1 (13) | 4 (25) | 8 (100) |
| Myalgia | 11 (34) | 3 (38) | 4 (25) | 4 (50) |
| Diarrhea | 12 (38) | 4 (50) | 6 (38) | 2 (25) |
| Anosmia / Ageusia | 9 (28) | 0(0) | 7 (44) | 2 (25) |

| **Laboratory findings, median (IQR)** | | | | |
|---|---|---|---|---|
| Leukocytes (G/L) | 6.6 (1.9-23.4) | 7.4 (6.2-9.8) | 5.8 (4.0-6.3) | 10.7 (9.4-12.7) |
| Neutrophils (G/L) | 4.6 (3.4-7.5) | 4.3 (3.6-5.1) | 3.4 (1.5-4.6) | 7.6 (7.2-10.1) |
| Lymphocytes (G/L) | 1.6 (0.5-3.9) | 2.8 (2.0-3.1) | 1.0 (0.8-1.3) | 1.5 (1.3-1.7) |
| Monocytes (G/L) | 0.5 (0.1-1.7) | 0.5 (0.4-0.6) | 0.3 (0.2-0.5) | 0.6 (0.5-0.9) |
| Hemoglobin (g/dL) | 12.6 (10.8-13.5) | 13.0 (13.6-14.4) | 12.6 (11.6-13.3) | 8.5 (7.3-9.8) |
| Platelet count (G/L) | 261.0 (86.0-526.0) | 285 (252-324) | 232 (173-394) | 224 (131-300) |
| Fibrinogen (g/L) | | | 726 (227-1326) | 3721 (2134-5544) |
| D-dimers (ng/mL) | | | 1.4 (1.4-36.7) | 109.6 (74.5-127.5) |
| CRP (mg/L) | | | 10.0 (0.9-104.0) | 169.0 (128.0-232.0) |
| IL-6 (ng/L) | | | 4.4 (3.1-6.2) | 7.5 (6.7-9.9) |
| Ferritin (mg/L) | | | 643 (504-856) | 1252 (1175-1455) |
| LDH (UI/L) | | | 240 (240-293) | 462 (370-681) |

| **Chest CT findings, n (%)** | | | | |
|---|---|---|---|---|
| <10% | | | 1 (6) | 0(0) |
| 10-25% | | | 0(0) | 2 (25) |
| 25-50% | | | 5 (31) | 1 (13) |
| >50% | | | 2 (13) | 3 (0) |
| NA | | | 8 (50) | 2 (25) |

| **Anti-inflammatory drugs, n** | | | | |
|---|---|---|---|---|
| Dexamethasone | 2 | | 1 | 1 |
| anti-IL1 (anakinra) | 2 | | 0 | 2 |
| anti-IL6 (sari, toci) | 2 | | 1 | 1 |

| **Severity criteria** | | | | |
|---|---|---|---|---|
| PE, n (%) | 3 (9) | 0(0) | 1 (6) | 2 (25) |
| Maximal O2 or MV | | | 7 (1-10)* | MV = 8 (100) |
| PaO2/FiO2, med (range) | | | | 146 (99-249) |
| SOFA, med (range) | | | | 9 (4-12) |
| SOFA ≥ 8, n (%) | | | | 5 (63) |
| Death, n (%) | 0(0) | 0(0) | 0(0) | 0(0) |

| **Sampling, n (%)** | | | | |
|---|---|---|---|---|
| Delay since 1^{st} day hospital ≤ 10 days | 30 (94) | 8 (100) | 15 (94) | 7 (88) |

| | | | | |
|---|---|---|---|---|
| IQR, interquartile range; LDH, Lactate deshydrogenase; NA, not available; CT: computed tomography scan; ); sari, sarilumab ; toci, tocilizumab; PE, pulmonary embolism; SOFA, Sequential Organ Failure Assessment score; Maximal 02, L/min; MV, mechanical ventilation; PaO2/FiO2 (mmHg) at sampling; med, median (range. | | | | |

**Table 9: Characteristic of patients with circulating protein level measurements (results shown on Figure 9 and Figure 10)**

| | **All patients** | **SARS-CoV-2 negative Controls** | **SARS-CoV-2 positive Mild / Moderate** | **SARS-CoV-2 positive Severe** |
|---|---|---|---|---|
| Number | 84 (100) | 40 (48) | 19 (23) | 25 (30) |
| Age (median, IQR) | 53 (40-62) | 52 (36-58) | 49 (28-58) | 66 (59-73) |
| Age > 60 | 25 (30) | 6 (15) | 3 (16) | 16 (64) |
| Male, n (%) | 35 (42) | 14 (35) | 3 (16) | 18 (72) |

| **Comorbidities, n (%)** | | | | |
|---|---|---|---|---|
| Overweight | 45 (54) | 18 (45) | 6 (32) | 21 (84) |
| Cardiovascular | 29 (35) | 13 (22) | 4 (21) | 12 (48) |
| Diabetes | 16 (19) | 3 (8) | 3 (16) | 10 (40) |
| Respiratory | 15 (18) | 9 (23) | 1 (5) | 5 (20) |
| Cancer | 4 (5) | 2 (5) | 1 (5) | 1 (4) |

| **Initial symptoms, n (%)** | | | | |
|---|---|---|---|---|
| Fever | 30 (36) | 7 (18) | 4 (21) | 19 (76) |
| Cough | 46 (55) | 17 (43) | 12 (63) | 17 (65) |
| Dyspnea | 24 (29) | 0(0) | 3 (16) | 21 (84) |
| Myalgia | 31 (37) | 18 (45) | 6 (32) | 7 (28) |
| Diarrhea | 20 (24) | 6 (15) | 7 (37) | 7 (28) |
| Anosmia / Ageusia | 18 (45) | 8 (20) | 5 (26) | 5 (20) |

| **Laboratory findings, median (IQR)** | | | | |
|---|---|---|---|---|
| Leukocytes (G/L) | 7.1 (5.6-10.3) | 6.7 (5.6-8.5) | 5.6 (4.3-6.8) | 5.7 (4.7-8.9) |
| Neutrophils (G/L) | 4.4 (3.0-7.4) | 3.7 (2.9-5.2) | 2.8 (2.3-3.5) | 4.6 (3.4-6.1) |
| Lymphocytes (G/L) | 1.6 (1.0-2.1) | 1.9 (1.5-2.3) | 1.6 (1.0-2.1) | 1.0 (0.7-1.3) |
| Monocytes (G/L) | 0.5 (0.4-0.8) | 0.5 (0.4-0.6) | 0.5 (0.4-0.7) | 0.4 (0.2-0.7) |
| Hemoglobin (g/dL) | 12.6 (9.9-13.9) | 13.7 (12.8-14.7) | 13.0 (11.3-13.6) | 10.5 (9.6-13.0) |
| Platelet count (G/L) | 255 (205-320) | 257 (227-303) | 210 (171-296) | 191 (123-323) |
| Fibrinogen (g/L) | | | | 2270 (1285-5944) |
| D-dimers (ng/mL) | | | | 91.2 (48.8-176.6) |
| CRP (mg/L) | | | | 50.5 (30.6-98.0) |
| IL-6 (ng/L) | | | | 6.7 (5.4-8.2) |
| Ferritin (mg/L) | | | | 781 (416-1472) |
| LDH (UI/L) | | | | 418 (350-464) |

| **Chest CT findings, n (%)** | | | | |
|---|---|---|---|---|
| <10% | 0(0) | | 0(0) | 0(0) |
| 10-25% | 6 (7) | | 1 (5) | 4 (16) |
| 25-50% | 7 (8) | | 3 (16) | 4 (16) |
| >50% | 18 (21) | | 3 (16) | 15 (60) |
| NA | 54 (64) | 40 (100) | 12 (63) | 2 (8) |

| **Anti-inflammatory drugs, n** | | | | |
|---|---|---|---|---|
| Dexamethasone | 2 | | 0 | 2 |
| anti-IL1 (anakinra) | 1 | | 0 | 1 |
| anti-IL6 (sari, toci) | 2 | | 1 | 1 |

| **Severity criteria** | | | | |
|---|---|---|---|---|
| PE, n (%) | 9 (11) | 0(0) | 2 (11) | 7 (28) |
| Maximal O2/min | | | 2 (1-9)* | 15 (9-50) or MV = 20 |
| PaO2/FiO2, med (range) | | | | 164 (75-404) |
| SOFA, med (range) | | | | 7 (2-14) |
| SOFA ≥ 8, n (%) | | | | 10 (50) |
| Death, n (%) | 6 (7) | 0(0) | 0(0) | 6 (24) |

| **Sampling, n (%)** | | | | |
|---|---|---|---|---|
| Delay since 1^{st} day hospital ≤ 10 days | 78 (93) | 40 (100) | 19 (100) | 19 (76) |

| | | | | |
|---|---|---|---|---|
| IQR, interquartile range; LDH, Lactate deshydrogenase; NA, not available; CT: computed tomography scan; PE, pulmonary embolism; SOFA, Sequential Organ Failure Assessment score; MV, mechanical ventilation; PaO2/FiO2 (mmHg) at sampling; med, median (range); Sari, sarilumab ; toci, tocilizumab. | | | | |

### 2.5. Spectral flow analyses validate a contrasted innate immune cell signature in mild versus severe COVID-19

Driven by the scRNAseq-based identification of CD37, CD63 (LAMP3), CD169 (SIGLEC-1) and CD184 (CXCR4) biomarkers of blood cell subsets whose relative proportions differ in mild and severe COVID-19 patients, we added antibodies targeting these proteins to the spectral flow cytometry panel and applied this new panel to samples from an independent cohort of 90 patients (**Table 3**). This cohort included 48 controls patients and 42 COVID-19-positive, of which 16 had mild disease and 26 had severe disease (**Figure 11A** **and Table 10**).

Non-supervised analysis and UMAP visualization identified the main cell populations in the three categories of patients combined. Analyzing patients individually confirmed the significant decrease in B cell, CD4⁺ T cell and CD8⁺ T cell fractions in severe patients compared to control and mild groups, while again the NK cell fraction remained unchanged (**Figure 11B****)**. Similarly, this analysis validated the significantly greater fraction of neutrophils (**Figure 11C****)**, and within that, specifically of CD10^{Low}CD101⁻ neutrophils (**Figure 11D and 11E**) and the subset of CD10^{Low}CD101⁻ neutrophils that express CXCR4 (CD10^{Low}CD101⁻CXCR4⁺ cells) (**Figure 11F**) that we previously observed in severe patients. Finally, the fraction of CD10^{Low}CD16^{Low} neutrophils was also higher in severe patients (**Figure 12A**), further arguing for the accumulation of immature neutrophils in the blood of severe COVID patients.

Regarding monocytes, scRNAseq analyses had indicated differential changes in the distribution of non-classical CD14^{Low}CD16^{High} monocyte fractions between the two patients with severe disease (**see** **Figure 5B**). Since samples were collected from patients at various time points after admission in ICU, we suspected an effect of time spent in ICU on monocyte subset distribution. We analyzed this parameter in the 26 severe patients of this cohort, showing a significant correlation between the time spent in ICU and the fraction of non-classical monocyte subset, irrespective of the presence or absence of concurrent bacterial infection (**Figure 11G**). To better control the temporal aspect of our data, we split this cohort of severe patients into two groups, based on the mean time spent in ICU of patients with less than 5% non-classical monocytes (5.46 days) compared to those with 5% and more (8.83 days) (**Figure 11H**, **11I**). Then, we examined other monocyte subsets: in the majority of mild patients we observed a high fraction of classical monocytes that express CD169, which was not the case in either short- or long- ICU stay severe patients (**Figure 11J, 11K** **and** **12B**). These observations are consistent with a type I interferon response in mild COVID-19, as high CD169 expression also correlated with plasma levels of IFNα2a (**Figure 12C**). Independent of the time they spent in ICU, severe patients also showed a larger fraction of classical monocytes expressing high levels of CD141 compared to controls (**Figure 11J 11M**) and of monocytes expressing low levels of HLA-DR compared to controls and mild patients (**Figure 12D**). Finally, the time spent in ICU did not significantly affect the repartition of lymphocyte populations or neutrophil subsets (**Figure 12D** **and** **12E**).

Thus, mild COVID-19 was associated with high levels of CD169+ classical monocytes in blood, a signature of an ongoing IFN response, while severe COVID-19 patients exhibit a transient decrease in non-classical monocytes frequencies, a stable decrease in HLA-DR^{Low}CD141⁺ classical monocytes and major increase in CD10^{Low}CD101⁻ CXCR4^{+/-} immature neutrophils.

**Table 10. Characteristics of patients included in the validation cohort analyzed by spectral flow cytometry (CYTEK) (results shown on Figure 11 and Figure 12)**

| | **All patients** | **SARS-CoV-2 negative Controls** | **SARS-CoV-2 positive Mild / Moderate** | **SARS-CoV** - **2 positive Severe** |
|---|---|---|---|---|
| Number | 90 | 48 (53) | 16 (18) | 26 (29) |
| Age (median IQR) | 49 (31-59) | 42 (30-52) | 45 (28-58) | 62 (47-72) |
| Age>60 | 20 (22) | 3 (12) | 3 (19) | 14 (54) |
| Male, n(%) | 37 (41) | 15 (31) | 4 (25) | 18 (69) |

| **Comorbidities, n (%)** | | | | |
|---|---|---|---|---|
| Overweight | 48 (53) | 20 (42) | 4 (25) | 24 (92) |
| Cardiovascular | 25 (28) | 11 (23) | 4 (25) | 10 (38) |
| Diabetes | 11 (12) | 1 (2) | 3 (19) | 7 (27) |
| Respiratory | 17 (19) | 9 (19) | 1 (8) | 7 (27) |
| Cancer | 6 (7) | 2 (4) | 2 (13) | 2 (8) |

| **Initial symptoms, n (%)** | | | | |
|---|---|---|---|---|
| Fever | 27 (30) | 6 (12) | 4 (25) | 23 (88) |
| Cough | 49 (54) | 24 (50) | 10 (63) | 18 (69) |
| Dyspnea | 21 (23) | 1 (2) | 3 (19) | 23 (88) |
| Myalgia | 32 (36) | 20 (42) | 6 (38) | 10 (38) |
| Diarrhea | 21 (23) | 12 (25) | 6 (38) | 6 (23) |
| Anosmia / Ageusia | 15 (17) | 8 (17) | 3 (19) | 5 (19) |

| **Laboratory findings, median (IQR)** | | | | |
|---|---|---|---|---|
| Leukocytes (G/L) | 7.6 (5.6-9.9) | 6.5 (5.6-8.1) | 5.1 (4.0-6.2) | 11.1 (8.8-14.5) |
| Neutrophils (G/L) | 4.4 (2.9-7.0) | 3.4 (2.9-4.8) | 3.5 (2.9-4.9) | 8.4 (6.4-12.1) |
| Lymphocytes (G/L) | 1.8 (1.3-2.3) | 2.0 (1.7-2.5) | 1.5 (1.0-2.1) | 1.5 (0.8-1.9) |
| Monocytes (G/L) | 0.5 (0.4-0.7) | 0.5 (0.4-0.6) | 0.5 (0.4-0.6) | 0.7 (0.5-0.9) |
| Hemoglobin (g/dL) | 13.1 (11.2-14.3) | 14.0 (13.2-14.8) | 13.0 (11.3-13.9) | 9.8 (8.3-11.3) |
| Platelet count (G/L) | 256 (210-320) | 254 (225-303) | 186 (132-293) | 292 (233-394) |
| Fibrinogen (g/L) | | | | 7.7 (5.8-9.1) |
| D-dimers (ng/mL) | | | | 2330 (1632-5790) |
| CRP (mg/L) | | | | 165.5 (104.0-245.0) |
| IL-6 (ng/L) | | | | 83.7 (54.5-172.8) |
| Ferritin (mg/L) | | | | 994 (627-1633) |
| LDH (UI/L) | | | | 424 (339-452) |

| **Chest CT findings, n (%)** | | | | |
|---|---|---|---|---|
| <10% | 0 (0) | | 0 (0) | 0(0) |
| 10-25% | 5 (6) | | 2 (13) | 3 (12) |
| 25-50% | 14 (16) | | 2 (13) | 12 (46) |
| >50% | 8 (9) | | 0 (0) | 8 (31) |
| NA | 63 (70) | 48 (100) | 12 (100) | 3 (12) |

| **Anti-inflammatory drugs, n (%)** | | | | |
|---|---|---|---|---|
| Dexamethasone | 1 | | 0 | 1 |
| anti-IL 1 (anakinra) | 2 | | 0 | 2 |
| anti-IL6 (sari, toci) | 3 | | 1 | 2 |

| **Severity criteria** | | | | |
|---|---|---|---|---|
| PE, n (%) | 8 (9) | 0 (0) | 2 (13) | 6 (23) |
| Maximal O2/min | | | 2 (2-4)* | 12 (1) or MV (25)** |
| PaO2/FiO2, med (range) | | | | 159 (75-404) |
| SOFA, med (range) | | | | 4 (2-14) |
| SOFA ≥ 8, n (%) | | | | 5 (19) |
| Death, n (%) | | 0 (0) | 0 (0) | 7 (26) |

| **Sampling, n (%)** | | | | |
|---|---|---|---|---|
| Delay from 1^{st} day in hospital ≤ 10 days | 86 (95) | 48 (100) | 16 (100) | 21 (81) |

| | | | | |
|---|---|---|---|---|
| IQR, interquartile range; LDH, Lactate deshydrogenase; NA, not available; CT: computed tomography scan; PE, pulmonary embolism; SOFA, Sequential Organ Failure Assessment score; MV, mechanical ventilation; PaO2/FiO2 (mmHg) at sampling; med, median (range); Sari, sarilumab ; toci, tocilizumab. | | | | |

### 2.6. High calprotectin level and loss of non-classical monocytes correlate with COVID-19 severity

We next investigated whether changes in circulating myeloid cell phenotypes could be used to discriminate patients doomed to develop severe COVID-19 patients in routine clinics. Within our previous cohort, we separated mild patients (n=12) from moderate patients (n=6) and severe patients (n=27) using clinical criteria, and then examined differences within these 3 distinct groups. This analysis showed that among all the parameters previously segregating mild from severe COVID-19 patients, only few remained significantly different between "moderate" and severe COVID-19. Patients classified as "moderate" demonstrated intermediate changes between those of mild outpatients and severe patients in ICU (**Figure 14A**). The fraction of CD10LowCD101 - neutrophils in moderate patients was intermediate but not significantly different to any group (**Figure 13A**). However, the amount of calprotectin measured in moderate COVID-19 patients was significantly higher than mild outpatients but still significantly lower than severe COVID-19 patients (**Figure 13B**). In comparison, IFNa levels were not significantly different between moderate and mild or severe patients (**Figure 14B**). The difference in non-classical monocyte fraction was significant between mild and moderate patients, dropping down to levels comparable to severe cases (**Figure 13C**).

Thus, we hypothesized that the decreased non-classical monocyte fraction could be used as a fast and simple diagnostic test to distinguish moderate from mild COVID-19 cases, especially in cases were clinical symptoms may be substantially overlapping. This would facilitate rapid and accurate identification of currently classified as "mild" patients at the cusp of potentially progressing to more severe disease.

We employed a low dimensional flow cytometry approach that measures the fraction of classical (CD14^{High}CD16^{Low}), intermediate (CD14^{High}CD16^{High}) and non-classical (CD14^{Low}CD16^{High}) monocyte subsets among total peripheral blood monocytes and was applied initially to a learning cohort of 98 patients, consisting of 16 mild, 10 moderate and 16 severe COVID-19 patients, along with 56 controls (**Table 11**). All hospitalized patients were sampled within ten days of admission to limit the potential impact of time in ICU (see **Figure 11G**); the mean time spent in ICU was 5.5 days at the point of sampling. The cohort also included 56 controls.

We found that patients with mild disease showed a fraction of non-classical monocytes similar to that observed in controls. In contrast, moderate patients showed lower levels of non-classical monocytes, as observed in severe patients (**Figures 13D**).

To measure the global performance of this test, we used a receiver operating characteristic (ROC) curve (Hajian-Tilaki, 2013). The point of the ROC corresponding to the best sensitivity/specificity compromise indicated that a non-classical monocyte fraction below 4% separated patients with moderate or severe COVID-19 from those with mild or no disease with 76.9% sensitivity (95% bootstrap confidence interval (BCI) [61.5%; 92.3%]) and 89% specificity (95% BCI [80.6%; 95.8%]) (**Figure 14C**). We then applied these analyses to blood samples from an independent validation cohort of 24 hospitalized patients from a different clinical center (10 controls, 3 mild, 4 moderate and 7 severe COVID-19 patients) (**Table 9**). Here we found that a non-classical monocyte fraction below 4% of total circulating monocytes, as defined in the learning cohort, also segregated those with a mild disease from moderate and severe disease with high sensitivity (81.8%, 95% BCI [72.7%; 100%]) and specificity (92.3%, 95% BCI [83.3%; 100%]) (**Figure 13** **E**). These results confirmed specificity and sensitivity of our assay to discriminate patients with different COVID-19 prognosis. Further confirming these observations, we performed serial sampling two severe patients who responded to anti-IL-6R antibodies, and again documented that their clinical recovery was associated with the reappearance of non-classical monocytes in the blood (**Figure 14D**). Alongside, one patient who was referred initially with limited symptoms (atypical thoracic pain) and was SARS-Cov-2 PCR negative unexpectedly exhibited a low fraction of non-classical monocytes (3.4%), accompanied by 10% HLA-DR^{Low} classical monocytes. The following day, pulmonary symptoms appeared, the patient was hospitalized requiring oxygen therapy, and a lung CT-scan revealed characteristic COVID-associated injury. Such cases suggest that the loss of non-classical monocyte fraction, which has been described so far only in an ageing-associated chronic leukemia (Selimoglu-Buet et al, 2015) and a rare inherited disease (Hofer TP et al, 2015), could be a strong indicator of existing or impending severe COVID-19.

Additional informative parameters could be added in this flow assay, to increase its specificity to identify a transition to severe COVID-19, including a decreased expression of HLA-DR at the surface of classical monocytes (**Figure 14E**) that is associated with a decrease in non-classical monocyte fraction below 4% (**Figure 14F**), and an increase in the fraction of CD16^{Low} neutrophils (**Figure 14G**).

Together, flow identification of a decrease in non-classical monocyte fraction below 4% of total monocytes could provide improved resolution to clinical observations when categorizing patients at the borders of mild and moderate/severe COVID-19. This would potentially identify those individuals at greatest risk of rapid decline and highlight the need for pro-active management/intervention and intensive monitoring. This assay could be reinforced by analysis of HLA-DR^{Low} classical monocyte and CD16Low neutrophil fractions.

**Table 11. Characteristics of patients tested by conventional flow cytometry for non-classical monocyte fraction measurement (results shown on Figure 13 and Figure 14)**

| | **Learning cohort** | | | | | **Validation cohort** | | | | |
|---|---|---|---|---|---|---|---|---|---|---|
| | **All patie nts** | **SARS -CoV-2 nega tive Cont rots** | **SARS CoV-2 posit ive Mild** | **SARS-CoV-2 positi ve Mode rate** | **SARS CoV-2 posit ive Seve re** | **All patie nts** | **SARS -CoV-2 nega tive Cont rols** | **SARS CoV-2 posit ive Mild** | **SARS-CoV-2 positi ve Mode rate** | **SARS CoV-2 posit ive Seve re** |
| n | 98 | 56 (57) | 16 (16) | 10 (10) | 16 (16) | 24 | 10 | 3 | 4 | 7 |
| Age (median IQR) | 50 (20-89) | 46 (31-54) | 37 (26-58) | 57 (46-74) | 68 (49-75) | 60 (54 - 68) | 63 (57 - 68) | 56 (52 - 68) | 69 (65 - 74) | 46 (43 - 58) |
| Age>60 | 23 (23) | 6 (11) | 2 (13) | 5 (50) | 10 (62) | 12 (50) | 7 (70) | 1 (33) | 4 (100) | 0(0) |
| Male, n(%) | 39 (40) | 19 (34) | 5 (31) | 3 (30) | 12 (75) | 12 (50) | 6 (60) | 1 (33) | 1 (25) | 4 (57) |

| **Comorbidities, n (%)** | | | | | | | | | | |
|---|---|---|---|---|---|---|---|---|---|---|
| Overweig ht | 45 (46) | 24 (43) | 4 (25) | 5 (50) | 12 (75) | 7 (29) | 3 (30) | 1 (33) | 1 (25) | 2 (29) |
| Cardiovas cular | 33 (34) | 15 (27) | 2 (13) | 6 (60) | 9 (56) | 10 (42) | 4 (40) | 2 (67) | 3 (75) | 1 (14) |
| Diabetes | 16 (16) | 4 (7) | 1 (6) | 4 (40) | 7 (44) | 8 (33) | 5 (50) | 0 (0) | 2 (50) | 1 (14) |
| Respirato ry | 19 (19) | 12 (21) | 2 (13) | 2 (20) | 3 (19) | 8 (33) | 5 (50) | 0 (0) | 1 (25) | 3 (43) |
| Cancer | 10 (10) | 3 (5) | 2 (13) | 3 (30) | 2 (12) | 21 (87) | 10 (100) | 3 (100) | 4 (100) | 4 (57) |

| **Initial symptoms, n (%)** | | | | | | | | | | |
|---|---|---|---|---|---|---|---|---|---|---|
| Fever | 50 (51) | 25 (45) | 10 (63) | 5 (50) | 10 (62) | 9 (37) | 1 (10) | 1 (33) | 1 (25) | 6 (86) |
| Cough | 19 (19) | 1 (2) | 0 (0) | 5 (50) | 13 (81) | 10 (42) | 2 (20) | 1 (33) | 3 (75) | 4 (57) |
| Dyspnea | 38 (39) | 23 (41) | 8 (50) | 1 (10) | 6 (38) | 1 (4) | 0 (0) | 0 (0) | 1 (25) | 0 (0) |
| Myalgia | 24 (24) | 12 (21) | 5 (31) | 4 (40) | 3 (19) | 0 (0) | 0 (0) | 0 (0) | 0 (0) | 0 (0) |
| Diarrhea | 23 (23) | 11 (20) | 7 (44) | 1 (10) | 4 (25) | 1 (4) | 0 (0) | 0 (0) | 0 (0) | 1 (14) |
| Anosmia / Ageusia | 25 (26) | 8 (14) | 1 (6) | 5 (50) | 11 (69) | 12 (50) | 2 (20) | 1 (33) | 4 (100) | 5 (71) |

| **Laboratory findings, median (IQR)** | | | | | | | | | | |
|---|---|---|---|---|---|---|---|---|---|---|
| Leukocyt es (G/L) | 6.4 (1.9-22.9) | 6.5 (5.6-8.2) | 4.9 (4.3-6.0) | 5.0 (3.6-6.9) | 10.5 (9.0-16.4) | 10.5 (6.8 - 12.1) | 8.8 (5.6-12.1) | 11.2 (7.9 - 13.0) | 10.4 (6.2 - 11.9) | 10.6 (6.8 - 15.2) |
| Neutrophi ls (G/L) | 3.5 (1.1-19.1) | 3.4 (2.8-4.6) | 2.8 (2.4-3.3) | 3.4 (2.8-5.5) | 7.7 (6.5-12.4) | 7.2 (3.9 - 9.1) | 7.2 (3.7-9.6) | 8.5 (3.4 - 9.0) | 7.1 (3.9 - 8.6) | 7.9 (4.0 - 12.5) |
| Lymphocy tes (G/L) | 1.8 (0.2-3.9) | 2.0 (1.7-2.4) | 1.7 (1.3-2.2) | 1.0 (0.5-1.3) | 0.9 (0.6-1.6) | 1.0 (0.8 - 1.7) | 1.0 (0.6-1.5) | 1.6 (0.3 - 3.5) | 1.7 (0.6-2.6) | 1.4 (0.8 - 2.3) |
| Monocyte s (G/L) | 0.5 (0.0-9.3) | 0.5 (0.4-0.6) | 0.5 (0.4-0.7) | 0.2 (0.2-0.6) | 0.8 (0.4-1.1) | 0.9 (0.6 - 1.2) | 0.8 (0.4-1.1) | 1.0 (0.6 - 1.7) | 1.1 (0.7 - 1.4) | 1.2 (0.5 - 1.3) |
| Hemoglob in (g/dL) | 13.2 (11.6 - 14.1) | 13.7 (13.1 - 14.7) | 13.1 (11.4 - 13.7) | 11.5 (9.8-13.3) | 9.8 (8.1-12.0) | 10.1 (8.9 - 12.8) | 12.6 (9.7 - 15.2) | 9.1 (8.9 - 11.9) | 9.4 (8.6 - 10.1) | 8.7 (8.2 - 11.1) |
| Platelets (G/L) | 247 (43.0 - 737. 0) | 255 (225-302) | 231 (159-317) | 140 (106-233) | 246 (203-339) | 298 (200 - 494) | 272 (212 - 433) | 456 (155 - 507) | 445 (186 - 613) | 264 (153 - 444) |
| Fibrinoge n (g/L) | | | | 5.1 (3.9-7.2) | 6.9 (5.6-9.2) | | | | 5.5 (4.9 - 7.2) | 8.0 (4.9 - 8.5) |
| D-dimers (ng/mL) | | | | 1052 (409-2292) | 3172 (120 9-5301 ) | | | | 1630 (860 - 7360) | 2490 (605 - 5073 ) |
| CRP (mg/L) | | | | 44.2 (30.6-79.7) | 123. 0 (86.0 - 179. 0) | | | | 37.0 (5.1 - 266.5) | 155. 1 (52.9 - 248. 9) |
| IL-6 (ng/L) | | | | 20.2 (5.4-74.5) | 73.3 (55.9 - 111. 1) | | | | | |
| Ferritin | | | | 724 (412-1371) | 972 (694-1419 ) | | | | 1376 (327-5100) | 1730 (114 4-3060 ) |
| LDH (UI/L) | | | | 254 (204-382) | 435 (341-455) | | | | 256 (200-318) | 338 (312-1022 ) |

| **Chest CT findings, n (%)** | | | | | | | | | | |
|---|---|---|---|---|---|---|---|---|---|---|
| NA | 71 (72) | 54 (96) | 14 (88) | 1 (10) | 2 (12) | 7 (29) | 7 (70) | 0 (0) | 0 (0) | 0 (0) |
| <10% | 4 (4) | 1 (2) | 2 (13) | 1 (10) | 0 (0) | 3 (13) | 1 (10) | 1 (33) | 1 (25) | 0 (0) |
| 10-25% | 9 (9) | 1 (2) | 0 (0) | 5 (50) | 3 (19) | 2 (8) | 1 (10) | 0(0) | 2 (50) | 0 (0) |
| 25-50% | 5 (5) | 0 (0) | 0 (0) | 2 (20) | 3 (19) | 7 (29) | 0 (0) | 2 (67) | 1 (25) | 5 (71) |
| >50% | 8 (8) | 0 (0) | 0 (0) | 0 (0) | 8 (50) | 3 (13) | 1 (10) | 0 (0) | 0 (0) | 2 (29) |

| **Anti-inflammatory drugs, n** | | | | | | | | | | |
|---|---|---|---|---|---|---|---|---|---|---|
| Dexameth asone | 6 | | | 2 | 4 | 0 | | | 0 | 0 |
| anti-IL 1 (anakinra ) | 4 | | | 1 | 3 | 0 | | | 0 | 0 |
| anti-IL6 (sari, toci) | 3 | | | 2 | 1 | 3 | | | 0 | 3 |

| **Severity criteria** | | | | | | | | | | |
|---|---|---|---|---|---|---|---|---|---|---|
| PE, n (%) | 7 (7) | 1 (2) | 0 (0) | 2 (20) | 4 (25) | 1 (4) | 0 (0) | 0 (0) | 1 (25) | 0(0) |
| SOFA, median (range) | | | | | 11 (2-14) | | | | | 3 (0 - 6)** |
| Death, n (%) | 4 (4) | 0 (0) | 0 (0) | 0 (0) | 4 (25) | 1 (4) | 0 (0) | 0 (0) | 0 (0) | 1 (14) |

| | | | | | | | | | | |
|---|---|---|---|---|---|---|---|---|---|---|
| Delay between 1^{st} day in hospital and sampling ≤10 days for all patients. SOFA: Sequential Organ Failure Assessment score; * SOFA calculated for 13 pts; ** SOFA calculated for 3 pts ; NA: not available ; IQR: interquartile range. LDH, lacticodeshydrogenase; IQR, interquartile range; LDH, Lactate deshydrogenase; CT: computed tomography scan; Sari, sarilumab ; toci, tocilizumab ; PE, pulmonary embolism; SOFA, Sequential Organ Failure Assessment score; MV, mechanical ventilation; PaO2/FiO2 (mmHg) at sampling; med, median (range); . | | | | | | | | | | |

### 2.7. Integration of lung and blood scRNAseq reveals abnormal myeloid cell populations discriminating severe from mild COVID-19

The lungs, are the major organ affected in severe patients that develop acute respiratory distress syndrome. In order to better understand how the distinctive cell signatures found in the blood of severe COVID-19 patients, particularly, the presence of immature neutrophils and HLA-DRLow monocytes associated with high calprotectin, affected immune cell compartments in the lungs, we integrated our dataset using the SEURAT V3 pipeline (Stuart et al, 2019) with the published scRNAseq dataset of cells from 12 bronchoalveolar lavage fluids (BALF) of control (n=3), mild (n=3) and severe (n=6) COVID-19 patients (Liao et al, 2020). This analysis provided an unbiased global map of immune cells in the blood and BALF of control, mild and severe COVID-19 patients (see integration of scRNAseq paragraph in Patients and Methods). Using dimensional reduction, we identified 5 regions based on DEGs across pooled data from all samples (**Figure 13F**), including T cells (characterized by the expression of genes including NKG7, CD8A, CST7, GZMB and GZMA), B cells (IGLV3-19, IGHV4-34, IGHG1, IGHA1 and JCHAIN), neutrophils (G0S2, RSAD2, IL1R2 and IL1RN), alveolar macrophages (APOE, MSR1, MARCO and FBP1) and monocytes/macrophages (FN1, CXCL10, CD68 and NUPR1). Validating this approach, the alveolar macrophage region was mainly present in BALF of control patients but was dramatically decreased in mild and severe COVID-19 patients and only one cell from our blood scRNAseq matched in this region (**Figure 13F and 13G**). We also observed further differences between BALF samples from mild or severe COVID-19 patients versus controls; notably changes in the monocyte/macrophage region in SARS-CoV-2 patients, and a dramatic neutrophil accumulation in severe disease (**Figure 13G**).

Having observed clear changes in the monocyte/macrophage region in BALF from COVID-19 patients, we wanted to understand whether they related to the monocyte gene signature that we established in the blood. We began by focusing on the monocyte/macrophage region common to blood and BALF (**Figure 13H**). We robustly identified a larger population of monocytes/macrophages in mild BALF compared to control and severe groups (**Figure 13I**), characterized by the expression of the ISGs (SIGLEC-1, IFI44 and IFITM3), similar to the blood monocytes of mild patients (**Figure 13J**). Pathway analysis of blood and lung monocyte/macrophage DEGs from mild patients revealed higher expression of genes implicated in defense response to viruses, as well as interferon type I signaling, and response to type I interferon. Pathway analysis performed as previously, on lung monocytes/macrophages DEGs between mild and severe patients, revealed major changes in mild patients with upregulation of pathways implicated in the regulation of viral replication and interferon type I signaling, whereas monocytes/macrophages in severe patient were characterized by nitric-oxide synthase biosynthetic process and monocyte chemotaxis. Furthermore, again similar to blood monocytes from severe COVID-19 patients, BALF monocytes/macrophages from severe patients expressed lower levels of HLA-DRA and HLA-DRB1 and higher levels of NFKBIA mRNA compared to controls or mild COVID-19 patients (**Figure 13J**). Finally, we compared blood and BALF neutrophils (**Figure 13K**). Neutrophils were present at high frequencies in BALF from severe COVID-19 patients but not in BALF from controls or mild patients (**Figure 13L**). UMAP integration indicated that BALF neutrophils in severe COVID-19 were relatively similar to blood neutrophils of severe patients (**Figure 13L**), and were characterized by high expression of S100A, S100A9 as well as CXCR4, which indicated an immature state (**Figure 13M, 13N**).

Altogether our integration analysis reveals a common interferon signature in mild COVID-19 blood monocytes and BALF monocytes/macrophages, whereas monocytes/macrophages (not including alveolar macrophages) in severe patients are characterized by the loss of HLA-DRA and HLA-DRB1 and high NFKBIA expression. Moreover, an accumulation of neutrophils expressing high levels of S100A8/A9 and CXCR4 is observed in the blood and BALF of severe COVID-19 patients. All these results suggest a crucial role of monocytes and neutrophils associated with calprotectin in the establishment of severe COVID-19 lung infection.

### 3. Discussion

This study presents evidence that most patients infected with mild SARS-CoV-2 infection may develop a transient and beneficial interferon response whereas those with more severe disease instead exhibit high levels of calprotectin and inflammatory cytokines and chemokines correlating with an emergency myelopoiesis generating ROS- and NOS- expressing immunosuppressive myeloid cells (HLA-DR^{Low} monocytes, immature subsets of neutrophils and erythroblasts).

As in other viral infections, the first line of defense in patients infected with SARS-CoV-2 may be a protective innate response that involves the transient and strong production of type I IFNs. Through inducing expression of ISGs, type I IFNs could inhibit virus replication and promote an effective innate and adaptive immune response (Thevarajan et al., 2020; Totura and Baric, 2012). Such a response was detected in patients with mild COVID-19 by combining serial single cell gene expression profile analysis, high dimensional spectral flow cytometry and plasma cytokine level measurements. A transient increase in IFNa2a plasma level was associated with increased SIGLEC1 gene expression and the expression of CD169 (SIGLEC1) on classical monocytes. Furthermore, the plasma level of CXCL8, whose gene expression level is negatively regulated by type I IFNs (Nozell et al., 2006; Nyhlen et al., 2000), was lower in outpatients with mild COVID-19 compared to control patients with negative SARS-CoV-2 PCR. A clear type I interferon response was similarly found in the monocytes/macrophages (excluding alveolar macrophages) collected from the BALF of mild patients (Liao et al, 2020), indicative of a systemic antiviral response in mild patients.

In COVID-19, this antiviral response is impaired in patients who suddenly evolve into clinically threatening disease (Hadjadj J et al, 2020 *in press*). Severe COVID-19 frequently develops in the context of advanced age and comorbidities including hypertension, obesity and diabetes mellitus, which all provide a degree of underlying systemic chronic inflammation (Furman et al., 2019). Such pre-existing inflammation could disrupt the timing of type I IFN response relative to the kinetics of virus replication (Teran-Cabanillas and Hernandez, 2017). This timing was shown to be critical in mouse models of coronavirus infection, the IFN-I response to MERS-CoV infection occurs simultaneously with the peak viral replication and promotes virus clearance (Channappanavar et al., 2019), whereas in SARS-CoV-infected mice the type I IFN response is deleterious, lagging behind peak viral replication, and thereby promoting the accumulation of inflammatory monocytes and macrophages and sensitizes T cells to cell death by apoptosis (Channappanavar, 2016). An imbalance between the type I IFN and inflammatory responses could also be favored by the highly efficient replication of SARS-CoV-2 in human tissues, as measured in an *ex vivo* lung model (Chu et al., 2020), and by the IFN neutralizing effects of structural and non-structural viral components shared between SARS-CoV-2 and other virulent human coronaviruses (Chen et al., 2014; Yang et al., 2015).

Severe COVID-19 patients exhibited abnormal partition of circulating monocytes, and of neutrophils expressing S100A8 and S100A9 alarmin genes. In addition, we confirmed the presence of high serum levels of such alarmins in severe patients. In our cohort, patients with COVID-19 have a high prevalence of obesity, cardiovascular disease and/or diabetes mellitus; as alarmins S100A8 (calgranulin A / myeloid-related protein 8) and S100A9 (calgranulin B / myeloid-related protein 14) are chronically released in most of these inflammatory diseases (Nagareddy et al., 2014; Wang et al., 2018) the presence of comorbidities in severe COVID-19 patients was accompanied by further increases in these molecules. Calprotectin, the heterodimer formed by S100A8 and S100A9 proteins, was also more abundant in controls with comorbidities compared to other controls. The release of massive amounts of calprotectin is a striking event associated with severe COVID19. These proteins comprise approximately 45% of the cytoplasmic proteins in neutrophils, with lower levels in monocytes. They promote cell migration and boost NADPH (Nicotinamide Adenine Dinucleotide Phosphate) Oxidase activity. When released, calprotectin is a TLR4 and RAGE (receptor for advanced glycation end products) ligand that, upstream of TNFα (Vogl et al., 2018) and CXCL8 (Simard et al., 2014) synthesis and secretion, promotes NF-kB activation (Riva et al., 2012) and the secretion of multiple inflammatory proteins as IL-6 (Wang et al., 2018). Importantly, an accumulation of neutrophils expressing high levels of S100A8/A9 was also observed in the BALF of severe COVID-19 patients. Thus, we propose that calprotectin may account for, and possibly trigger the cytokine release syndrome that characterizes severe COVID-19. Its production may be amplified by tissue damage, generating a harmful hyper-inflammation loop (Kuipers et al., 2013) that precludes these peptides from exerting more protective functions (Austermann et al., 2014; Freise et al., 2019; Ulas et al., 2017; Vogl et al., 2018). All these results suggest the presence of chronic inflammation from comorbidities, synergizes with SARS-CoV-2 viral infection, to induces a systemic release of IL-6 and calprotectin in severe COVID-19 patients which translate by the up-regulation of NF-KB and the loss of HLA-DR on classical monocytes and the presence of immature neutrophils, altogether converging to a state of chronic inflammatory induced immunosuppression.

In healthy conditions, roughly 85% of total circulating monocytes are CD14^{High}CD16^{Low}HLA-DR^{High} cells that express high levels of CCR2 (the chemokine MCP-1 receptor) but low levels of CX3CR1 (the fractalkine receptor) and are rapidly recruited to inflamed tissues (Guilliams et al., 2018). As in other severe illnesses (Lukaszewicz et al., 2009), the expression of HLA-DR on CD14^{High} circulating monocytes is low in COVID-19, which correlates with, and could be mediated by, IL-6 overproduction (Giamarellos-Bourboulis et al., 2020). A more specific feature of COVID-19 is the low fraction of CD14^{Low}CD16^{+High}non-classical monocytes. This fraction commonly increases in patients with sepsis and inflammatory diseases, including viral infections (Kratofil et al., 2017). The decrease in non-classical monocyte fraction could involve the ability of calprotectin to fasten the trans-endothelial migration of leucocytes (Fassl et al., 2015), unless these cells strongly adhere to the endothelium, thus are not collected through conventional bleeding regimes (Guilliams et al., 2018), or the conversion of classical into non-classical monocytes is stuck as a consequence of *NR4A1* and *CSF-1R* down-regulation (Hanna et al., 2011; Hofer et al., 2015) or epigenetic modifications (Selimoglu-Buet et al., 2018). Whatever the mechanism, the lower than normal frequencies of non-classical monocytes (Thevarajan et al., 2020) (Hadjadj J et al, 2020) suggests a SARS-CoV-2 characteristic effect that has not been reported in other viral infections. Most importantly, this decrease generates an unusual and highly characteristic biological signature of COVID-19's aggressive form, with the potential to be easily measured using standard diagnostic flow cytometry and to provide information on the real-time immunological severity of the infection which may not always be clear from the clinical picture.

The burst of calprotectin detected in COVID-19 patients may trigger NF-kB-driven emergency myelopoiesis, generating immature and dysplastic cells (Basiorka et al., 2016; Chen et al., 2013). This effect could be amplified if SARS-CoV-2, by interacting with hACE2 expressed by stromal cells of the bone marrow, disrupts the interaction of the receptor with granulocyte colony-stimulating factor (Kohlstedt et al., 2018). In addition to TLR4 and RAGE, CD33 expressed on myeloid cells is a receptor for S100A9 that initiates suppressive inflammatory signaling cascades (Eksioglu et al., 2017). Finally, given the considerable hematopoietic potential of the lung (Lefrancais et al., 2017), the burst of calprotectin could also promote the contribution of lung megakaryocytes to disease pathogenesis in this organ.

The immature and mature cells released into the peripheral blood by emergency bone marrow myelopoiesis may be endowed with immunosuppressive functions, suggesting myeloid derived suppressive cells (MDSCs) as detected in cancer, inflammation and other diseases (Veglia et al., 2018) might be important in COVID-19. In addition to HLA-DRLow monocytes whose phenotype is that of monocytic MDSCs (M-MDSCs) (Veglia et al., 2018), CD71+ erythroblasts expressing arginase-2 (Elahi et al., 2013) can be detected in the blood of the most severe COVID-19 patients. Granulocytes are also functionally heterogeneous (Evrard et al., 2018), exerting T-cell suppressive functions when released under conditions of acute inflammation (Pillay et al., 2012) and mobilized by G-CSF (Devi et al., 2013; Marini et al., 2017). Here, we show that their heterogeneity increases with disease progression which expands a population of CD10LowCD101-CXCR1+ immature cells, reminiscent of granulocytic MDSCs (G-MDSCs) (Aarts et al., 2019; Mastio et al., 2019; Veglia et al., 2018). These cells express nitric oxide synthase and generate ROS and immunosuppressive cytokines including IL-6. A link was recently suggested between some of these granulocytes that form neutrophil extracellular traps (Carlucci et al., 2018) and pulmonary disease and capillary thrombosis in response to SARS-CoV-2 infection (Barnes et al., 2020; Zuo et al., 2020). A major increase of CD10LowCD101- immature subsets of neutrophils, including a fraction of them expressing CXCR4, was observed in severe patients, indicating that neutrophil precursors such as the pre-neutrophil (preNeu) population that are CXCR4 positive (Evrard et al., 2018), may be released into the blood from the bone marrow prematurely. These immature neutrophils infiltrated the lung tissue in severe patients. Consistent with this notion, this population was relatively abundant in the blood of severe COVID-19 patients, as well as in the BALF neutrophils (expressing CXCR4, S100A8/A9) of these patients from another study (Liao et al, 2020). Taken together, the emergence of this population could be a predictor of switch to a severe disease. Further research will be required to determine their specific role in disease development.

Altogether, we observed that severe COVID-19 is specifically associated with 1) the absence of a type I IFN response; 2) a burst of circulating calprotectin that precedes cytokine release syndrome as calprotectin level in moderate patients that do not present cytokine release syndrome is higher than mild patients; 3) low levels of non-classical monocytes in the peripheral blood and 4) an emergency myelopoiesis that releases immature and dysplastic myeloid cells with an immune suppressive phenotype

We also showed that the presence of low frequencies of non-classical monocytes in the blood of patients could be implemented in a routine lab to identify those mild/moderate patients with early immunological signs consistent with developing more severe disease. The burst in calprotectin plasma level warrants testing it as another biomarker of the progression to severe COVID-19.

Finally, in addition to the network of potential drug targets recently depicted by analysis of SARS-CoV-2 interactions (Gordon et al., 2020), our work provides further rationale for the testing of several clinical strategies, including: blocking emergency myelopoiesis with lenzilumab (NCT04351152), a recombinant anti-human GM-CSF antibody (Patnaik et al., 2020), testing the oral quinoline-3-carboxamide Tasquinimod, (Fizazi et al., 2017) and developing both the preclinical small-molecule inhibitor, ABR-215757 (Paquinimod) which blocks the binding of S100A9 to TLR4 and RAGE (Kraakman et al., 2017; Raquil et al., 2008) and the preclinical anti-CD33 monoclonal antibodies (Walter, 2018) which may prevent the interaction of S100A9 with myeloid progenitors.

## Claims

1. An *in vitro* method for the prognosis, for the monitoring, for the stratification, and/or for determining the outcome of a betacoronavirus disease in a subject in need thereof, said method comprising the step of detecting, in a biological sample from said subject, an innate immune response driven by a calprotectin burst.

2. The *in vitro* method of claim 1, wherein said innate immune response involves a decrease of CD14^{low} /CD16⁺ monocytes, an increase of HLA-DR^{low} classical monocytes; and/or an increase of CD16^{low} neutrophils.

3. The *in vitro* method of claim 1 or 2, further comprising the step of measuring the level of circulating calprotectin.

4. The *in vitro* method of any one of claim 1 to 3, comprising the steps of:
a) Quantifying, in a biological sample from the subject, the level of at least one biomarker selected from the group consisting of:
- CD14^{low}/CD16⁺ monocytes;
- HLA-DR^{low} classical monocytes;
- circulating calprotectin; and/or
- CD16^{low} neutrophils;
b) Comparing the level(s) of the biomarker(s) quantified obtained in step a) to (a) reference level(s) of the same biomarker(s); and
c) Determining the prognosis of the betacoronavirus disease, monitoring the betacoronavirus disease, stratifying the patients suffering from the betacoronavirus disease, determining the outcome of betacoronavirus disease, based on the comparison of step b).

5. The *in vitro* method of claims 1 to 4, for selecting a therapy for treating said subject, or for assessing the efficacy of a therapy in a treated subject.

6. The method of any one of claims 1 to 5, wherein :
(i) If the level of CD14^{low}/CD16⁺ monocytes quantified in step a) is lower than the reference level of step b), wherein said reference level is the mean level of CD14low/CD16+ monocytes quantified in a healthy subject or in a subject suffering from an asymptomatic or a mild COVID;
(ii) If the level of HLA-DR^{low} classical monocytes quantified in step a) is higher than the reference level of step b), wherein said reference level is the mean level of HLA-DR^{low} classical monocytes quantified in a healthy subject or in a subject suffering from an asymptomatic or a mild COVID;
(iii) If the level of S100A8/S100A9 calprotectins quantified in step a) is higher than the reference level of step b), wherein said reference level is the mean level of S100A8/S100A9 calprotectins quantified in a healthy subject or in a subject suffering from an asymptomatic or a mild COVID; and/or
(iv) If the level of CD16^{low} neutrophils quantified in step a) is higher than the reference level of step b), wherein said reference level is the mean level of CD16^{low} neutrophils quantified in a healthy subject or in a subject suffering from an asymptomatic or a mild COVID;
then the betacoronavirus disease is exacerbated and/or has switched to a severe form, worsening the outcome and the prognosis of the disease; the risk of switching to a severe COVID form is high, and the subject is identified as being at risk of switching to a severe COVID form.

7. The method of any one of the preceding claims, further comprising the steps of quantifying all the monocytes in the sample(s) and calculating the ratio of CD14^{low}/CD16⁺ monocytes and/or the ratio of HLA-DR^{low} classical monocytes to all monocytes.

8. The method of any one of the preceding claims, wherein a ratio of CD14^{low}/CD16⁺ monocytes to all monocytes lower than 4% is characteristic of a poor prognosis.

9. The method of any one of the preceding claims, further comprising the steps of quantifying all the neutrophils in the sample(s) and calculating the ratio of CD16^{low} neutrophils to all neutrophils.

10. The method of any one of the preceding claims, wherein the level of CD14^{low}/CD16⁺ monocytes, the level of HLA-DR^{low} classical monocytes, the level of S100A8/S100A9 calprotectins and/or the levels of CD16^{low} neutrophils is quantified by flow cytometry.

11. The method of any one of the preceding claims, wherein said sample is a blood sample, a plasma sample, or a bone marrow sample.

12. A S100A8/S100A9 calprotectin inhibitor for use in treating a betacoronavirus disease, wherein the S100A8/S100A9 calprotectin inhibitor is preferably selected from the group consisting of tasquinimod, paquinimod, pharmaceutically acceptable salts thereof, and any combination thereof.

13. The method of any one of claims 1 to 11, or the S100A8/S100A9 calprotectin inhibitor for use according to claim 12, wherein said betacoronavirus disease is the cororonavirus disease 19 (COVID-19).
